# EUROPEAN PATENT APPLICATION

(11) **EP 1 712 621 A1**
(43) Date of publication of application: **18.10.2006**
(21) Application number: 05704011.5
(22) Date of filing: 21.01.2005
(51) Int. Cl.: C12N 15/09, A01H 5/00, C12Q 1/68

(54) **GENE MARKERS LINKED TO FUSARIUM HEAD BLIGHT-RESISTANCE FACTOR AND UTILIZATION THEREOF**

(30) Priority: 22.01.2004 JP 2004014839; 16.09.2004 JP 2004270268
(71) Applicant: Japan Science and Technology Agency, Kawaguchi-shi, Saitama 332-0012 (JP)
(72) Inventor: TAKEDA, Kazuyoshi, c/o Japan Sci. and Tech.Agency, Kawaguchi-shi, Saitama 332-0012 (JP); SATO, Kazuhiro, c/o Japan Sci. and Tech.Agency, Kawaguchi-shi, Saitama 332-0012 (JP)
(74) Representative: Schwarz, Albin
(86) International application number: PCT/JP2005/000790
(87) International publication number: WO 2005/071076

(57) **Abstract**

QTL analysis on the resistance to Fusarium head blight is performed by using an RI line (RHI population) grown by crossing Russia 6 (two-row, resistant) with H.E.S.4 (six-row, susceptible), an RI line (RI2 population) grown by crossing Harbin 2-row (resistant) with Turkey 6 (susceptible), and a DH line (DHHS population) grown by crossing Haruna Nijo (resistance) with H602 (susceptible). As a result, QTLs are detected on the 2H, 4H and 5H chromosomes in the RHI population, on the 2H, 4H and 6H chromosomes in the RI2 population and on the 2H, 4H and 5H chromosomes in the DHHS population. Furthermore, genetic markers linked to these QTLs (MM314, FM677, FM426, MM1057, MMtgaEatc128, FMgcgEatc 530, FXLRRfor_XLRRrev119, FMacgEcgt288, HVM67. FMataEagc408, HVM11, Bmag125, k04002, k05042, k03289, HvLOX and k00835) are found out.

SEQUENCE LISTING

<110> Japan Science and Technology Agency

<120> Genetic markers that link to genes associated with Fusarium head blight
resistance, and use thereof

<130> A181-09PCT

<150> JP 2004-014839
<151> 2004-01-22

<150> JP 2004-270268
<151> 2004-09-16

<160> 56

<170> PatentIn Ver. 2.1

<210> 1
<211> 20
<212> DNA
<213> Artificial Sequence

<220>
<223> Description of Artificial Sequence:Artificially
      Synthesized Primer Sequence

<400> 1
agagatccct gctcagcttg                                              20


<210> 2
<211> 20
<212> DNA
<213> Artificial Sequence

<220>
<223> Description of Artificial Sequence:Artificially
      Synthesized Primer Sequence

<400> 2
tcgtattaag gccgcatagg                                              20


<210> 3
<211> 20
<212> DNA
<213> Artificial Sequence

<220>
<223> Description of Artificial Sequence:Artificially
      Synthesized Primer Sequence

<400> 3
gcacgtagcg ttcaacatca                                              20


<210> 4
<211> 20
<212> DNA
<213> Artificial Sequence

<220>
<223> Description of Artificial Sequence:Artificially
      Synthesized Primer Sequence

<400> 4
   aacttttccc aaccctttcc                                                20

<210> 5
<211> 20
<212> DNA
<213> Artificial Sequence

<220>
<223> Description of Artificial Sequence:Artificially
      Synthesized Primer Sequence

<400> 5
ccgtgtgtcg tctaggtcaa                                                20

<210> 6
<211> 20
<212> DNA
<213> Artificial Sequence

<220>
<223> Description of Artificial Sequence:Artificially
      Synthesized Primer Sequence

<400> 6
caactttggt gggacgtagg                                                20

<210> 7
<211> 20
<212> DNA
<213> Artificial Sequence

<220>
<223> Description of Artificial Sequence:Artificially
      Synthesized Primer Sequence

<400> 7
gttttcgcca tcactcttcc                                                20

<210> 8
<211> 208
<212> DNA
<213> Hordeum vulgare

<400> 8
gcacgtagcg ttcaacatca catctcatct ttggcaaaag aaatggtcaa tcggcacact 60
ctcagttttt tgcaccaaaa tgtcccatga gaccaccaac atgtgcttcc cgcaatagta 120
gtaaacgaac aaagcaatct agaatgcata gtttgttagc acgaaacagg aaaccatcat 180
gcacataaaa cttttcccaa ccctttcc                                       208

<210> 9
<211> 208
<212> DNA
<213> Hordeum vulgare

<400> 9
gcacgtagcg ttcaacatca catctcatct ttggcaaaag aaatggtcaa tcggcacact 60
ctcagttttt tgcaccaaaa tgtcccatga gaccaccaac atgtgcttcc cgcaatagta 120
gtaaacgaac aaagctatct agaatgcata gtttgttagc acgaaacagg aaaccatcat 180
gcacataaaa cttttcccaa ccctttcc                                       208

<210> 10
<211> 335

<212> DNA
<213> Hordeum vulgare

<400> 10
ccgtgtgtcg tctaggtcaa ccatgacaac aactacgtgt ggtcgtgact ttggcctgct 60
gttgtcacct caccatggat tgtggtctcc atcaaaagtt atttgtgaga gtttcatctc 120
tgcagatctg gtggttgact ttggtggcat gatcataact atggacggag tcttgatccg 180
gagggatggt tgtgcgccaa cgaaggtcac attgcatgta attgttcnga tcacggaaga 240
gtgatggcga aaacacatga gtgacttcaa tggtggtgtt acttcagtat ccggtatcaa 300
gcttcanggc aaaagcctac gtcccaccaa agttg 335

<210> 11
<211> 335
<212> DNA
<213> Hordeum vulgare

<400> 11
ccgtgtgtcg tctaggtcaa ccatgacaac aactacgtgt ggtcgtgact ttggcctgct 60
gttgtcacct caccatggat tgtggtctcc atcaaaagtt atttgtgaga gtttcatctc 120
tgcagatctg gtggttgact ttggtggcat gatcataact atggacggag tcttgatccg 180
gagggatggt tgtgcggcaa cgaaggtcac attgcatgta attgttcnga tcacggaaga 240
gtgatggcga aaacacatga gtgacttcaa tggtggtgtt acttcagtat ccggtatcaa 300
gcttcanggc aaaagcctac gtcccaccaa agttg 335

<210> 12
<211> 254
<212> DNA
<213> Hordeum vulgare

<400> 12
ccgtgtgtcg tctaggtcaa ccatgacaac aactacgtgt ggtcgtgact ttggcctgct 60
gttgtcacct caccatggat tgtggtctcc atcaaaagtt atttgtgaga gtttcatctc 120
tgcagatctg gtggttgact ttggtggcat gatcataact atggacggag tcttgatccg 180
gagggatggt tgtgcgccaa cgaaggtcac attgcatgta attgttcnga tcacggaaga 240
gtgatggcga aaac 254

<210> 13
<211> 254
<212> DNA
<213> Hordeum vulgare

<400> 13
ccgtgtgtcg tctaggtcaa ccatgacaac aactacgtgt ggtcgtgact ttggcctgct 60
gttgtcacct caccatggat tgtggtctcc atcaaaagtt atttgtgaga gtttcatctc 120
tgcagatctg gtggttgact ttggtggcat gatcataact atggacggag tcttgatccg 180
gagggatggt tgtgcggcaa cgaaggtcac attgcatgta attgttcnga tcacggaaga 240
gtgatggcga aaac 254

<210> 14
<211> 17
<212> DNA
<213> Artificial Sequence

<220>
<223> Description of Artificial Sequence:Artificially
      Synthesized Primer Sequence

<400> 14
ctcgtagact gcgtacc 17

<210> 15
<211> 18
<212> DNA

<213> Artificial Sequence

<220>
<223> Description of Artificial Sequence:Artificially
      Synthesized Primer Sequence

<400> 15
aattggtacg cagtctac                                                    18


<210> 16
<211> 16
<212> DNA
<213> Artificial Sequence

<220>
<223> Description of Artificial Sequence:Artificially
      Synthesized Primer Sequence

<400> 16
gacgatgagt cctgag                                                      16


<210> 17
<211> 14
<212> DNA
<213> Artificial Sequence

<220>
<223> Description of Artificial Sequence:Artificially
      Synthesized Primer Sequence

<400> 17
tactcaggac tcat                                                        14


<210> 18
<211> 16
<212> DNA
<213> Artificial Sequence

<220>
<223> Description of Artificial Sequence:Artificially
      Synthesized Primer Sequence

<400> 18
gactgcgtac caattc                                                      16


<210> 19
<211> 16
<212> DNA
<213> Artificial Sequence

<220>
<223> Description of Artificial Sequence:Artificially
      Synthesized Primer Sequence

<400> 19
gatgagtcct gagtaa                                                      16


<210> 20
<211> 19
<212> DNA
<213> Artificial Sequence

<220>

<223> Description of Artificial Sequence:Artificially
      Synthesized Primer Sequence

<400> 20
gactgcgtac caattcccg                                                    19


<210> 21
<211> 19
<212> DNA
<213> Artificial Sequence

<220>
<223> Description of Artificial Sequence:Artificially
      Synthesized Primer Sequence

<400> 21
gatgagtcct gagtaaacg                                                    19


<210> 22
<211> 18
<212> DNA
<213> Artificial Sequence

<220>
<223> Description of Artificial Sequence:Artificially
      Synthesized Primer Sequence

<400> 22
ccgttggaca ggaaggag                                                     18


<210> 23
<211> 18
<212> DNA
<213> Artificial Sequence

<220>
<223> Description of Artificial Sequence:Artificially
      Synthesized Primer Sequence

<400> 23
cccatagacc ggactgtt                                                     18


<210> 24
<211> 21
<212> DNA
<213> Artificial Sequence

<220>
<223> Description of Artificial Sequence:Artificially
      Synthesized Primer Sequence

<400> 24
aattagcgag aacaaaatca c                                                 21


<210> 25
<211> 18
<212> DNA
<213> Artificial Sequence

<220>
<223> Description of Artificial Sequence:Artificially
      Synthesized Primer Sequence

<400> 25
agataacgat gcaccacc                                                    18


<210> 26
<211> 20
<212> DNA
<213> Artificial Sequence

<220>
<223> Description of Artificial Sequence:Artificially
      Synthesized Primer Sequence

<400> 26
gacacaggac ctgaagcaca                                                  20


<210> 27
<211> 20
<212> DNA
<213> Artificial Sequence

<220>
<223> Description of Artificial Sequence:Artificially
      Synthesized Primer Sequence

<400> 27
cggcaggctc tactatgagg                                                  20


<210> 28
<211> 20
<212> DNA
<213> Artificial Sequence

<220>
<223> Description of Artificial Sequence:Artificially
      Synthesized Primer Sequence

<400> 28
cagcatatcc atctgatctg                                                  20


<210> 29
<211> 21
<212> DNA
<213> Artificial Sequence

<220>
<223> Description of Artificial Sequence:Artificially
      Synthesized Primer Sequence

<400> 29
cacccttatt tattgcctta a                                                21


<210> 30
<211> 20
<212> DNA
<213> Artificial Sequence

<220>
<223> Description of Artificial Sequence:Artificially
      Synthesized Primer Sequence

<400> 30
tccatgttcc cagctacaca                                                  20

<210> 31
<211> 20
<212> DNA
<213> Artificial Sequence

<220>
<223> Description of Artificial Sequence:Artificially
      Synthesized Primer Sequence

<400> 31
aggaacacat tggttctggc                                                    20


<210> 32
<211> 19
<212> DNA
<213> Artificial Sequence

<220>
<223> Description of Artificial Sequence:Artificially
      Synthesized Primer Sequence

<400> 32
gatgagtcct gagtaaatc                                                     19


<210> 33
<211> 19
<212> DNA
<213> Artificial Sequence

<220>
<223> Description of Artificial Sequence:Artificially
      Synthesized Primer Sequence

<400> 33
gactgcgtac caattctga                                                     19


<210> 34
<211> 19
<212> DNA
<213> Artificial Sequence

<220>
<223> Description of Artificial Sequence:Artificially
      Synthesized Primer Sequence

<400> 34
gatgagtcct gagtaaatc                                                     19


<210> 35
<211> 19
<212> DNA
<213> Artificial Sequence

<220>
<223> Description of Artificial Sequence:Artificially
      Synthesized Primer Sequence

<400> 35
gactgcgtac caattcgcg                                                     19


<210> 36
<211> 19

<212> DNA
<213> Artificial Sequence

<220>
<223> Description of Artificial Sequence:Artificially
      Synthesized Primer Sequence

<400> 36
gatgagtcct gagtaaacg                                                    19


<210> 37
<211> 19
<212> DNA
<213> Artificial Sequence

<220>
<223> Description of Artificial Sequence:Artificially
      Synthesized Primer Sequence

<400> 37
gactgcgtac caattccgt                                                    19


<210> 38
<211> 18
<212> DNA
<213> Artificial Sequence

<220>
<223> Description of Artificial Sequence:Artificially
      Synthesized Primer Sequence

<400> 38
gtcgggctcc attgctct                                                     18


<210> 39
<211> 18
<212> DNA
<213> Artificial Sequence

<220>
<223> Description of Artificial Sequence:Artificially
      Synthesized Primer Sequence

<400> 39
ccggtaccca gtgacgac                                                     18


<210> 40
<211> 20
<212> DNA
<213> Artificial Sequence

<220>
<223> Description of Artificial Sequence:Artificially
      Synthesized Primer Sequence

<400> 40
atacatgcat gccattgtgg                                                   20


<210> 41
<211> 20
<212> DNA
<213> Artificial Sequence

<220>
<223> Description of Artificial Sequence:Artificially
      Synthesized Primer Sequence

<400> 41
atccatccac tgtttgaggg                                                    20


<210> 42
<211> 20
<212> DNA
<213> Artificial Sequence

<220>
<223> Description of Artificial Sequence:Artificially
      Synthesized Primer Sequence

<400> 42
tgctctgcat ttcattcagc                                                    20


<210> 43
<211> 20
<212> DNA
<213> Artificial Sequence

<220>
<223> Description of Artificial Sequence:Artificially
      Synthesized Primer Sequence

<400> 43
cagcgttaca ggcattctca                                                    20


<210> 44
<211> 19
<212> DNA
<213> Artificial Sequence

<220>
<223> Description of Artificial Sequence:Artificially
      Synthesized Primer Sequence

<400> 44
gatgagtcct gagtaaata                                                     19


<210> 45
<211> 19
<212> DNA
<213> Artificial Sequence

<220>
<223> Description of Artificial Sequence:Artificially
      Synthesized Primer Sequence

<400> 45
gactgcgtac caattcagc                                                     19


<210> 46
<211> 18
<212> DNA
<213> Artificial Sequence

<220>
<223> Description of Artificial Sequence:Artificially
      Synthesized Primer Sequence

<400> 46
ccggtcggtg cagaagag                                                    18


<210> 47
<211> 24
<212> DNA
<213> Artificial Sequence

<220>
<223> Description of Artificial Sequence:Artificially
      Synthesized Primer Sequence

<400> 47
aaatgaaagc taaatgggcg atat                                             24


<210> 48
<211> 251
<212> DNA
<213> Hordeum vulgare

<220>
<221> n
<222> (7)

<220>
<221> n
<222> (17)

<220>
<221> n
<222> (29)

<220>
<221> n
<222> (47)

<220>
<221> n
<222> (98)

<400> 48
ttacagntat aataggnggt ttcagttgnt agtgctttta caacagnaat agaagggttt 60
cactgtgaat ccacaacata tcaagtggta cacatagnac catgtcattc atacatgcat 120
gccattgtgg ggagaagcac aggtgggtgg gaaattccct ctcctcaaag agaagatgtg 180
gctatgcctt gagcatccgc aggtagtctt cgattatgga catggctgac gagcggtacc 240
cagagccgaa a                                                           251


<210> 49
<211> 251
<212> DNA
<213> Hordeum vulgare

<220>
<221> n
<222> (35)

<220>
<221> n
<222> (37)

<400> 49
tgttgggaca tggagctgca gctcttgcac aaccnanctc ctctgctctg catttcattc 60
agcaaataaa ctcatggcct tgctgctgct tccctaaaac ctaaagacta gcaactactg 120
gtacacgcaa cctcctcctc catctctagc tatgaacaag gtaaatttac ccaccttttc 180

tatcacaaaa aaaatatgaa caccaagccg aatcacgact gagaatttta cacaatgata 240
atcgctcagc c 251

<210> 50
<211> 249
<212> DNA
<213> Hordeum vulgare

<220>
<221> n
<222> (19)

<400> 50
   acaagcgcac attttaatnc ttattatcgc tcactcactc gaagagttgc gtattaaaat 60
   tacactaaaa acctaacagc tcagctagct aagctcctcc tcaaccatca acatgggaac 120
   aatccatgtt cccagctaca catatgatac atacatcagc taagctcttc tagcaaccca 180
   accaatgacc ctggttggat tcagaagttg ctcttgctcg tcgaaggctt cgacctcttg 240
   ctgttgagc 249

<210> 51
<211> 21
<212> DNA
<213> Hordeum vulgare

<400> 51
gaaaacatgc aggtgaactc a 21

<210> 52
<211> 21
<212> DNA
<213> Hordeum vulgare

<400> 52
gaaaacatgc cggtgaactc a 21

<210> 53
<211> 21
<212> DNA
<213> Hordeum vulgare

<400> 53
gcgggcgccg cgggtcgagc g 21

<210> 54
<211> 21
<212> DNA
<213> Hordeum vulgare

<400> 54
gcgggcgccg tgggtcgagc g 21

<210> 55
<211> 519
<212> DNA
<213> Hordeum vulgare

<400> 55
agcactccag tgttggggca ttgtcgactt cagtgcacgg ccatctcaca acaagcagca 60
accgcttgat gggaacatgt ttattaacta tatcgtcagg aagtgctgtg accttggcat 120
tcagatgaac aagacagcat gttttgtgca tctatcagaa atgtcagtgc tatcggatcc 180
acaccaactg cacgaggagc tgaacaaagc aaagcaggct gcggtgaaga agaaccagaa 240
gctgcaactc ctcttctgcc cgatgtctga gcagcatcat gggtacaaga cactgaagct 300

gatctgcgag acgcagctgg ggatccaaac ccagtgtttc ttgagccacc tcgcaaacaa 360
aacccagggc caggaccagt acatgtccaa tcttgctctc aagatcaacg gcaagctcgg 420
gggcatcaac acccagctcc aggacaagct cccactggac aacggtgttc cttacatgtt 480
cataggcgca gacgtgaacc acccatcacc tgggaatgg 519

<210> 56
<211> 538
<212> DNA
<213> Hordeum vulgare

<220>
<221> n
<222> (41)

<400> 56
agtcgctcgt tgtggctttg catatcatca gactggcaag nggccaggca accacataac 60
tatagtataa aagacacaga cctgaagcac acgcacaatg acagaaccag caatacgttc 120
caagttcaaa gttcagacca tacgaaacag ttcggagtac atgttctacc ggaccaagtt 180
cagaccatat gaagcagtct gtatatatgt tctaccggac caagttcaga ccatatgaag 240
cagtctgtat atatgttcta ccggaccaag ttcagaccat atgaagcagt ctgtatatat 300
gttctaccgg accaagacgc taagcggcac cggtgagaag ccggcgtcag atgaagaaca 360
tgttgtcctg aagatccacg tgcagcgtcg ggaagttgct gaagtcacaa gcccccgccg 420
ctgtggagga ggccgacgac gacgagctgc gcacctgggg ctgggacgcc agcatgccct 480
catagtagag cctgccgcgg tacgccgcca ggtcggcgta gtagacgggc gtcgccag 538

## Description

### TECHNICAL FIELD

The present invention relates to genetic markers present in genomic DNA of Gramineae plants such as Hordeum and Triticum (hereinafter, Hordeum and (or) the like), especially barley, and linked to Fusarium head blight-resistance factor (locus relating to Fusarium head blight, a gene providing the Fusarium head blight resistance), and utilization thereof.

### BACKGROUND ART

Fusarium head blight of barley is known as a serious disease, because it does not only cause quality deterioration and crop yield reduction of barley but also produce mycotoxins such as deoxynivalenol, which are toxic to human as well. The recent increase in infection with Fusarium head blight over the world leads to a strong demand for development of a cultivar resistant to Fusarium head blight.

Fusarium head blight is a disease caused by generally-existing fungus such as Fusarium graminearum, F. roseum, F. culmorum, and the like. In imperfect stage thereof, the disease-causing fungus is saprogenically parasitic to Gramineae grasses, rice straws, barley straws, organic material undersurface, etc. Under conditions such as appropriate temperature, day length, and the like, the disease-causing bacteria form perithecia. When wetted, the perithecia are swelled and cleaved thereby scattering acospores to infect Hordeum or the like such as barley. The infection to Hordeum or the like occurs in a relatively short time around its sporulation. Conidium formed in susceptible spike become secondary infection source to further spread the infection. The conidium contains viscous material that becomes suspensoid when wetted with rain or dew, and causes further spreading of the infection. The infection system of Fusarium head blight of barley is found that the first infection occurs via debris of anther remained in glume, or via degenerated organ. Then, hypha grown therein invade into a shell, or the disease-causing fungus invade via anther stuck out of the glume, and the like path. That is, anther plays a significant role in the infection of Fusarium head blight.

Moreover, some cultivars of barley are resistance to Fusarium head blight. Studies on resistance system of the Fusarium head blight-resistant cultivar of barley suggested that traits such as row type, spike length, heading date, rachis-internode length, and the like relates to the Fusarium head blight resistance (for example, non-Patent Documents 1 to 5). Moreover, it is considered that barley is not immunologically resistant to Fusarium head blight, and a relatively small number of genes of barley have a quantitative trait relating to the resistance (For instance, see non-Patent Document 6).

For breeding of excellent cultivar of agricultural crops, it has been conventionally required to cross cultivars, wild species, or the like having a target trait, actually growing many plants, selecting a plant having the target trait, and then genetically fixing the target trait. This needs a large agricultural field, large labor, and significant length of time. For example, in a case
where the target trait is the Fusarium head blight resistance, it is conventionally necessary to grow a population of plants, and evaluate each plant in terms of the Fusarium head blight resistance in order to specify the plant to select. Moreover, if the target trait is highly susceptible to an environmental factor on growth, it is difficult to judge whether or not the target trait expressed by the selected plant is the phenotype from the gene.

In order to attain needs of a shorter breeding time, less labor and a smaller agricultural field, and to ensure selection of useful gene, breeding methods based on selection using a genetic marker as an indicator have been recently getting popular. The breeding using a genetic marker allows selection in the stage of seedling by referring to genotype of the marker. This also makes it easy to check whether the tested plant has the target trait or not. Thus, the use of genetic marker can realize an efficient breeding. In order to realize a breeding method using a genetic marker, it is essential to develop a genetic marker tightly linked to the target trait.

Incidentally, many agriculturally important traits exhibit sequential variations over later generations in hybridization. Such traits are measured in quantitative units such as weight, length, and the like, and referred to as quantitative traits. The quantitative traits are generally not a trait dominated mainly by a single gene, determined by effect of multiple genes. Many traits targeted to be modified in crop breeding, such as crop yield, quality, taste, and the like, are quantitative traits.

Genetic loci of genes relating to a quantitative trait on a chromosome are referred to as QTLs (Quantitative Trait Loci). To deduce an QTL, a QTL analysis using a genetic marker located in the vicinity of the QTL is used. After the advent of genetic marker in late 1980, construction of detailed linkage maps is greatly facilitated by the use of genetic markers. QTL analysis on many organisms have been carried out based on the linkage map thus constructed.

As described above, the development of a genetic marker linked to a target trait has become possible due to highly accurate QTL analysis based on a detailed linkage map covering the whole chromosome. The use of a genetic marker obtained in this way realizes efficient breeding.

As some of the techniques relating to the genetic marker, the inventors of the present invention have suggested, for example regarding barley: 1) a technique relating to a genetic marker linked to a gene providing aluminum resistance to barley, and utilization thereof (see Patent Document 1); 2) a novel primer set for detecting, in the background of wheat, a DNA marker derived from barley chromosome, (see Patent Document 2) and utilization thereof; 3) and the like.
[Non-Patent Document 1]
   Steffen BJ. 1998. " Fusarium head blight of barley: epidemics, impact, and breeding for resistance." NBAA Tech Quart 35: 177-184.
[Non-Patent Document 2]
   Stuchlakova E and Sip V. 1996. "Resistance of Czech and Slovak winter wheat varieties to Fusarium head blight. " Genet a Slecht Praha (Genetics and plant Breeding) 32:79-94.
[Non-Patent Document 3]
   Hideo HETA, Unji HIURA, 1962. "Differences in Fusarium head blight resistance between cultivar: Research on disease resistance of barley, 13th Report" Nougaku Kenkyu 49:177-187.
[Non-Patent Document 4]
   Kazuyoshi TAKEDA, Hideo HETA, 1989. "Development of detection method of Fusarium head blight in barley and search for disease resistant cultivar" Ikushugaku zasshi 39.
[Non-Patent Document 5]
   Zhu, H., L. Gilchrist, P. Hayes, A. Kleinhofs, D. Kudruna, Z. Liu, L. Porm, B. Steffenson, T. Toojinda, P. Vivar. 1999. "Does function follow form? Principal QTLs for Fusarium head bright (FHB) resistance are coincident with QTLs for inflorescence trits and plant height in a doubled-haploide population of barley." Theor Appl Genet 99: 1221-1232.
[Non-Patent Document 6]
   Masao HORI, 1985. "Mechanism of occurrence of Fusarium head blight in wheat and barley and method of prevention", Nogyo oyobi engei 60:431-436
[Patent Document 1]
   Patent application publication, Tokukai, No. 2002-291474 (published on October 8, 2002)
[Patent Document 2]
   Patent application publication, Tokukai, No. 2003-111593 (published on April 15, 2003)
   While the resistance mechanism of the Fusarium head blight-resistant cultivar of barley has been studied, a gene relating to the Fusarium head blight-resistance in barley and the mechanism of the resistance have not been understood.
   The present invention is accomplished in view of the problems mentioned above. An object of the present invention is to find out a genetic marker existing in the genomic DNA of barley and linked to the Fusarium head blight-resistance factor (locus relating to Fusarium head blight resistance), and thereby to provide utilization thereof such as isolation of a gene relating to the Fusarium head blight resistance of barley, understanding of the mechanism of the Fusarium head blight resistance of barley, breeding of a Fusarium head blight resistant plant (Gramineae plant (e.g., Hordeum or the like such as barley)), judging whether or not a plant is a Fusarium head blight resistant plant (Gramineae plant (e.g., Hordeum or the like such as barley)), and the like.

### DISCLOSURE OF INVENTION

The inventors of the present invention, in order to attain the object, performed QTL analysis on Fusarium head blight resistance for recombinant inbred lines (RI lines (hereinafter, they are referred to as RHI populations where appropriate)) from a cross of Russia 6 (two-row, resistant) and H.E.S. 4 (six-row, susceptible), which are barley cultivars having largely different resistance against Fusarium head blight.

As a result of the QTL analysis, two QTLs relating to Fusarium head blight were found on 2H chromosome, and one QTL relating to Fusarium head blight on 5H chromosome. One of the QTLs detected on 2H chromosome was identical with the position of a well-known row type gene (vrs1). This suggested a possibility of pleiotropism of vrs1 gene. The other QTLs relating to Fusarium head blight were further investigated, thereby finding genetic marker (DNA markers etc.) respectively linked to these QTLs.

Moreover, the inventors of the present invention also performed QTL analysis on Fusarium head blight resistance for the RHI populations, recombinant inbred lines (RI lines (hereinafter, they are referred to as RH2 populations where appropriate)) from a cross of Harbin 2-row (resistant) and Turkey 6 (susceptible), and double haploid lines derived from a cross of Haruna Nijo (resistant) and H602 (susceptible) (hereinafter, double haploid lines is referred to as "DH", and these lines are referred to as "DHHS population").

As a result of the QTL analysis, one QTL was detected each on 2H and 4H chromosomes in the RHI population, 2H, 4H, and 6H chromosomes in the RI2 population, 2H, 4H, and 5H chromosomes in the DHHS population. Furthermore, genetic markers linked to these QTLs were found.

The present invention is accomplished based on the studies mentioned above. That is, a genetic marker according to the present invention is, in order to attain the object, a genetic marker, which exists in a genomic DNA of a Gramineae plant and is linked to a Fusarium head blight-resistance factor, wherein: a distance from the Fusarium head blight-resistance factor to the genetic marker is within a range of approximately 0 to 10cM.

In order to attain the object, the genetic marker according to the present invention may be arranged such that the Gramineae species is a Hordeum or Triticum.

In order to attain the object, the genetic marker according to the present invention may be arranged such that the Hordeum or Triticum is barley.

In order to attain the object, the genetic marker according to the present invention may be arrangded such that the genomic DNA is 2H chromosome.

In order to attain the object, the genetic marker according to the present invention may be arrangded such that the genetic marker is for being amplified with a first primer set that is a combination of a primer having the base sequence of S.E.Q. ID. NO. 1, and a primer having the base sequence of S.E.Q. ID. NO. 2.

In order to attain the object, the genetic marker according to the present invention may be arrangded to be distanced from the Fusarium head blight-resistance factor by approximately 0cM.

In order to attain the object, the genetic marker according to the present invention may be arrangded such that the genetic marker is for being amplified with a second primer set that is a combination of a primer having the base sequence of S.E.Q. ID. NO. 3, and a primer having the base sequence of S.E.Q. ID. NO. 4.

In order to attain the object, the genetic marker according to the present invention may be arrangded to be distanced from the Fusarium head blight-resistance factor by approximately 0.6cM.

In order to attain the object, the genetic marker according to the present invention may be arranged such that the genetic marker has the base sequence of S.E.Q. ID. NO. 8 or 9.

In order to attain the object, the genetic marker according to the present invention may be arrangded such that the genetic marker is for being amplified with a sixth primer set that is a combination of a primer having the base sequence of S.E.Q. ID. NO. 22, and a primer having the base sequence of S.E.Q. ID. NO. 23.

In order to attain the object, the genetic marker according to the present invention may be arrangded such that the genetic marker is for being amplified with a seventh primer set that is a combination of a primer having the base sequence of S.E.Q. ID. NO. 24, and a primer having the base sequence of S.E.Q. ID. NO. 25.

In order to attain the object, the genetic marker according to the present invention may be arrangded such that the genetic marker is for being amplified with an eighth primer set that is a combination of a primer having the base sequence of S.E.Q. ID. NO. 26, and a primer having the base sequence of S.E.Q. ID. NO. 27.

In order to attain the object, the genetic marker according to the present invention may be arrangded such that the genomic DNA is 5H chromosome.

In order to attain the object, the genetic marker according to the present invention may be arrangded such that the genetic marker is for being amplified with a third primer set that is a combination of a primer having the base sequence of S.E.Q. ID. NO. 5, and a primer having the base sequence of S.E.Q. ID. NO. 6.

In order to attain the object, the genetic marker according to the present invention may be arrangded to be distanced from the Fusarium head blight-resistance factor by approximately 9cM.

In order to attain the object, the genetic marker according to the present invention may be arrangded such that the genetic marker has the base sequence of S.E.Q. ID. NO. 10 or 11.

In order to attain the object, the genetic marker according to the present invention may be arrangded such that the genetic marker is for being amplified with a fourth primer set that is a combination of a primer having the base sequence of S.E.Q. ID. NO. 5, and a primer having the base sequence of S.E.Q. ID. NO. 7.

In order to attain the object, the genetic marker according to the present invention may be arrangded to be distanced from the Fusarium head blight-resistance factor by approximately 9cM.

In order to attain the object, the genetic marker according to the present invention may be arrangded such that the genetic marker has the base sequence of S.E.Q. ID. NO. 12 or 13.

In order to attain the object, the genetic marker according to the present invention may be arranged such that amplification of the genetic marker is carried out by (a) ligating MseI adaptors having the base sequences of S.E.Q. ID. NOs. 16 and 17 and EcoRI adaptors having the base sequences of S.E.Q. ID. NOs. 14 and 15 to a DNA fragment obtained by digesting a genomic DNA of a Gramineae plant with restriction enzymes MseI and EcoRI, (b) performing pre-amplification of the ligated DNA fragment by using MseI universal primer having the base sequence of S.E.Q. ID. NO. 19, and EcoRI universal primer having the base sequence of S.E.Q. ID. NO. 18, and (c) amplifying the pre-amplified fragment by using a fifth primer set that is a combination of a primer having the base sequence of S.E.Q. ID. NO. 21, and a primer having the base sequence of S.E.Q. ID. NO. 20.

In order to attain the object, the genetic marker according to the present invention may be arrangded to be distanced from the Fusarium head blight-resistance factor by approximately 2.2cM.

In order to attain the object, the genetic marker according to the present invention may be arrangded such that the genetic marker is for being amplified with a ninth primer set that is a combination of a primer having the base sequence of S.E.Q. ID. NO. 28, and a primer having the base sequence of S.E.Q. ID. NO. 29.

In order to attain the object, the genetic marker according to the present invention may be arrangded such that the genetic marker is for being amplified with a tenth primer set that is a combination of a primer having the base sequence of S.E.Q. ID. NO. 30, and a primer having the base sequence of S.E.Q. ID. NO. 31.

In order to attain the object, the genetic marker according to the present invention may be arrangded such that the genomic DNA is 4H chromosome.

In order to attain the object, the genetic marker according to the present invention may be arranged such that amplification of the genetic marker is carried out by (a) ligating MseI adaptors having the base sequences of S.E.Q. ID. NOs. 16 and 17 and EcoRI adaptors having the base sequences of S.E.Q. ID. NOs. 14 and 15 to a DNA fragment obtained by digesting a genomic DNA of a Gramineae plant with restriction enzymes MseI and EcoRI, (b) performing pre-amplification of the ligated DNA fragment by using MseI universal primer having the base sequence of S.E.Q. ID. NO. 19, and EcoRI universal primer having the base sequence of S.E.Q. ID. NO. 18, and (c) amplifying the pre-amplified fragment by using a tenth primer set that is a combination of a primer having the base sequence of S.E.Q. ID. NO. 32, and a primer having the base sequence of S.E.Q. ID. NO. 33.

In order to attain the object, the genetic marker according to the present invention may be arranged such that amplification of the genetic marker is carried out by (a) ligating MseI adaptors having the base sequences of S.E.Q. ID. NOs. 16 and 17 and EcoRI adaptors having the base sequences of S.E.Q. ID. NOs. 14 and 15 to a DNA fragment obtained by digesting a genomic DNA of a Gramineae plant with restriction enzymes MseI and EcoRI, (b) performing pre-amplification of the ligated DNA fragment by using MseI universal primer having the base sequence of S.E.Q. ID. NO. 19, and EcoRI universal primer having the base sequence of S.E.Q. ID. NO. 18, and (c) amplifying the pre-amplified fragment by using a twelfth primer set that is a combination of a primer having the base sequence of S.E.Q. ID. NO. 34, and a primer having the base sequence of S.E.Q. ID. NO. 35.

In order to attain the object, the genetic marker according to the present invention may be arranged such that amplification of the genetic marker is carried out by (a) ligating MseI adaptors having the base sequences of S.E.Q. ID. NOs. 16 and 17 and EcoRI adaptors having the base sequences of S.E.Q. ID. NOs. 14 and 15 to a DNA fragment obtained by digesting a genomic DNA of a Gramineae plant with restriction enzymes MseI and EcoRI, (b) performing pre-amplification of the ligated DNA fragment by using MseI universal primer having the base sequence of S.E.Q. ID. NO. 19, and EcoRI universal primer having the base sequence of S.E.Q. ID. NO. 18, and (c) amplifying the pre-amplified fragment by using a thirteenth primer set that is a combination of a primer having the base sequence of S.E.Q. ID. NO. 36, and a primer having the base sequence of S.E.Q. ID. NO. 37.

In order to attain the object, the genetic marker according to the present invention may be arrangded such that the genetic marker is for being amplified with a fourteenth primer set that is a combination of a primer having the base sequence of S.E.Q. ID. NO. 38, and a primer having the base sequence of S.E.Q. ID. NO. 39.

In order to attain the object, the genetic marker according to the present invention may be arrangded such that the genetic marker is for being amplified with a fifteenth primer set that is a combination of a primer having the base sequence of S.E.Q. ID. NO. 40, and a primer having the base sequence of S.E.Q. ID. NO. 41.

In order to attain the object, the genetic marker according to the present invention may be arrangded such that the genetic marker is for being amplified with a sixteenth primer set that is a combination of a primer having the base sequence of S.E.Q. ID. NO. 42, and a primer having the base sequence of S.E.Q. ID. NO. 43.

In order to attain the object, the genetic marker according to the present invention may be arrangded such that the genomic DNA is 6H chromosome.

In order to attain the object, the genetic marker according to the present invention may be arranged such that amplification of the genetic marker is carried out by (a) ligating MseI adaptors having the base sequences of S.E.Q. ID. NOs. 16 and 17 and EcoRI adaptors having the base sequences of S.E.Q. ID. NOs. 14 and 15 to a DNA fragment obtained by digesting a genomic DNA of a Gramineae plant with restriction enzymes MseI and EcoRI, (b) performing pre-amplification of the ligated DNA fragment by using MseI universal primer having the base sequence of S.E.Q. ID. NO. 19, and EcoRI universal primer having the base sequence of S.E.Q. ID. NO. 18, and (c) amplifying the pre-amplified fragment by using a seventeenth primer set that is a combination of a primer having the base sequence of S.E.Q. ID. NO. 44, and a primer having the base sequence of S.E.Q. ID. NO. 45.

In order to attain the object, the genetic marker according to the present invention may be arrangded such that the genetic marker is for being amplified with an eighteenth primer set that is a combination of a primer having the base sequence of S.E.Q. ID. NO. 46, and a primer having the base sequence of S.E.Q. ID. NO. 47.

The genetic marker according to the present invention exists in the genomic DNA of a Gramineae plant, especially Hordeum or Triticum (barley), and is linked to a Fusarium head blight-resistance factor (e.g., locus relating to the Fusarium head blight resistance). That is, it is less probable that recombination of the genetic marker and that of the locus relating to Fusarium head blight resistance take place separately. Thus, the use of the genetic marker makes it possible to acquire a DNA fragment including the Fusarium head blight-resistance factor (e.g., locus relating to the Fusarium head blight resistance), to judge whether the Fusarium head blight resistance is present or not, to judge whether a plant is a Fusarium head blight-resistant plant (Hordeum or the like such as barley).

Meanwhile, a DNA fragment isolating method according to the present invention includes isolating, by using any one of the genetic markers, a DNA fragment including a Fusarium head blight-resistance factor. The genetic markers are linked to Fusarium head blight-resistance factors (e.g., locus relating to the Fusarium head blight resistance). Thus, cloning that targets the DNA makes it possible to isolate the targeted DNA fragment easily.

A method according to the present invention for producing a Fusarium head blight-resistance plant, includes introducing, into a genomic DNA of a plant, the DNA fragment that is obtained by the DNA fragment isolating method and is including the Fusarium head blight-resistance factor.

The method according to the present invention for producing the Fusarium head blight-resistance plant may be arranged such that the plant is a Gramineae.

The method according to the present invention for producing the Fusarium head blight-resistance plant may be arranged such that the Gramineae plant is a Hordeum or Triticum.

The method according to the present invention for producing the Fusarium head blight-resistance plant may be arranged such that the Hordeum or Triticum is barley.

Any one of the methods for producing the Fusarium head blight-resistance plant includes, introducing into a plant (e.g., Gramineae plant (Hordeum or the like such as barley), a DNA fragment including the Fusarium head blight-resistance factor (e.g., locus relating to the Fusarium head resistance). Because the DNA fragment including the Fusarium head blight-resistance factor (e.g., locus relating to the Fusarium head resistance) provides the Fusarium head blight resistance, it is possible to give the Fusarium head blight resistance to a plant susceptible to Fusarium head blight, or improve a plant in terms of the Fusarium head blight resistance.

A Fusarium head blight-resistant plant according to the present invention is a Fusarium head blight-resistant plant obtained by any one of the methods for producing the Fusarium head blight-resistant plant.

The Fusarium head blight-resistant plant according to the present invention is a plant (e.g., a Gramineae plant (Hordeum or the like such as barley) obtained by the methods for producing the Fusarium head blight-resistant plant. With this Fusarium head blight-resistant plant, it is possible to attain better crop yield and quality of the crop (e.g., a Gramineae species (Hordeum or the like such as barley), and the like effect.

A method according to the present invention for judging whether a plant is a Fusarium head blight-resistant plant or not, includes detecting any one of the genetic markers according to the present invention.

The genetic marker according to the present invention is linked to the Fusarium head blight-resistance factor (e.g., locus relating to the Fusarium head resistance). By performing detection of any one of the genetic markers, it is possible to test a plant (e.g., a Gramineae species (Hordeum or the like such as barley)) to easily judge with high probability whether the plant is a Fusarium head blight-resistant plant (e.g., a Gramineae species (Hordeum or the like such as barley)) or not. Furthermore, the judgment can be performed at the stage of seedling, and thus can provide a result quickly with less labor.

A kit according to the present invention is for performing judgment by the method for judging whether a plant is a Fusarium head blight-resistant plant or not.

A kit according to the present invention is for performing judgment by the method for judging whether a plant is a Fusarium head blight-resistant plant or not can be developed by providing the kit with a reagent(s), an enzyme(s), or the like necessary for performing the method for judging whether a plant is the Fusarium head blight-resistant plant or not. With this kit for judging whether a plant is a Fusarium head blight-resistant plant or not, it is possible to more easily judge (determine) whether a plant is a Fusarium head blight-resistant plant or not.

A gene detecting apparatus according to the present invention is arranged such that at least one of the genetic markers is fixed.

The gene detecting apparatus according to the present invention is reacted with a probe prepared from a plant to be tested, and a signal emitted from the reaction is detected, whereby it is possible to detect a plurality of genetic markers at the same time easily. Therefore, the gene detecting apparatus can be used as a means for detecting a polymorphism of a genetic marker according to the present invention. For example, the gene detecting apparatus is effective to easily perform the judgment whether a plant (e.g., Gramineae plant) is a Fusarium head blight-resistant plant or not.

A primer population according to the present invention is for use in detecting the genetic markers as set forth in Claims 1 to 33, and includes at least two of primers having the base sequences of S.E.Q. ID. NOs. 1 to 7, and 20 to 47.

With any one of the primer set according to the present invention, it is possible to amplify the genetic marker according to the present invention by amplification reaction such as PCR or the like. Thus, by performing the detection of a polymorphism of the genetic marker according to the present invention, for example, it is possible to easily judge whether a plant is a Fusarium head blight-resistant plant (Gramineae plant) or not.

For a fuller understanding of the nature and advantages of the invention, reference should be made to the ensuing detailed description taken in conjunction with the accompanying drawings.

### BRIEF DESCRIPTION OF DRAWINGS

FIG 1(a) is a view illustrating a positional relationship between DNA markers (MM314, FM677) on 2H chromosome and 2H-2 factor.
FIG 1(b) is a view illustrating a positional relationship between DNA markers (FM426, MM1057) on 5H chromosome and 5H-1 factor.
FIG. 2(a) is a view illustrating the whole base sequence (SEQ. ID. NO.8) of FM677 of resistant type (Harbin 2).
FIG. 2(b) is a view illustrating the whole base sequence (SEQ. ID. NO.9) of FM677 of susceptible type (Turkey 6).
FIG. 2(c) is a view illustrating the whole base sequence (SEQ. ID. NO.10) of FM426 of resistant type (Russia 6).
FIG. 2(d) is a view illustrating the whole base sequence (SEQ. ID. NO.11) of FM426 of susceptible type (H.E.S.4).
FIG. 3(a) is a graph illustrating a result of QTL analysis (CIM) of 2H chromosome of barley regarding Fusarium head blight resistance of barley.
FIG. 3(b) is a graph illustrating a result of QTL analysis (CIM) of 5H chromosome of barley regarding Fusarium head blight resistance of barley.
FIG. 4 is a view illustrating PCR reaction conditions in Example 2.
FIG. 5(a) is a photograph showing a result of electrophoresis of a PCR product obtained by PCR using primers of S.E.Q. ID. NOs. 1 and 2, and using genomic DNA from Russia 6, H.E.S.4 and RI lines as templates.
FIG. 5(b) is a photograph showing a result of electrophoresis of a PCR product, which is obtained by PCR using primers of S.E.Q. ID. NOs. 5 and 7, and using genomic DNA from Russia 6, H.E.S.4 and RI lines as templates, and then subjected to HhaI digestion.
FIG. 6 is a histogram illustrating a relationship between Fusarium head blight resistance scores and results of judgments whether barley is Fusarium head blight-resistant or not using the DNA markers as indicators.
FIG. 7 is an electrophoresis view illustrating a result of detection of polymorphism of a genetic marker "MM 1057" by AFLP analysis.
FIG. 8 is an electrophoresis view illustrating a result of detection of polymorphism of a genetic marker "MmtgaEatc128" in Example 5.
FIG. 9 is an electrophoresis view illustrating a result of detection of polymorphism of a genetic marker "FMgcgEatc530" in Example 6.
FIG. 10 is an electrophoresis view illustrating a result of detection of polymorphism of a genetic marker "FXLRRfor_XLRRrev119" in Example 7.
FIG. 11 is an electrophoresis view illustrating a result of detection of polymorphism of a genetic marker "FmacgEcgt288" in Example 8.
FIG. 12 is an electrophoresis view illustrating a result of detection of polymorphism of a genetic marker "HVM67" in Example 9.
FIG. 13 is an electrophoresis view illustrating a result of detection of polymorphism of a genetic marker "FMataEagc408" in Example 10.
FIG. 14 is an electrophoresis view illustrating a result of detection of polymorphism of a genetic marker "HVM11" in Example 11.
FIG. 15 is an electrophoresis view illustrating a result of detection of polymorphism of a genetic marker "Bmag125" in Example 12.
FIG. 16 is an electrophoresis view illustrating a result of detection of polymorphism of a genetic marker "k04002" in Example 13.
FIG. 17 is an electrophoresis view illustrating a result of detection of polymorphism of a genetic marker "k05042" in Example 14.
FIG. 18 is an electrophoresis view illustrating a result of detection of polymorphism of a genetic marker "k03289" in Example 15.
FIG. 19 is an electrophoresis view illustrating a result of detection of polymorphism of a genetic marker "HvLOX" in Example 16.
FIG. 20 is an electrophoresis view illustrating a result of detection of polymorphism of a genetic marker "k00835" in Example 17.
FIG. 21 is a view illustrating SNPs between Haruna Nijo and H602, and base sequences in the vicinity of the SNPs in the use of the genetic marker k05042.
FIG. 22 is a view illustrating SNPs between Haruna Nijo and H602, and base sequences in the vicinity of the SNPs in the use of the genetic marker k03289.

### BEST MODE FOR CARRYING OUT THE INVENTION

### [Embodiment 1]

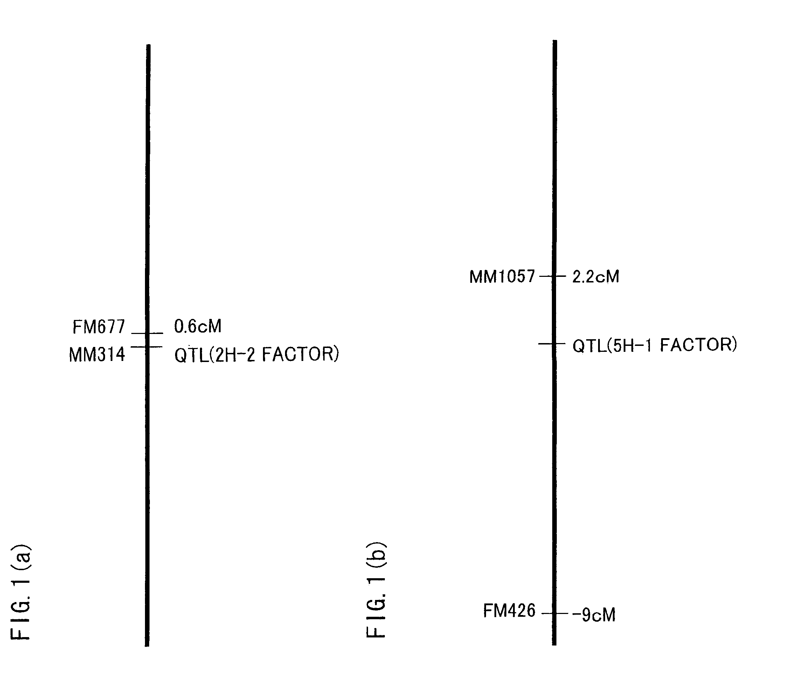

One embodiment according to the present invention is described below, referring to FIGS. 1 to 7. It should be noted that the present invention is not limited to this.

The present invention relates to a genetic marker (e.g., DNA marker) linked to Fusarium head blight-resistance factor, and utilization thereof, which are described below respectively.

### [1. Genetic Marker According to the Present Invention]

The genetic marker according to the present invention exists in a genomic DNA of a Gramineae species (Hordeum and the like (such as barley)), and is linked to a Fusarium head blight-resistance factor.

In the present invention, the "Hordeum and the like" encompasses: barley, wheat, rye, triticale, and the like.

### <1-1Fusarium Head Blight and Fusarium Head Blight-Resistance Factor>

"Fusarium head blight" is a disease caused by infection of Fusarium spp. to Hordeum and the like, as described above. Fusarium head blight is a serious disease because not only poor ripening and low crop yield are caused but also mycotoxin (e.g., deoxynivalenol) is produced in Fusarium head blight, which mycotoxin would cause food toxining in human and animals fed with the Hordeum and the like in which this disease occurs.

There are some barley cultivar having resistance to Fusarium head blight. The mechanism of the Fusarium head blight resistance has not been understood in details, but the researches so far suggest that traits such as row type, spike length, heading date, rachis-internode length, relates to the Fusarium head blight resistance. Moreover, it is said that barley has no immunological resistance to the Fusarium head blight and a relatively small number of genes are contributed as quantitative trait loci relating to the resistance. In the present invention, the gene having a trait that gives the Fusarium head blight resistance, or a locus relating to the Fusarium head blight resistance is referred to as "Fusarium head blight-resistance factor".

QTL analysis is suitable as a means for searching for the Fusarium head blight-resistance factor. As described above, the inventors of the present invention performed QTL analysis for Fusarium head blight resistance of barley in order to search for Fusarium head blight-resistance factor. Specifically, the QTL analysis is performed as follows. The QTL analysis was performed with recombinant inbred lines (RI lines) that were derived from a cross of Russia 6 (two-row, resistant) and H.E.S. 4 (six-row, susceptible), which were barley cultivars having largely different resistances against Fusarium head blight. The Fusarium head blight resistance was evaluated by a modified "Cut-Spike Test" and scored by 0 (resistant) to 10 (susceptible) (see Non-Patent Document 4). Algorisms used in the analysis were simple interval mapping (SIM) and composite interval mapping (CIM), which were run by analysis software, MAPMARKER/QTL and QTL Cartographer, respectively.

The QTL analysis detected two QTLs on 2H chromosome of barley and one QTL on 5H chromosome of barley. One of the QTLs on 2H chromosome of barley was identical with the position of a well known row type gene (vrs1). This suggests a possibility of pleiotropism of the vrs1 gene. This result supports the alleged relevance of the row type to the Fusarium head blight resistance. However, the Fusarium head blight-resistance factor, which is expected to exist at a loci other than the QTL at which the vrs1 gene is unknown. So, it is considered that there is a Fusarium head blight-resistance factor other than the row type gene. Here, the Fusarium head blight-resistance factor located at the other one of the QTLs on 2H chromosome of barley than the vrs1 loci is named as "2H-2 factor". Meanwhile, the Fusarium head blight-resistance factor located at the QTL on 5H chromosome of barley is named as "5H-1 factor". The Fusarium head blight-resistance factor located at the one of the QTLs on 2H chromosome of barley that is same as the vrs1 locus is referred to as "2H-1 factor" for easy explanation.

### <1-2. Genetic Marker>

Based on information from a high-density linkage map prepared by the inventors of the present invention, genetic markers located in the vicinity of the loci of the Fusarium head blight-resistance 2H-2 factor and 5H-1 factor were searched for. As a result, genetic markers that are tightly linked especially to Fusarium head blight-resistance factors, that are genetic markers according to the present invention, were found out. The genetic markers were cloned to find sequences thereof (STSs). Based on information of the STSs, primers from which the genetic markers are to be amplified are designed.

More specifically, MM314 and FM677 were found as the genetic markers linked to the 2H-2 factor.

MM314 is a genetic marker to be amplified using barley genomic DNA as a template and a first primer set, which is a combination of a primer having the base sequence of AGAGATCCCTGCTCAGCTTG (S.E.Q. ID. NO. 1) and a primer having the base sequence of TCGTATTAAGGCCGCATAGG (S.E.Q. ID. NO. 2). The locus of MM314 is distanced from the locus of the 2H-2 factor by approximately 0 centiMorgan (hereinafter, cM). That is, the locus of MM314 almost overlaps with the locus of the 2H-2 factor.

Meanwhile, FM677 is a genetic marker to be amplified using barley genomic DNA as a template and a second primer set, which is a combination of a primer having the base sequence of GCACGTAGCGTTCAACATCA (S.E.Q. ID. NO. 3) and a primer having the base sequence of AACTTTTCCCAACCCTTTCC (S.E.Q. ID. NO. 4). The locus of FM677 is distanced from the locus of the 2H-2 factor by approximately 0.6 cM.

FM677, which is amplified with the second primer set, has a resistant type and a susceptible type to Fusarium head blight. The base sequences of the resistant type and susceptible type are indicated in S.E.Q. ID. NOs. 8 and 9, respectively.

As to the genetic markers linked to 5H-1 factor, FM426 and MM 1057 were found.

FM426 is a genetic marker to be amplified using barley genomic DNA as a template and a third primer set, which is a combination of a primer having the base sequence of CCGTGTGTCGTCTAGGTCAA (S.E.Q. ID. NO. 5) and a primer having the base sequence of CAACTTTGGTGGGACGTAGG (S.E.Q. ID. NO. 6), or a fourth primer set, which is a combination of a primer having the base sequence of CCGTGTGTCGTCTAGGTCAA (S.E.Q. ID. NO. 5) and a primer having the base sequence of GTTTTCGCCATCACTCTTCC (S.E.Q. ID. NO. 7). The locus of FM426 is distanced from the locus of the 5H-1 factor by approximately 9 cM.

FM426, which is amplified with the third primer set, has a resistant type and a susceptible type to Fusarium head blight. The base sequences of the resistant type and susceptible type are indicated in S.E.Q. ID. NOs. 10 and 11, respectively.

FM426, which is amplified with the fourth primer set, has a resistant type and a susceptible type to Fusarium head blight. The base sequences of the resistant type and susceptible type are indicated in S.E.Q. ID. NOs. 12 and 13, respectively.

MM 1057 is located at a locus distanced by approximately 2.2 cM from the locus of the 5H-1 factor.

FIG. 1 illustrates the positional relationships between the genetic markers and the Fusarium head blight-resistance factors (2H-2 factor, 5H-1 factor). In FIG. 1(a), the positional relationship between the genetic markers and the 2H-2 factor on 2H chromosome is illustrated, meanwhile the positional relationship between the genetic markers and the 5H-1 factor on 5H chromosome is illustrated in FIG. 1(b). The left side of FIGS. 1 is the short arm (5' end) of the chromosomes, while the right side thereof is the long arm (3' end) thereof. Locations on the short-arm side with respect to the locus of the 2H-2 factor or the 5H-1 factor are indicated with negative values and location on the long-arm side are indicated with positive values.

According to FIG. 1(a), MM314 is at the locus (approximately 0cM) substantially overlapping the 2H-2 factor. FM677 is located at the locus distanced by approximately 0.6cM from the 2H-2 factor on the long-arm side. According to FIG. 1 (b), FM426 is located at the locus distanced by approximately 9cM (approximately -9cM) from the 5H-1 factor on the short-arm side, whereas MM 1057 is located at the locus distanced by approximately 2.2cM from the 5H-1 factor on the long-arm side.

Here, morgan (M) is explained. 1 morgan (M), which is a unit of a distance on a chromosome, corresponds to such a probability that one crossover event occurs during one meiosis on average. For example, 2.2 cM indicates that recombination between the Fusarium head blight-resistance factor and the genetic marker occurs 22/1000 times per chromatid on average. That is, it is indicated that recombination percentage is approximately 2.2% in this case.

Incidentally, the genomic DNA used as the template in the amplification can be extracted from a plant tissue of barley in conventional and well-known methods. Specifically, One suitable example of such methods is a generally-used method for extracting a genomic DNA from a plant tissue (see Murray, M. G. and W. F. Thompson (1980) Nucleic Acids Res. 8: 4321-4325, etc.) Moreover, the genomic DNA can be extracted from any tissues such as roots, stems, leaves, reproductive organs, and the like, which constitute the plant tissue of the barley. Moreover, in some cases, the genomic DNA can be extracted from callus of barley. The reproductive organs encompasses flower organ (including male/female reproductive organs) and seeds. The genomic DNA is extracted, e.g., from a leaf of barley during (seedling period). This is because trituration of the tissue is relatively easy, a mixture ratio of impurity such as polysaccarides is relatively low, and only short time is necessary to grow a plant from a seed to seedling.

The amplification using the genomic DNA of barley as a template and the combination of the primers can be performed by a conventional and well-known DNA amplification method. In general, a PCR method (polymerase chain reaction method), or a modified PCR method is used. There is no particular limitation as to conditions under with the PCR method or the modified PCR method is performed. It is possible to perform the PCR method or the modified PCR method under conditions similar to generally adopted ones.

The use of a genetic marker according to the present invention makes it possible to isolate DNA fragments including the Fusarium head blight-resistance factor (2H-2 factor, 5H-1 factor). The DNA fragments can be utilized for finding a gene relating to the Fusarium head blight resistance of barley, and for understanding the mechanism of the Fusarium head blight resistance. Moreover, by introducing the DNA fragment of the resistance gene into a plant (e.g., a Hordeum or the like such as barley), it is possible to produce (breed) a Fusarium head blight-resistant plant.

Moreover, the genetic markers are linked to the Fusarium head blight-resistance factors (2H-2 factor, 5H-1 factor). Thus, it is possible to test a plant (e.g., Hordeum or the like such as barley) to judge whether the plant (e.g., Hordeum or the like such as barley) has a Fusarium head blight-resistance factor or not, by detecting for polymorphism of any of the genetic markers in the genomic DNA of the plant. Similarly, because the genetic markers are linked to the Fusarium head blight-resistance factors (2H-2 factor, 5H-1 factor), it is possible to test a plant (e.g., Hordeum or the like such as barley) to judge whether the plant (e.g., Hordeum or the like such as barley) is a Fusarium head blight-resistant plant or not, by detecting for polymorphism of any of the genetic markers in the genomic DNA of the plant. A kit developed by comprising a primer for amplification of any of the genetic markers or a DNA microarray to which any of the genetic markers is fixed can be provided as a kit for judging whether or not a Fusarium head blight-resistance factor are present in a plant (e.g., Hordeum or the like such as barley), or a kit for judging whether a plant is a Fusarium head blight-resistant plant or not.

As described above, the genetic marker according to the present invention is widely applicable to various uses evidently. Examples of the uses of the genetic markers according to the present invention will be explained later in detail.

### [2. Use of Genetic Marker according to the Present Invention]

### <2-1. Isolation Method of DNA Fragment Including Fusarium Head Blight-Resistance Factors according to the Present Invention]

As described above, the genetic markers (MM314, FM677) according to the present invention are linked to the 2H-2 factors among the Fusarium head blight-resistance factors. On the other hand, the other genetic markers (FM426, MM1057) are linked to the 5H-1 factor among the Fusarium head blight-resistance factors. Thus, the use of the genetic marker MM314 or FM 677 makes it possible to isolate DNA fragments including the 2H-2 factor, whereas the use of the genetic marker FM426 or MM 1057 makes it possible to isolate DNA fragments including the 5H-1 factor.

The isolation of the DNA fragment including the Fusarium head blight-resistance factor by using the genetic markers according to the present invention is not particularly limited. For example, the isolation may be performed as follows.

For Barley, one BAC library of genomic DNA has been developed while some other BAC libraries thereof are under development. By using such a BAC library, identification of a BAC clone including the genetic marker of the present invention can be performed with the Fusarium head blight-resistance factors and the genetic markers according to the present invention by using a conventional and well-known map based cloning method. Based on the identification, it is possible to prepare a contiguous sequences of BAC, thereby identifying its base sequence. This finally leads to finding of the Fusarium head blight-resistance factors.

In isolation of the DNA fragment including a Fusarium head blight-resistance factor, which is performed by the method mentioned above or another method, it is preferable that a genetic marker whose locus is closer to a targeted Fusarium head blight-resistance factor be selected to use. This reduces a possibility of recombination between the targeted Fusarium head blight-resistance factor and the genetic marker, and ensures more reliable isolation of the targeted Fusarium head blight-resistance factor. For example, MM314 (distanced from 2H-2 by approximately 0cM) is more suitable than FM677 (distanced from 2H-2 by approximately 0.6 cM) for isolating the 2H-2 factor. Moreover, MM 1057 (distanced from 5H-1 by approximately 2.2cM) is more suitable than FM426 (distanced from 5H-1 by approximately 9cM) for isolating the 5H-1 factor.

### <2-2. Method According to Present Invention for Producing Fusarium Head Blight-resistant Plant (e.g., Gramineae plant (Hordeum or the like such as barley)), and Fusarium Head Blight-resistant Plant (e.g., Gramineae plant (Hordeum or the like such as barley)) according to the Present Invention Produced by same Method.>

It is possible to produce a Fusarium head blight-resistant plant by introducing, into a genomic DNA of a plant, the DNA fragment including the Fusarium head blight-resistance factor, which fragment is obtained by the method according to the present invention for isolating the DNA fragment including the Fusarium head blight-resistance factor. Especially, it is possible to produce a Fusarium head blight-resistant Gramineae plant by introducing the DNA fragment into a genomic DNA of a Gramineae plant. Furthermore, it is possible to produce a Fusarium head blight-resistant barley by introducing the DNA fragment into a genomic DNA of barley.

More specifically, the production of the Fusarium head blight-resistant plant (e.g., a Gramineae plant (Hordeum or the like such as barley)) may be performed by introducing the DNA fragment into the genomic DNA by a well-known method (e.g., agrobacterium method or particle gun method). In this way, a Fusarium head blight-resistant cultivar can be obtained. For example, Sonia Tingay et al., in the Plant Journal(1997) 11(6), 1369-1376, discloses a method for transformation of barley using Agrobacterium tumefaciens. It is possible to adopt this method in order to produce a transformant barley.

Besides barley, the DNA fragments may be introduced to other plants, for example, food plants such as fruits, vegetables; horticultural plants such as flowers, woods, and the other useful plants; industrial plants; animal-feed plants, and the like. For example, Nogakudaijiten (Agricultural Dictionary) modified 4th edition, page 8 to 16 (Yokendo, published on April 30, 1997) can be referred for some specific examples of the food crops, horticultural crops, industrial crops, and feed crops. Because Fusarium head blight is a problem associated with Hordeum and the like crops (such as barley, wheat, rye, triticale, and the like), the present invention is more effectively applicable to these crops.

While it is possible to produce a Fusarium head blight-resistant plant by introducing either a DNA fragment including the 2H-2 factor or a DNA fragment including the 5H-1 factor in a plant (e.g., a Gramineae plant (Hordeum or the like such as barley)), it is more preferable to introduce both the DNA fragments (the DNA fragment including the 2H-2 factor and the DNA fragment including the 5H-1 factor) into the genomic DNA of the plant, because this gives the plant a higher Fusarium head blight resistance.

It is possible to give a plant (Hordeum or the like such as barley) a higher Fusarium head blight resistance by introducing the 2H-1 factor (which existed on 2H chromosome as well as the 2H-2 factor) in addition to the DNA fragment including the 2H-2 factor or the DNA fragment including the 5H-1 factor, compared with that of the plant to which only the DNA fragment including the 2H-2 factor or the DNA fragment including the 5H-1 factor is introduced. Furthermore, it is possible to give a plant (Hordeum or the like such as barley) a higher Fusarium head blight resistance by introducing the 2H-1 factor in addition to the DNA fragment including the 2H-2 factor and the DNA fragment including the 5H-1 factor, compared with that of the plant to which only the DNA fragment including the 2H-2 factor and the DNA fragment including the 5H-1 factor are introduced.

Using analysis software (MAPMARKER/QTL and QTL Cartographer), the inventors of the present invention worked out and compared Fusarium head blight resistances obtained in the cases where the DNA fragment including 2H-2 factor were the DNA fragment including 5H-1 factor were introduced solely and in combination, and the three kinds of DNA fragments mentioned above were introduced. One example of results of the MAPMARKER/QTL analysis is given in Table 1 below.

**Table 1**

| | Effect to Improve FHBR |
|---|---|
| 2H-2 factor | 1.8076 |
| 5H-1 factor | 1.1733 |
| 2H-2 factor + 5H-1 factor | 2.7338 |
| 2H-1 factor (vrs1 gene) | 1.3234 |
| 2H-2 factor | 1.5319 |
| 5H-1 factor | 1.6083 |
| 2H-1factor + 2H-2 factor + 5H-1 factor | 3.6759 |

| | |
|---|---|
| Abbreviation: FHBR stands for Fusarium head blight resistance | |

Table 1 shows how the introductions of the DNA fragment including the 2H-2 factor and/or the DNA fragment including the 5H-1 factor improved the Fusarium head blight resistance of the barley. Further, Table 1 shows how the introductions of one and all of the DNA fragment including the 2H-1 factor, the DNA fragment including the 2H-2 factor, and the DNA fragment including the 5H-1 factor improved the Fusarium head blight resistance of the barley.

According to Table 1, the effect of the introduction of the DNA fragment including the 2H-2 factor alone to improve the Fusarium head blight resistance of the barley was 1.8076, and the effect of the introduction of the DNA fragment including the 5H-1 factor alone to improve the Fusarium head blight resistance of the barley was 1.1733, whereas the effect of the introduction of both of these DNA fragments to improve the Fusarium head blight resistance of the barley was 2.7338. Thus, from the results shown on Table 1, it is clearly understood that the introductions of both the DNA fragments causes a synergic improvement. Further, it is understood that the introductions of the three DNA fragments including Fusarium head blight-resistance factors including the 2H-1 factor more remarkably improves the Fusarium head blight resistance (the effect of the introduction of the DNA fragment including the 2H-1 factor to improve the Fusarium head blight resistance of the barley was 1.3234, the effect of the introduction of the DNA fragment including the 2H-2 factor to improve the Fusarium head blight resistance of the barley was 1.5319, the effect of the introduction of the DNA fragment including the 5H-1 factor to improve the Fusarium head blight resistance of the barley was 1.6083, and the effect of the introduction of all the three DNA fragments was 3.6759). In Table 1, the higher value of the effect to improve FHBR indicates the effect is higher.

With the Fusarium. head blight-resistant plant, such as the Fusarium head blight-resistant barley, obtained by the method according to the present invention, it becomes possible to effectively avoid the Fusarium head blight-causing quality deterioration and crop yield reduction, and damages to human and domestic animals caused when they are fed with the plant infected with the Fusarium head blight. Thus, the Fusarium head blight-resistant plant is quite effective in the agricultural and livestock industries and the like. Further, the Fusarium head blight-resistant plant can alleviate food shortage problem.

The terms "Fusarium head blight-resistant plant", "Fusarium head blight-resistant Gramineae plant", and "Fusarium head blight-resistant barley" encompass a plant (e.g., Gramineae plant such as barley) that originally has no Fusarium head blight resistance at all but is bred to have the Fusarium head blight resistance, and a plant (e.g., Gramineae plant such as barley) that originally has the Fusarium head blight resistance, but is bred to have an improved Fusarium head blight resistance.

### <2-3 Method According to the Present Invention for Detecting Genetic Marker Linked to the Fusarium Head Blight-Resistance Factor>

As mentioned above, the inventors of the present invention found the genetic markers linked to the Fusarium head blight-resistance factor, and designed primers for amplification of the genetic markers.

Specifically, MM314 is a genetic marker that is amplified using a barley genomic DNA as a template and the first primer set that is a combination of the primer having the base sequence of AGAGATCCCTGCTCAGCTTG (S.E.Q. ID. NO. 1) and the primer having the base sequence of TCGTATTAAGGCCGCATAGG (S.E.Q. ID. NO. 2). MM314 is linked to 2H-2 factor.

FM677 is a genetic marker that is amplified using a barley genomic DNA as a template and the second primer set that is a combination of the primer having the base sequence of GCACGTAGCGTTCAACATCA (S.E.Q. ID. NO. 3) and the primer having the base sequence of AACTTTTCCCAACCCTTTCC (S.E.Q. ID. NO. 4). FM677 is linked to 2H-2 factor.

FM426 is a genetic marker that is amplified using a barley genomic DNA as a template and the third primer set that is a combination of the primer having the base sequence of CCGTGTGTCGTCTAGGTCAA (S.E.Q. ID. NO. 5) and the primer having the base sequence of CAACTTTGGTGGGACGTAGG (S.E.Q. ID. NO. 6), or the fourth primer set that is a combination of the primer having the base sequence of CCGTGTGTCGTCTAGGTCAA (S.E.Q. ID. NO. 5) and the primer having the base sequence of GTTTTCGCCATCACTCTTCC (S.E.Q. ID. NO. 7). FM426 is linked to 5H-1 factor.

By performing amplification reaction using any one of the first to fourth primer sets, it is possible to detect the genetic marker linked to the Fusarium head blight-resistance factor (2H-2 factor or 5H-1 factor). Especially, the use of the first primer set makes it possible to detect MM314 linked to the 2H-2 factor, whereas the use of the second primer set allows to detect FM 677 linked to the 2H-2 factor. The use of the third or fourth primer set makes it possible to detect FM426 linked to 5H-1 factor.

Therefore, the use of at least one of the four primer sets allows to detect the corresponding one(s) of the genetic markers. It is preferable to use a combination of the first and/or the second primer set, and the third and/or fourth primer set, because this makes it possible to detect the 2H-2 factor and 5H-1 factor.

By performing the detection of the genetic marker linked to the Fusarium head blight-resistance factor, it is possible to judge whether the Fusarium head blight-resistance factor is present or not, and whether a plant has the Fusarium head blight resistance or not, as described below. A specific method for detecting the genetic marker linked to the Fusarium head blight-resistance factor will be explained in Sections "Method according to the Present Invention for Judging Whether Fusarium Head Blight-Resistance Factor is Present or not", and "Method for Judging whether Plant has Fusarium Head Blight Resistance or not".

### (2-3-1Method according to the Present Invention for Judging Whether Fusarium Head Blight-Resistance Factor is Present or not)

The method according to the present invention for judging whether a Fusarium head blight-resistance factor is present or not (hereinafter, this method is referred to as "the present resistance factor judging method") is performed by detecting a polymorphism of at least one of the genetic markers detected by the method according to the present invention for detecting the genetic marker (MM314, FM677, FM426, or MM1057) linked to the Fusarium head blight-resistance factor.
The genetic markers are linked to the Fusarium head blight-resistance factors. Thus, when a plant (Hordeum or the like such as barley) that is tested has a Fusarium head blight-resistant genetic marker in its genomic DNA, it is possible to judge with high probability whether the plant has a Fusarium head blight-resistance factor or not. It is needless to say that whether the 2H-2 factor is present or not can be judged by detecting the polymorphism of MM314 or FM677, and the 5H-1 factor can be detected by detecting the polymorphism of FM426 or MM1057.

The present resistance factor judging method may be performed on a conventional barley or a barley mutated with a chemical or the like, or a barley (plant) bred by crossing. Furthermore, the present resistance factor judging method is applicable to barley and plant described in Section 2-2 to which a DNA fragment including the Fusarium head blight-resistance factor(s) (2H-2 factor, 5H-1 factor) is introduced. Thus, the present resistance factor judging method is applicable to wide variations of plants (e.g., Hordeum and the like such as barley).

The accuracy (probability) in judgment performed by the present resistance factor judging method is as follows. Recombination between the genetic marker distanced by 2.2cM from the Fusarium head blight-resistance factor and the Fusarium head blight-resistance factor occurs 22/1000 times per chromatid on average. That is, the recombination occurs with approximately 2.2% probability. Thus, when a Fusarium head blight-resistant type genetic marker is detected from among the genetic markers, it can be deduced with 97.8% probability that the Fusarium head blight-resistance factor is present. Thus, the shorter distance between the Fusarium head blight-resistance factor and the genetic marker leads to a higher probability in judging whether the Fusarium head blight-resistance factor is present or not.

On this reason, the use of any of the genetic markers for performing detection of a polymorphism makes it possible to judge whether the Fusarium head blight-resistance factor is present or not. However, it is preferable to arranged to detect a genetic marker having its locus closer to the corresponding Fusarium head blight-resistance factor. That is, if the present resistance factor judging method was to detect the 2H-2 factor, MM314 (distanced by approximately 0cM from the 2H-2 factor) is more preferable than FM677 (distanced by approximately 0.6cM from the 2H-2 factor). Especially, MM314 is located at the almost same locus as the 2H-2 factor. Thus, if MM314 was detected, it could be judged with substantially 100% accuracy that the Fusarium head blight-resistance factor is present. Moreover, for detecting 5H-1 factor, MM1057 (distanced by approximately 2.2cM from the 5H-1 factor) is more preferable than FM426 (distanced by approximately 9cM from the 5H-1 factor).

This detecting method may be arranged to detect polymorphism of a plurality of genetic markers. Especially, the accuracy (probability) of the judgment can be improved by arranging the method to detect polymorphism of genetic markers located sandwiching the Fusarium head blight-resistance factor. One combination of genetic markers sandwiching the 2H-2 factor is a pair of MM314 and FM677 (see FIG. 1(a)). One combination of genetic markers sandwiching the 5H-1 factor is a pair of FM426 and MM1057 (see FIG. 1(b)).

The accuracy (probability) of the present resistance factor judging method is explained below in more details, discussing, by way of example, cases where the polymorphism is detected by solely using FM426 and MM1057, and a case where the polymorphisms of both FM426 and MM1057. The locus of FM 426 is distanced by approximately 9cM on the short-arm side from the 5H-1 factor. The accuracy (probability) of this judging method in the case where the polymorphism of FM 426 is detected using solely FM 426 is (1 - 90 ÷ 1000) × 100 = approximately 91%. On the other hand, the locus of MM1057 is distanced by approximately 2.2cM on the long-arm side from the 5H-1 factor. The accuracy (probability) of this judging method in the case where the polymorphism of MM 1057 is detected using solely MM1057 is 97.8%, which is calculated in a similar manner. The accuracy (probability) of this judging method in the case where the polymorphisms of both the genetic markers are detected using of the genetic markers is (1 - (90 ÷ 1000) × (22 ÷ 1000)) × 100 = 99.802%. Thus, the judgment whether 5H-1 is present or not can be performed with high probability. Therefore, in the present resistance factor judging method it is preferable to be performed by detecting genetic markers whose loci sandwiching the Fusarium head blight-resistance factor.

The detection of the genetic marker(s) in the present resistance factor judging method may be performed by using a conventional and well-known DNA amplification method. In general, a PCR method (polymerase chain reaction method), or a modified PCR method is used. There is no particular limitation as to conditions under with the PCR method or the modified PCR method is performed. It is possible to perform the PCR method or the modified PCR method under conditions similar to generally adopted ones. By judging whether the genetic marker obtained by the amplification method is resistant type or susceptible type, it is possible to test the plant (Hordeum or the like such as barley) to judge whether the plant has the Fusarium head blight-resistance factor or not.

The genetic markers according to the present invention can be amplified using the following combinations of primers.

MM314 can be amplified by using the genomic DNA as a template, and the first primer set that is the combination of the primer having the base sequence of AGAGATCCCTGCTCAGCTTG (S.E.Q. ID. NO. 1) and the primer having the base sequence of TCGTATTAAGGCCGCATAGG (S.E.Q. ID. NO. 2).

FM677 can be amplified by using the genomic DNA as a template, and the second primer set that is the combination of the primer having the base sequence of GCACGTAGCGTTCAACATCA (S.E.Q. ID. NO. 3) and the primer having the base sequence of AACTTTTCCCAACCCTTTCC (S.E.Q. ID. NO. 4).

FM426 can be amplified by using the barley genomic DNA as a template, and the third primer set that is the combination of the primer having the base sequence of CCGTGTGTCGTCTAGGTCAA (S.E.Q. ID. NO. 5) and the primer having the base sequence of CAACTTTGGTGGGACGTAGG (S.E.Q. ID. NO. 6), or the fourth primer set that is the combination of the primer having the base sequence of CCGTGTGTCGTCTAGGTCAA (S.E.Q. ID. NO. 5) and the primer having the base sequence of GTTTTCGCCATCACTCTTCC (S.E.Q. ID. NO. 7).

Table 2 below shows DNA fragment lengths (amplification fragment sizes) attained by the amplification reactions using the respective combinations of the primers.

**Table 2**

| | | Amplified Fragment Size (bp) | |
|---|---|---|---|
| | Combination of Primers | Resistant (Russia 6 etc.) | Susceptible (H.E.S.4 etc.) |
| MM314 | S.E.Q NOs. 1&2 | > 524 | >581 |
| FM677 | S.E.Q NOs. 3&4 | 208 | 208 |
| FM426 | S.E.Q NOs. 5&6 | 335 | 335 |
| FM426 | S.E.Q NOs. 5&7 | 254 | 254 |

Among the DNA fragments attained by amplification using the combinations of the primers, there are a resistant type (polymorphism) attained by amplification using, as the template, a Fusarium head blight-resistant cultivar of barley such as Russia 6, Harbin 2, or the like, and a susceptible type (polymorphism) attained by amplification using, as the template, a Fusarium head blight-susceptible cultivar of barley such as H.E.S. 4, Turkey 6, or the like. When a genetic marker of the resistant type (i.e., MM314, FM677, FM426, or MM1057) is detected by the present resistance factor judging method, then it is judged that the tested plant (e.g., a Gramineae plant (Hordeum or the like such as barley)) has the Fusarium head blight-resistance factor. On the other hand, when a genetic marker of the susceptible type (i.e., MM314, FM677, FM426, or MM1057) is detected by the present resistance factor judging method, then it is judged that the tested plant (e.g., a Gramineae plant (Hordeum or the like such as barley)) has no Fusarium head blight-resistance factor.

In case of a genetic marker (e.g., MM314) whose resistant type and susceptible type are amplified to have different fragment sizes, the judgment whether the amplified M314 is of the resistant type or susceptible type can be made easily by agarose gel electrophoresis or the like by which the fragment size of the amplified M314 is compared. That is, if the amplified fragment was > 524bp in fragment size, it would be found that MM314 of the resistant type was amplified, and judged that the tested plant (e.g., a Gramineae plant (Hordeum or the like such as barley) has the Fusarium head blight-resistance factor. On the other hand, if the amplified fragment was > 581 bp in fragment size, it would be found that MM314 of the susceptible type was amplified, and judged that the tested plant (e.g., a Gramineae plant (Hordeum or the like such as barley) has no Fusarium head blight-resistance factor. This will be further described in Example 2 later.

For FM677 and FM 426, however, there is no difference in fragment size after amplification, as shown in Table 2. Thus, the judgment whether the amplified genetic marker is of the resistant type or susceptible type can be made easily by agarose gel electrophoresis or the like. In this case, whether the genetic marker is of the resistant type or susceptible type is judged based on base sequence information obtained from sequencing of the amplified fragment. Or, the judgment whether the amplified fragment is of the resistant type or the susceptible type may be made based on restriction enzyme site information regarding restriction enzymes for the genetic markers.

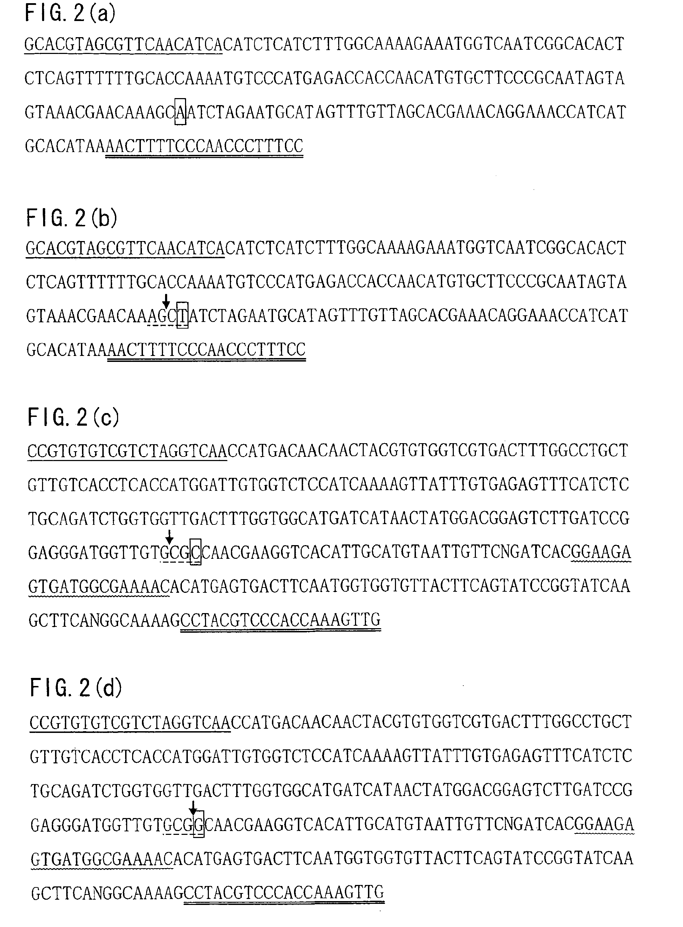

How to make the judgment whether the amplified fragment is of the resistant type or the susceptible type by using the base sequence information and the restriction enzyme site information is described more specifically referring to FIG. 2. FIG. 2(a) illustrates the whole base sequence (S.E.Q. ID. NO. 8) of FM677 of the resistant type (Harbin 2-row). FIG. 2(b) illustrates the whole base sequence (S.E.Q. ID. NO. 9) of FM677 of the susceptible type (Turkey 6). FIG. 2(c) illustrates the whole base sequence (S.E.Q. ID. NO. 10) of FM426 of the resistant type (Russia 6). FIG. 2(d) illustrates the whole base sequence (S.E.Q. ID. NO. 11) of FM426 of the susceptible type (H.E.S. 4). In FIGS. 2(a) and 2(b), the underlined portions indicate where a sense primer (S.E.Q. ID. NO. 3) is introduced, whereas the double-underlined portions indicate where an anti-sense primer (a complement sequence of S.E.Q. ID. NO. 4) is introduced. In FIGS. 2(c) and 2(d), the underlined portions indicate where a sense primer (S.E.Q. ID. NO. 5) is introduced, the double-underlined portions indicate where an anti-sense primer (a complement sequence of S.E.Q. ID. NO.6) is introduced, and the wavy-underlined portions indicate where an anti-sense primer (a complement sequence of S.E.Q. ID. NO.7) is introduced. The bases of the characters surrounded with a square in FIG. 2(a) to 2(d) indicate where the base sequences of the resistant type and the susceptible type are different. Thus, the sequencing of the amplified fragment obtained by the amplification reaction makes it possible to judge whether the amplified fragment is of resistant type or susceptible type.

Moreover, the dotted line in FIG. 2(b) indicates recognition sequence of restriction enzyme AluI. The dotted line in FIG. 2(c) indicates recognition sequence of restriction enzyme HhaI. The dotted line in FIG. 2(d) indicates recognition sequence of restriction enzyme AciI. The portion with the arrow in FIG. 2(b) indicates where restriction enzyme AluI cleaves. The portion with the arrow in FIG 2(c) indicates where restriction enzyme HhaI cleaves. The portion with the arrow in FIG 2(d) indicates
where restriction enzyme AciI cleaves. Thus, it is understood that FM677 of the resistant type has no AluI site, whereas FM677 of the susceptible type has one AIuI site. Further, it is understood that FM426 of the resistance type has one HhaI site, whereas FM426 of the susceptible type has one AciI site.

From these pieces of information, if the amplified fragment obtained by the amplification reaction using the second primer set of the primers of S.E.Q. ID. NOs. 3 and 4, that is, the amplification reaction for detecting FM677 was not cleaved when digested with AluI, and only the fragment of 208bp was detected, it would be judged that this FM677 is of the resistant type. If the amplified fragment was cleaved at one site into fragments of 118bp and 90bp, and these fragments were detected, it would be judged that this FM677 is of the susceptible type.

On the other hand, if the amplified fragment (335bp) obtained by the amplification reaction using the third primer set of the primers of S.E.Q. ID. NOs. 5 and 6, that is, the amplification reaction for detecting FM426 was cleaved at one site into fragments of 194bp and 141bp when digested with HhaI, and these fragments were detected, it would be judged that this FM426 is of the resistant type. If the amplified fragment was not cleaved, and only the fragment of 335bp was detected, it would be judged that this FM426 is of the susceptible type.

On the other hand, if the amplified fragment obtained by the same amplification reaction was not cleaved when digested with AciI, and only the fragment of 335bp was detected, it would be judged that this FM426 is of the resistant type. If the amplified fragment was cleaved at one site when digested with AciI, and the fragments of 196bp and 139bp were detected, it would be judged that this FM426 is of the susceptible type.

It is possible to perform the detection of FM426 by using amplification reaction using the fourth primer set of the primers of S.E.Q. ID. NOs. 5 and 7. If the amplified fragment (254bp) obtained by this amplification reaction was cleaved at one site into the fragments of 194bp and 60bp when digested with HhaI, and these fragments were detected, it would be judged that this FM426 is of the resistant type. If the amplified fragment was not cleaved, and only the fragment of 254bp was detected, it would be judged that this FM426 is of the susceptible type.

On the other hand, if the amplified fragment obtained by the same amplification reaction was not cleaved when digested with AciI and only the fragment of 254bp was detected, it would be judged that this FM426 is of the resistant type. If the amplified fragment was cleaved at one site into the fragments of 196bp and 58bp when digested with AciI, and these fragments were detected, it would be judged that this FM426 is of the susceptible type.

This will be specifically described later in Example 2.

It is possible to perform the detection of the polymorphism of the genetic markers by AFLP (Amplified Fragment Length Polymorphisms) analysis. In the following, how to detect the polymorphism of the genetic marker MM1057 according to the present invention by the AFLP analysis is described.

The AFLP analysis is not particularly limited to a particular procedure and may be performed by, for example, a method described in the literature (Pieter Vos, Rene Hogers, Marjo Bleeker, Martin Reijans, Theo van de Lee, Miranda Hornes, Adrie Frijters, Jerina Pot, Johan Peleman, Martin Kuiper and Marc Zabeau. (1995) AFLP: a new technique for DNA fingerprinting. Nucleic Asids Research. 23:21:4407-4414.) or a method modified therefrom. A specific example of the AFLP analysis of the genetic marker MM1057 performed by the inventors of the present invention is described below.

Fifty ng of a genomic DNA was subjected to double digestion for 12 hours at 37°C with EcoRI (Takara bio Inc.) and MseI (NEW ENGLAND Biolabs Inc.) of 1.5U each in a reaction system of 25µl in total. The DNA treated with the restriction enzymes were then ligated with 5µM of EcoRI adaptors (whose base sequences are shown in S.E.Q. ID. NOs. 14 and 15), and 50µM of MseI adaptors (whose base sequences are shown in S.E.Q. ID. NOs. 16 and 17), using 25U of T4 ligase (Takara bio Inc.) for 3 hours at 37°C. The ligated DNA fragment was pre-amplified with a universal primer (whose base sequence is shown in S.E.Q. ID. NO. 18) of EcoRI, and a universal primer (whose base sequence is shown in S.E.Q. ID. NO. 19) of MseI. Using 0.06ng/µl of a reaction solution of the pre-amplification, amplification reaction was performed with a selective primer of EcoRI whose base sequence is shown in S.E.Q. ID. NO. 20, and a selective primer of MseI whose base sequence is shown in S.E.Q. ID. NO. 21. Besides, the combination of the selective primer of EcoRI whose base sequence is shown in S.E.Q. ID. NO. 20, and the selective primer of MseI whose base sequence is shown in S.E.Q. ID. NO. 21 is referred to as "fifth primer set" here.

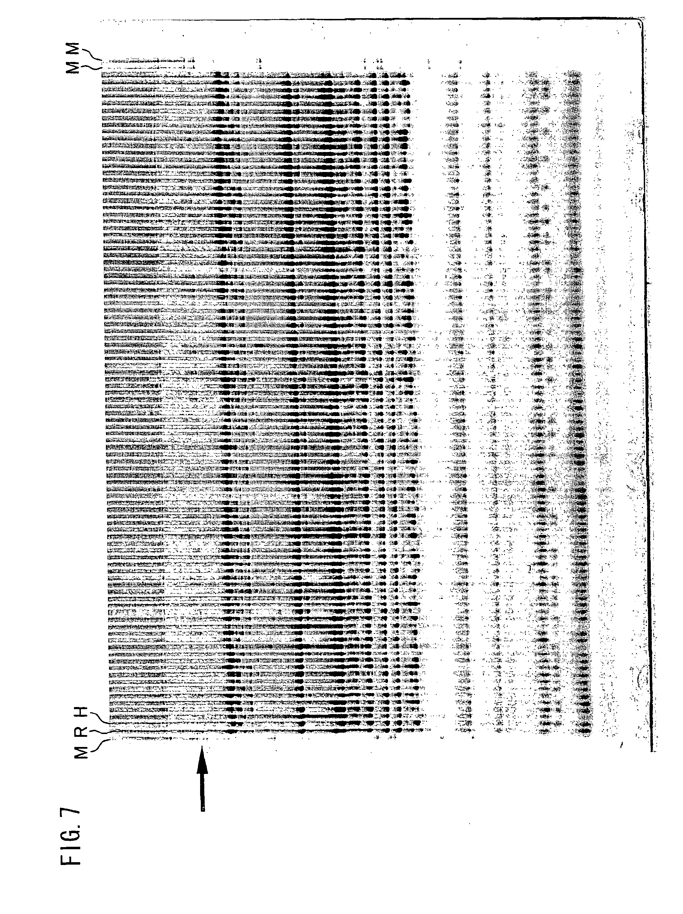

Electrophoresis of the DNA fragment obtained from the amplification reaction was performed, and its results are shown in FIG. 7. In FIG. 7, the lane labeled with "R" illustrates a result of the AFLP analysis of Russia 6, which is a Fusarium head blight-resistant barley cultivar. The lane labeled with "H" illustrates a result of the AFLP analysis of H.E.S.4, which is a Fusarium head blight-susceptible barley cultivar. The other lanes illustrate results of the AFLP analysis of recombinant inbed lines (RI lines) derived from the cross of Russia 6 and H.E.S.4.

The comparison of the results of Russia 6 and H.E.S.4 in FIG.7 shows that a DNA fragment of approximately 1057bp (indicated by the arrow in FIG. 7) was observed in Russia 6, which is a Fusarium head blight-resistant barley cultivar. This indicates that there is a polymorphism between the Fusarium head blight-resistant cultivar and the Fusarium head blight-susceptible cultivar. The genetic marker exhibiting the polymorphism is referred to as MM 1057. Therefore, if an AFLP analysis of an RI line found a DNA fragment of about 1057bp in size, it would be judged that this RI line has a Fusarium head blight-resistance factor. If not, it would be judged that this RI line has no Fusarium head blight-resistance factor. Besides, the lane labeled with "M" is a DNA size marker.

### (2-3-2. Method according to Present Invention for Judging Fusarium Head Blight-Resistant Plant (e.g., Gramineae plant (Hordeum or the like such as Barley))

In a manner similar to the method (present resistance factor judging method) according to the present invention for judging whether a Fusarium head blight-resistance factor is present or not, it is possible to test a plant (e.g., a Gramineae plant (Hordeum or the like such as barley)) in order to judge whether the plant is Fusarium head blight resistant or not. A plant having a Fusarium head blight-resistance factor is resistant to Fusarium head blight. Thus, it can be said that, by the present resistance factor judging method, it is possible to judge whether the plant (e.g., a Gramineae plant (Hordeum or the like such as barley)) is a Fusarium head blight-resistant plant or not. In other words, the present resistance factor judging method for judging whether a Fusarium head blight-resistance factor is present or not can be a method for judging whether a plant is a Fusarium head blight-resistant plant (e.g., a Gramineae plant (Hordeum or the like such as barley)) (hereinafter, the method for judging whether a plant is a Fusarium head blight-resistant plant is referred to as a "present judging method").

The present judging method can be used as an effective means for screening in breeding the Fusarium head blight-resistant plant. For example, the transformant plant (e.g., Hordeum or the like such as barley) to which the DNA fragment including the Fusarium head blight-resistance factor is introduced can be easily screened out from among a large number of transformant candidates in producing the Fusarium head blight-resistant plant (e.g., Hordeum or the like such as barley) according to the present invention as described in Section 2-2 above. Furthermore, the present judging method is also applicable as means for screening a plant (e.g., Hordeum or the like such as barley) attained by mutation using a chemical or the other means, or by breeding such as crossing.

As described in the section discussing the present judging method, the present judging method can separately make the judgments whether 2H-2 factor is present and whether 5H-1 factor is present. Therefore, according to present judging method, it is possible to judge whether the plant is a plant (e.g., Hordeum or the like such as barley) in which either the DNA fragment including 2H-2 factor or the DNA fragment including 5H-1 factor is solely introduced, or a plant (e.g., Hordeum or the like such as barley) in which both the DNA fragments are introduced.

The accuracy (probability) of the present judging method is same as the one described in the section discussing the present resistance factor judging method. That is, by performing the detection with a genetic marker located closer to a Fusarium head blight-resistance factor (2H-2 factor, 5H-1 factor), it is possible to test a plant (e.g., Hordeum or the like such as barley) to judge with higher accuracy (probability) whether the plant is a Fusarium head blight-resistant plant (e.g., Hordeum or the like such as barley)or not. Further, by performing the detection with genetic markers whose loci sandwich the Fusarium head blight-resistance factor therebetween, it is possible to test a plant (e.g., Hordeum or the like such as barley) to judge with even higher accuracy (probability) whether the plant is a Fusarium head blight-resistant plant (e.g., Hordeum or the like such as barley) or not.

### <2-4. Primer Population of Present Invention>

A primer population according to the present invention is a primer population for detecting a genetic marker(s) according to the present invention, and comprises at least two of the primers whose base sequences indicated in S.E.Q. ID. NOs. 1 to 7, and 20 to 47.

The primer population according to the present invention may have any combinations of primers. For example, it is preferable that the primer population include at least one set of: the first primer set including the combination of the primers of the base sequences of S.E.Q. ID. NOs. 1 and 2; the second primer set including the combination of the primers of the base sequences of S.E.Q. ID. NOs. 3 and 4; the third primer set including the combination of the primers of the base sequences of S.E.Q. ID. NOs. 5 and 6; the fourth primer set including the combination of the primers of the base sequences of S.E.Q. ID. NOs. 5 and 7; the fifth primer set including the combination of the primers of the base sequences of S.E.Q. ID. NOs. 20 and 21; a sixth primer set including the combination of the primers of the base sequences of S.E.Q. ID. NOs. 22 and 23; a seventh primer set including the combination of the primers of the base sequences of S.E.Q. ID. NOs. 24 and 25; an eighth primer set including the combination of the primers of the base sequences of S.E.Q. ID. NOs. 26 and 27; a ninth primer set including the combination of the primers of the base sequences of S.E.Q. ID. NOs. 28 and 29; a tenth primer set including the combination of the primers of the base sequences of S.E.Q. ID. NOs. 30 and 31; an eleventh primer set including the combination of the primers of the base sequences of S.E.Q. ID. NOs. 32 and 33;
a twelfth primer set including the combination of the primers of the base sequences of S.E.Q. ID. NOs. 34 and 35; a thirteenth primer set including the combination of the primers of the base sequences of S.E.Q. ID. NOs. 36 and 37; a fourteenth primer set including the combination of the primers of the base sequences of S.E.Q. ID. NOs. 38 and 39; a fifteenth primer set including the combination of the primers of the base sequences of S.E.Q. ID. NOs. 40 and 41; a sixteenth primer set including the combination of the primers of the base sequences of S.E.Q. ID. NOs. 42 and 43; a seventeenth primer set including the combination of the primers of the base sequences of S.E.Q. ID. NOs. 44 and 45; and an eighteenth primer set including the combination of the primers of the base sequences of S.E.Q. ID. NOs. 46 and 47.

The primer population according to the present invention is a primer population for use in detecting a genetic marker linked to a Fusarium head blight-resistance factor (2H-2 factor, 5H-1 factor, etc.)

As described above, the use of the first primer set makes it possible to amplify and detect MM314 linked to 2H-2 factor, whereas the use of the first primer set makes it possible to amplify and detect FM677 linked to 2H-2 factor. The use of the third or fourth primer set makes it possible to amplify and detect FM426 linked to 5H-1 factor. The use of the fifth primer set makes it possible to detect MM 1057 linked to 5H-1 factor. Genetic markers that can be detected by using the sixth to eighteenth primer sets respectively will be described in a second embodiment.

It is possible to detect a corresponding genetic marker with the primer population of the present invention comprising at least one of the 18 primer sets. However, for example, a primer population of the present invention comprising at least one of the first and second primer sets, and at least one of the third and fourth primer sets is more preferable because this primer population can amplify and detect both the genetic marker (MM314, FM677) linked to 2H-2 factor, and the genetic marker (FM426) linked to 5H-1. Furthermore, a primer population of the present invention comprising all the primer sets described above is most preferable, because all the genetic markers according to the present invention can be amplified and detected with this primer population.

The primer population of the present invention, which can detect the genetic marker according to the present invention, can be used as a primer for DNA amplification in "2-3-1. Method according to the Present Invention for Judging Whether Fusarium Head Blight-Resistance Factor is Present or not" and "2-3-2. Method according to Present Invention for Judging Fusarium Head Blight-Resistant Plant". Thus, a later-described kit for judging whether a Fusarium head blight-resistance factor is present or not, and a later-described kit for judging whether a plant is a Fusarium head blight-resistant plant (e.g., Hordeum or the like such as barley) may be arranged to include a primer population of the present invention.

### <2-5. Kit for Judging whether Fusarium Head Blight-Resistance Factor is present or not, and Kit for Judging Whether Plant is Fusarium Head Blight-Resistant Plant>

The kit according to the present invention for judging whether a Fusarium head blight-resistance factor is present or not (hereinafter, this kit is referred to as "present resistance factor judging kit", where appropriate) is a kit used in "2-3-1, Method according to the Present Invention for Judging Whether Fusarium Head Blight-Resistance Factor is Present or not " and comprises" Primer Population of Present Invention".

Thus, for example, a present resistance factor judging kit comprising the first primer set can detect MM314 linked to 2H-2 factor, whereas a present resistance factor judging kit comprising the second primer set can detect FM677 linked to 2H-2 factor, as described above. Moreover, a present resistance factor judging kit comprising the third or fourth primer set can detect FM426 linked to 5H-1 factor. Furthermore, a present resistance factor judging kit comprising the fifth primer set can detect MM 1057 linked to 5H-1 factor.

Therefore, a present resistance factor judging kit including a primer population including at least one of the 18 sets can detect a corresponding genetic marker(s). For example, a present resistance factor judging kit including a primer population including at least one of the first and second primer sets, and at least one of the third and fourth primer sets is more preferable because this kit can amplify and detect both the genetic marker (MM314, FM677) linked to 2H-2 factor, and the genetic marker (FM426) linked to 5H-1. A present resistance factor judging kit including a primer population comprising all the primer sets described above is most preferable, because all the genetic markers according to the present invention can be amplified and detected with this kit.

Besides these contents, the present judging kit may contain an enzyme, reagent, and/or the like for PCR, and may contain a reagent, a buffer, a centrifugal tube for the preparation of a genomic DNA that acts as a template, and may contain a genetic marker (such as MM314, FM677, FM426 or the like) necessary for detecting a targeted DNA size band, or an appropriate DNA size marker.

A kit for judging whether or not a plant is a Fusarium head blight-resistant plant (e.g., Hordeum or the like such as barley) can have the arrangement as the present kit. This is because, as described above, by the use of the resistance factor judging kit to judge whether the Fusarium head blight-resistance factor is present or not, it is possible to test a plant (e.g., Hordeum or the like such as barley) to judge whether the plant has a Fusarium head blight-resistance factor or not, that is, whether the plant is a Fusarium head blight-resistant plant or not.

### <2-6. Gene Detecting Apparatus of Present Invention>

A gene detecting apparatus of the present invention (e.g., DNA microarray) (hereinafter, this apparatus is referred to as "present gene detecting apparatus" where appropriate) includes a genetic marker(s) according to the present invention (such as MM314, FM677, FM426, and/or MM1057) fixed on an appropriate substrate (glass, silicon wafer, nylon membrane, or the like). The present gene detecting apparatus is reacted with a probe prepared from a plant to be tested, and a signal emitted from the reaction is detected, whereby it is possible to detect a plurality of genetic markers at the same time easily. Therefore, the present gene detecting apparatus can be used as means for performing detection of a polymorphism of genetic marker according to the present invention. Accordingly, the present gene detecting apparatus is applicable as means for detecting in the method for judging whether a Fusarium head blight-resistance factor is present or not, and in the method for judging whether a plant (e.g., Hordeum or the like such as barley) is a Fusarium head blight-resistant plant or not. Furthermore, it is possible to include the present gene detecting apparatus in the kit for judging whether a Fusarium head blight-resistance factor is present or not, or in the kit for judging whether a plant (e.g., Hordeum or the like such as barley) is a Fusarium head blight-resistant plant or not. The kits may includes a reagent, tool, apparatus, or the like for detecting the signal.

It is sufficient for the present gene detecting apparatus that at least one of the genes markers (such as MM314, FM677, FM426, MM1057, and the like) according to the present invention is fixed on the substrate. Moreover, the present gene detecting apparatus may be such that one or both of the genetic markers of the resistance type and of the susceptible type is fixed on the substrate. For higher accuracy (probability) in the judgment whether a Fusarium head blight-resistance factor is present or not, it is preferable that a combination of a plurality of genetic markers whose loci sandwich a Fusarium head blight-resistance factor (2H-2 factor, 5H-1 factor, etc.) to detect is fixed on the substrate. More specifically, one example of the combination of genetic markers whose loci sandwich 2H-2 factor is the combination of MM314 and FM677, and one example of the combination of genetic markers whose loci sandwich 5H-1 factor is the combination of FM426 and MM 1057.

If at least one of the combinations mentioned above is fixed on the substrate, then it is possible to make a judgment on whether 2H-2 factor or 5H-1 factor is present or not. Meanwhile, it is most preferable that the combination of MM314 and FM677, and the combination of FM426 and MM1057 be fixed on the substrate, because it is possible to detect both 2H-2 factor and 5H-1 factor.

By using the present gene detecting apparatus to which a plurality of the genetic marker fixed, it is possible to easily detect genetic markers with one test. Further, the judgment whether the Fusarium head blight-resistance factor is present or not can be done with high accuracy (probability) by using such a present gene detecting apparatus.

The present gene detecting apparatus may be arranged such that another or other genetic markers located in the vicinity of the genetic marker according to the present invention may be fixed to the present gene detecting apparatus in addition to the genetic marker(s) according to the present invention.

The present gene detecting apparatus is preferably arranged such that the genetic markers according to the present invention are fixed in the order in which they are aligned on the chromosome of barley, or that the genetic markers according to the present invention are fixed with sequence position information that corresponds to the order in which they are aligned on the chromosome of barley. With this arrangement, it is possible to perform the detection with higher accuracy when barley is to be tested. In analysis using a gene detecting apparatus such as a conventional DNA microarray or the like, it is necessary to double check in the event that no signal is obtained at a certain spot, in order to find out whether the lack of the signal indicates that the genetic marker that is the target for the detection is absent, or that the signal is not obtained due to an experimental error in the analysis. On the other hand, with the present gene detecting apparatus, in which the fixed genetic markers are so aliened that the order of the genetic markers on the chromosome can be confirmed, it is easy to judge whether the lack of the signal is due to an experimental error or not.

To specifically explain this, suppose a signal is obtained at spots before and after a spot at which no signal is obtained, for example. In an array according to the present invention, each spot is so aliened that the order on the chromosome can be confirmed. In general, to recombine only one of genes next to each other in line on the chromosome, recombination should occur twice in the vicinity of the recombination of only the one of the genes next to each other. Such a phenomenon occurs with quite low probability. So, it is judged that the lack of the signal occurs due to an experimental error. As described above, the present gene detecting apparatus can improve the accuracy of the analysis because it is possible to judge whether the lack of a signal at a spot is due to an experimental error or not.

It should be noted that the present invention encompasses the following inventions:
(1) A DNA marker existing in a genomic DNA of a Hordeum or Triticum, and linked to a Fusarium head blight-resistance factor,
   a distance from the Fusarium head blight-resistance factor to the DNA marker is within a range of approximately 0 to 9cM.
(2) The DNA marker as set forth in (1), wherein the Hordeum or Triticum is barley.
(3) The DNA marker as set forth in (2), wherein the genomic DNA is 2H chromosome.
(4) The DNA marker as set forth in (1) to (3), wherein the DNA marker is for being amplified with a first primer set that is a combination of a primer having the base sequence of S.E.Q. ID. NO. 1, and a primer having the base sequence of S.E.Q. ID. NO..2.
(5) The DNA marker as set forth in (4), being distanced from the Fusarium head blight-resistance factor by approximately 0cM.
(6) The DNA marker as set forth in any one of (1) to (3), wherein the DNA marker is for being amplified with a second primer set that is a combination of a primer having the base sequence of S.E.Q. ID. NO. 3, and a primer having the base sequence of S.E.Q. ID. NO. 4.
(7) The DNA marker as set forth in (6), being distanced from the Fusarium head blight-resistance factor by approximately 0.6cM.
(8) The DNA marker as set forth in (7), wherein the DNA marker has the base sequence of S.E.Q. ID. NO. 8 or 9.
(9) The DNA marker as set forth in (2), wherein the genomic DNA is 5H chromosome.
(10) The DNA marker as set forth in (1), (2), or (9), wherein the DNA marker is for being amplified with a third primer set that is a combination of a primer having the base sequence of S.E.Q. ID. NO. 5, and a primer having the base sequence of S.E.Q. ID. NO. 6.
(11) The DNA marker as set forth in (10), being distanced from the Fusarium head blight-resistance factor by approximately 9cM.
(12) The DNA marker as set forth in (11), wherein the DNA marker has the base sequence of S.E.Q. ID. NO. 10 or 11.
(13) The DNA marker as set forth in (1), (2), or (9), wherein the DNA marker is for being amplified with a fourth primer set that is a combination of a primer having the base sequence of S.E.Q. ID. NO. 5, and a primer having the base sequence of S.E.Q. ID. NO. 7.
(14) The DNA marker as set forth in (13), being distanced from the Fusarium head blight-resistance factor by approximately 9cM.
(15) The DNA marker as set forth in (14), wherein the DNA marker has the base sequence of S.E.Q. ID. NO. 12 or 13.
(16) The DNA marker as set forth in (9), being distanced from the Fusarium head blight-resistance factor by approximately 2.2cM.
(17) A DNA fragment isolating method, comprising:
   isolating, by using the DNA marker as set forth in any one of (1) to (16), a DNA fragment including the Fusarium head blight-resistance factor.
(18) A method for producing a Fusarium head blight-resistant plant, comprising:
   introducing, into a genomic DNA of a plant, the DNA fragment including the Fusarium head blight-resistance factor, the DNA fragment being obtained by the method as set forth in (17).
(19) The method as set forth in (18), wherein the plant is a Hordeum or Triticum.
(20) The method as set forth in (19), wherein the Hordeum or Triticum is barley.
(21) A Fusarium head blight-resistant plant obtained by the method as set forth in any one of (18) to (20).
(22) A DNA microarray wherein at least one of the DNA markers as set forth in (1) to (16) is fixed on a substrate.
(23) A method for detecting a DNA marker linked to a Fusarium head blight-resistance factor from a genomic DNA of a plant,
   the DNA marker is a DNA for being amplified by using at least one of a first to fourth primer sets,
   where:
   the first primer set is a combination of a primer having the base sequence of S.E.Q. ID. NO. 1, and a primer having the base sequence of S.E.Q. ID. NO. 2;
   the second primer set is a combination of a primer having the base sequence of S.E.Q. ID. NO. 3, and a primer having the base sequence of S.E.Q. ID. NO. 4;
   the third primer set is a combination of a primer having the base sequence of S.E.Q. ID. NO. 5, and a primer having the base sequence of S.E.Q. ID. NO. 6; and
   the fourth primer set is a combination of a primer having the base sequence of S.E.Q. ID. NO. 5, and a primer having the base sequence of S.E.Q. ID. NO. 7.
(24) The method as set forth in (23), wherein:
   at least one of the first and second primer sets is used; and
   at least one of the third and fourth primer sets is used.
(25) The method as set forth in (23) or (24), wherein the plant is a Hordeum or Triticum.
(26) The method as set forth in (25), the Hordeum or Triticum is barley.
(27) A method for judging whether or not a Fusarium head blight-resistance factor is present, comprising:
   performing detection of a polymorphism of at least one of DNA markers that are detectable from a genome of a plant by the method as set forth in any one of (23) to (26).
(28) A method for judging whether or not a plant has a Fusarium head blight-resistance factor, comprising:
   performing detection of a polymorphism of at least one of DNA markers that are detectable from a genome of the plant by the method as set forth in any one of (23) to (26).
(29) A primer population comprising at least one of first to fourth primer sets,
   where:
   the first primer set is a combination of a primer having the base sequence of S.E.Q. ID. NO. 1, and a primer having the base sequence of S.E.Q. ID. NO. 2;
   the second primer set is a combination of a primer having the base sequence of S.E.Q. ID. NO. 3, and a primer having the base sequence of S.E.Q. ID. NO. 4;
   the third primer set is a combination of a primer having the base sequence of S.E.Q. ID. NO. 5, and a primer having the base sequence of S.E.Q. ID. NO. 6; and
   the fourth primer set is a combination of a primer having the base sequence of S.E.Q. ID. NO. 5, and a primer having the base sequence of S.E.Q. ID. NO. 7.
(30) The primer population as set forth in (29) comprising:
   at least one of the first and second primer sets; and
   at least one of the third and fourth primer sets.
(31) A kit for judging whether a Fusarium head blight-resistance factor is present or not, by performing detection of a polymorphism of a DNA marker linked to the Fusarium head blight-resistance factor, comprising:
   the primer population as set forth in (29) or (30), as a primer set for use in amplification of the DNA marker.
(32) A kit for judging whether a plant is a Fusarium head blight-resistant plant or not, by performing detection of a polymorphism of a DNA marker linked to the Fusarium head blight-resistance factor, comprising:
   the primer population as set forth in (29) or (30), as a primer set for use in amplification of the DNA marker.

The DNA markers as set forth in (1) to (16) exist in the genomic DNA of the Hordeum and the like (e.g., barley) and are linked to a Fusarium head blight-resistance factor. That is, there is a low probability that recombination of the DNA marker occurs without recombination of the Fusarium head blight-resistance factor. Thus, the use of any of the DNA markers makes it possible to, for example, acquire a DNA fragment including the Fusarium head blight-resistance factor, judge whether the Fusarium head blight-resistance factor is present or not, judge whether a plant (Hordeum or the like such as barley) is a Fusarium head blight-resistant plant, or not, and do the like task.

The method as set forth in (17) for isolating a DNA fragment including a Fusarium head blight-resistance factor isolates the DNA fragment by using any of DNA markers (1) to (16). The DNA markers are linked to the Fusarium head blight-resistance factor. Thus, cloning of a DNA makes it possible to isolate the DNA fragment easily.

The method as set forth in any one of (18) to (20) includes introducing the DNA fragment including the Fusarium head blight-resistance factor into the genomic DNA of a plant (Hordeum or the like, barley). The Fusarium head blight-resistance factor is a gene having a trait of giving a resistance to Fusarium head blight. Thus, it is possible to give the resistance to a plant (Hordeum or the like, barley) susceptible to Fusarium head blight, or improve a plant (Hordeum or the like, barley) in its resistance to Fusarium head blight.

The Fusarium head blight-resistant plant as set forth in (21) is a plant (Hordeum or the like, barley) obtained by the method for producing the Fusarium head blight-resistant plant. Therefore, it is possible to prevent the Fusarium head blight diseases, and improve the crop yield, quality, and like of the Fusarium head blight-resistant plant (Hordeum or the like).

The DNA microarray as set forth in (22) has such a configuration that the DNA markers as set forth in (1) to (16) are fixed on the substrate. With this DNA microarray, it is possible to detect the multiple DNA markers without repeating the test, thereby making it possible to handle a larger number of samples in a shorter time with less labor.

In the method as set forth in (23) for detecting the DNA marker, the detection of the DNA marker existing in 2H chromosome and linked to the Fusarium head blight-resistance factor can be performed by carrying out amplification reaction with the first primer set or the second primer set. Meanwhile, the detection of the DNA marker existing on 5H chromosome and linked to the Fusarium head blight-resistance factor can be performed by carrying out amplification reaction with the third primer set or the fourth primer set.

According to the method as set forth in (24) for detecting a DNA marker, at least one of the first and second primer sets is used, and at least one of the third and fourth primer sets is used. Thus, with the method as set forth in (24), the detections of the DNA markers existing on 2H chromosome and 5H chromosome and linked to the Fusarium head blight-resistance factor can be performed together.

The method as set forth in (25) or (26) for detecting a DNA marker, the method as set forth in (23) or (24) is applied for Hordeum or the like, or barley. The DNA marker to detect exists in the genomic DNA of the Hordeum and the like such as barley, and is linked to a Fusarium head blight-resistance factor. Therefore, the methods for detecting the DNA marker are suitably applicable to Hordeum and the like such as barley.

The method as set forth in (27) for judging whether the Fusarium head blight-resistance factor is arranged to test a plant (e.g., Hordeum or the like such as barley) to judge whether a Fusarium head blight-resistance factor is present or not in the plant, by performing detection of polymorphism (Fusarium head blight-resistant type or Fusarium head blight-susceptible type) of at least one of the DNA markers that are detectable from the genomic DNA of the plant. The DNA marker is linked to the Fusarium head blight-resistance factor. Thus, by using the type of the marker as an indicator, it is possible to judge with high probability whether the Fusarium head blight-resistance factor is present or not.

The method as set forth in (28) for judging whether a plant is a Fusarium head blight-resistant plant or not judges whether the tested plant (e.g., Hordeum or the like such as barley) is a Fusarium head blight-resistant plant (e.g., Hordeum or the like such as barley) or not, by performing detection of a polymorphism of at least one of the DNA markers that are detectable from among a genomic DNA of the plant by the method as set forth in (23) or (26). The DNA markers are linked to Fusarium head blight-resistance factors. Thus, by using the type of the marker as an indicator, it is possible to judge with high probability whether the Fusarium head blight-resistance factor is present or not.

The primer population as set forth in (29) or (30) can be used for detecting a DNA marker linked to a Fusarium head blight-resistance factor present in 2H chromosome, and/or for detecting a DNA marker linked to a Fusarium head blight-resistance factor present in 5H chromosome. The primer populations as set forth in (29) and (30) are applicable to judgment whether a Fusarium head blight-resistance factor is present or not, or judgment whether a plant (e.g., Hordeum or the like such as barley) is a Fusarium head blight-resistant plant or not.

The kit as set forth in (31) for judging whether a Fusarium head blight-resistance factor is present or not includes a primer for use in amplification of a DNA marker linked to a Fusarium head blight-resistance factor present on 2H chromosome and/or for use in amplification of a DNA marker linked to a Fusarium head blight-resistance factor present on 5H chromosome. Therefore, the use of the kit makes it possible to easily judge whether a Fusarium head blight-resistance factor is present in the tested plant or not.

The kit as set forth in (32) for judging whether a plant is a Fusarium head blight-resistant plant or not includes a primer for use in amplification of a DNA marker linked to a Fusarium head blight-resistance factor present on 2H chromosome and/or for use in amplification of a DNA marker linked to a Fusarium head blight-resistance factor present on 5H chromosome. Therefore, the use of the kit makes it possible to easily judge whether a tested plant (e.g., Hordeum or the like such as barley) is a Fusarium head blight-resistant plant (e.g., Hordeum or the like such as barley) or not.

The present invention is described in details below referring to Examples which are not to limit the present invention.

### [Example 1: QTL Analysis Regarding Fusarium Head Blight Resistance of Barley]

A QTL analysis was conducted to search for a Fusarium head blight-resistance factor of barley.

### <Material>

Used were recombinant inbed lines (RI lines) derived from the cross of Russia 6 (two-row, resistant) and H.E.S.4 (six-row, susceptible), which are barley cultivar largely different in resistance against Fusarium head blight.

### <Evaluation method for Fusarium Head Blight Resistance>

A modified "Cut-Spike Test" (see Non-Patent Document 4) was used for the evaluation. The evaluation of the Fusarium head blight is briefly explained below.

The material was cultivated according to a generally-used method. For each RI line, a spike was cut off, with a flag leaf, from a stem at the first internode in flowering period. The spikes were held stand in a stainless tray into which tap water was continuously run. To the spikes the inoculum was sufficiently spayed, which was prepares such that fifteen conidiums of the Fusarium bacteria were observed per field of vision of a microscope of 200 × magnification. The spikes were cultivated at 25°C and under 100% humidity for first two days after the inoculation, and at 18°C and under approximately 90% humidity for next 6 days. Lighting condition was 14 hours day length and 10,000 lux luminance. On eighth day from the inoculation, the spikes were observed visually to be scored by 0 (resistant) to 10 (susceptible) according to the standard shown in Table 3.

**Table 3**

| | | | | | | |
|---|---|---|---|---|---|---|
| Score | 0 | 2 | 4 | 6 | 8 | 10 |
| SSR (%) | 0 | 0 to 5 | 6 to 20 | 21 to 40 | 41 to 60 | 61 to 100 |

| | | | | | | |
|---|---|---|---|---|---|---|
| Abbreviation: SSR stands for susceptible spike ratio (%). | | | | | | |

The algorisms used in the QTL analysis were simple interval mapping (SIM) and composite interval mapping (CIM). MAPMARKER/QTL and QTL Cartographer were used as analysis software respectively.

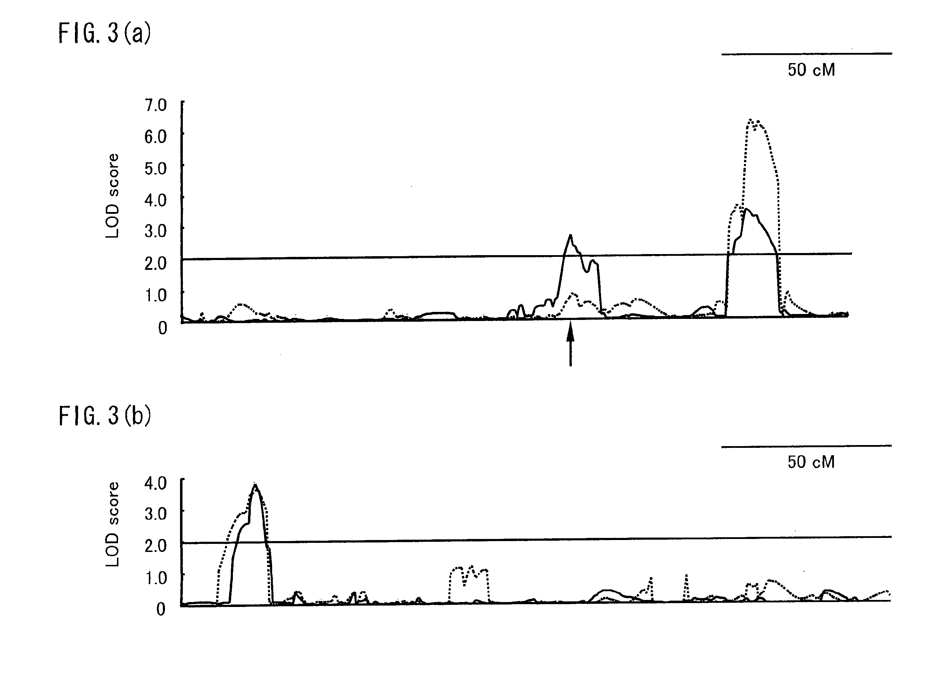

Results of the QTL analysis, especially, results of CIM are shown in FIG. 3. The QTL analysis was repeated twice for each population, and the results of both are shown (the results of the first QTL analysis are shown by the solid line, and the result of the second QTL analysis are shown by the dotted line). In FIG. 3(a), the results of the QTL analysis for 2H chromosome are shown, whereas in FIG. 3(b) the results of the QTL analysis for 5H chromosome are shown. The horizontal axis indicates loci on the chromosome, where the left side is the short-arm side (5' end side) and the right side is the long-arm side (3' end side). The vertical axis indicates LOD score (logarithmic likelihood score). The LOD score is a degree of linkage between a gene that controls the trait of the Fusarium head blight resistance, and a genetic marker located at the locus of the gene. In general, when the LOD score is above 2 or 3, it is deduced that the genetic marker is linked to the QTL, that is, there is a Fusarium head blight-resistance factor at the locus.

According to the results shown in FIG. 3(a), two QTLs relating to the Fusarium head blight resistance were detected on 2H chromosome of barley, meanwhile one QTL relating to the Fusarium head blight resistance was detected on 5H chromosome of barley according to the results shown in FIG. 3(b). One of the QTL detected on 2H chromosome of barley was identical with the locus of the publicly known row type gene (vrs1). This suggests that there is a possibility polymorphism of the vrs1 gene occurs. This result supports alleged relationship between the row type and the Fusarium head blight resistance. However, the genes located at the other QTLs were unknown. This suggests that there is a Fusarium head blight resistance-factor other than the row type gene. So, the Fusarium head blight-resistance factor located on the other locus than the locus of the vrs1 gene on 2H chromosome of barley is named "2H-2 factor", whereas the Fusarium head blight-resistance factor located on the 5H-1 chromosome of barley was name as "5H-1 factor".

Based on the high-density linkage map prepared by the inventors of the present invention, the genetic markers located respectively in the vicinity of the loci of the Fusarium head blight-resistance factors, that is, the 2H-2 factor and 5H-1 factor were searched for. As a result, the genetic markers tightly linked to the Fusarium head blight-resistance factors especially, that is, the genetic marker according to the present invention were found. As the genetic markers linked to the 2H-2 factor, MM314 and FM677 were found. Further, FM426 and MM1057 were found as the genetic markers linked to the 5H-1 factor.

Sequences of these genetic markers, which were AFLP markers respectively were found (STS). From sequence information of the STSs, primers for amplification of each genetic marker were designed. MM314 can be amplified with the first primer set of the primers of S.E.Q. ID. NOs. 1 and 2. FM677 can be amplified with the second primer set of the primers of S.E.Q. ID. NOs. 3 and 4. FM 426 can be amplified with the third primer set of the primers of S.E.Q. ID. NOs. 5 and 6, or the fourth primer set of the primers of S.E.Q. ID. NOs. 5 and 7.

### [Example 2: Judgment Whether Fusarium Head Blight-Resistance Factor was Present or not, by detecting Genetic Marker (Judging Whether Barley was Fusarium Head Blight-Resistant Barley or not)]

By detecting genetic markers according to the present invention, a barley was tested to judge whether the barley had a Fusarium head blight-resistance factor or not. Further, from the result of the judgment, it was judged whether the tested barley was a Fusarium head blight-resistant barley or not.

### <Tested Barley>

Used were recombinant inbed lines (RI lines) derived from the cross of Russia 6 (two-row, resistant) and H.E.S.4 (six-row, susceptible), which are barley cultivar largely different in resistance against Fusarium head blight.

### <Extraction of DNA>

From leaf blades of the RI lines grown in a glass house, DNAs were extracted according to a method of Langridge et al. (see Peter Langridge, Angelo Karakousis and Jan Nield. (1997) Practical Workshop in Basic Recombinant DNA Techniques Course Manual. Waite Agricultural Research Institute University of Adelaide.) DNA concentration was measured by spectrophotometer and 1% agarose gel electrophoresis, and then adjusted to 1µg/µl by adding RNase and 1×TE thereto.

### <PCR>

PCR reaction liquid was prepared by adding together 0.05µl of 5U/µl TaKaRa Ex Taq™ (Takara bio Inc.), 1µl of 10 × PCR Buffer, 0.8µl of 2.5mM dNTPs, 0.25µl each of 10µM Primers (sense primer and anti-sense primer), and 1µl of template DNA, and then making up the mixture to a total amount of 10.0µl with distilled water.

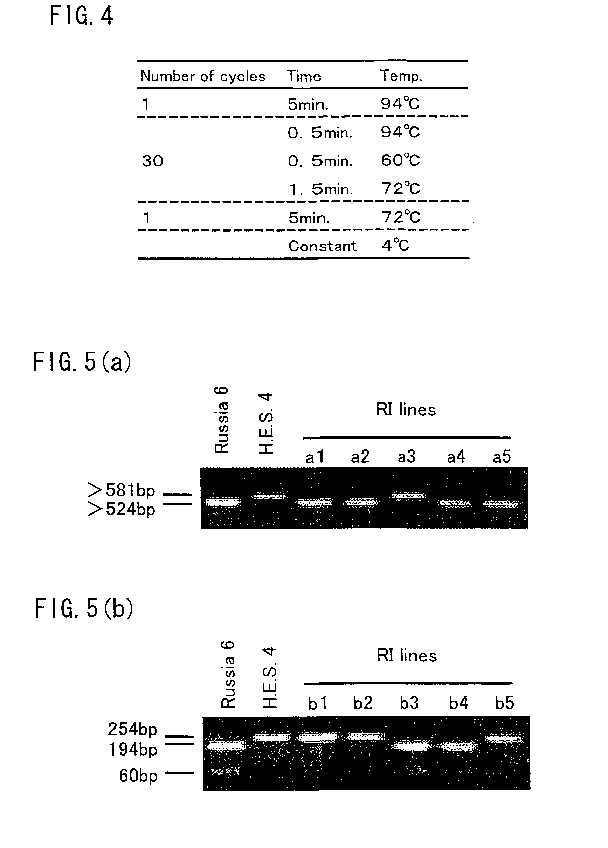

PCR reaction was carried out under the conditions in FIG. 4.

### <HhaI Digestion>

10µl of PCR product, 1µl of 10×M buffer (Takara bio Inc.), and 0.2µl of HhaI (Takara bio Inc.) were mixed together, and made up to a total amount of 10µl with distilled water.

The reaction was carried out at 37°C over night.

### <Detection of DNA Band>

After formaldehyde of equivalent amount was added thereto, the PCR product was heated for 5 minutes, and then quickly cooled in ice, thereby preparing a sample. The sample was subjected to electrophoresis with 7% polyacrylic amide gel (acryl amide: bisacryl amide = 19:1, urine: 8.5M, 0.5 × TBE) for 3 hours applying a voltage of 280V. Staining of DNA was carried out with Vistra Green nucleic acid gel stain (Amersham pharmacia biotech Co.). Moreover, Detection of DNA bands was carried out by a method using LAS-1000plus (Fuji photo film Co., Ltd.), or silver staining method (Yasukichi SUGANO, 1997, "Detection of amplified fragment and marker 5: front line of silver staining, PCR method, from basic to applied technique", edited by Takeo SEKIYA, and FUJINAGA, published by Kyoritsu Shuppan, pages 85 to 87) using sil-Best Stain for Protein/PAGE (Naclai Tesqc Inc.).

According to any one of the methods, the detection of DNA bands was carried out with the PCR product, if necessary, digested with a restriction enzyme.

### <Result>

With the primers of S.E.Q. ID. NOs. 1 and 2, PCR was carried out using genomic DNAs of Russia 6, H.E.S. 4, and RI lines as the templates. The results of the electrophoresis of the PCR products thus obtained are shown in FIG. 5(a).

FIG. 5(a) shows the results of the PCR using the primers having the base sequences of S.E.Q. ID. NOs. 1 and 2. In the results, the polymorphism of the genetic marker MM314 according to the present invention was detected. In FIG. 5(a), from the leftmost lane, the results for Russia 6, H.E.S.4, and the RI lines are shown in this order. The fragment of >524bp shown in the lane of the resistant Russia 6 is MM314 of the resistant type. Thus, if a fragment of >524pb is detected for an RI line, then it can be judged with a high possibility that the RI line has the 2H-2 factor, and further it can be judged with a high possibility that RI line is a Fusarium head blight-resistant barley. That is, it was judged that a1, a2, a4, and a5 among the RI lines in FIG. 5(a) highly probably had the 2H-2 factor, and that these RI lines were highly probably Fusarium head blight-resistant barleys.

On the other hand, it is highly probable that the RI line having the fragment of >581 bp that was observed in the lane of the susceptible H.E.S. 4 had no 2H-H factor, and that this RI line was Fusarium head blight-resistant barley. That is, it is highly probable that a3 among the RI lines in FIG. 5(a) had no 2H-2, and was a Fusarium head blight-susceptible barley.

Next; FIG. 5(b) shows the results of the electrophoresis of the PCR products digested with HhaI, the PCR products being obtained with the primers having the base sequences of S.E.Q. ID. NOs. 5 and 7 by using the genomic DNA of Russia 6, H.E.S. 4, and the RI lines as the templates.

The polymorphism of genetic marker FM426 according to the present invention was detected by PCR using the primers having the base sequences of S.E.Q. ID. NOs. 5 and 7. The polymorphism of FM426 cannot be detected by using the electrophoresis, because the sizes of the obtained fragments of FM426 are identical as shown in Table 2. Because of this, the PCR product was digested with HhaI, which is a restriction enzyme that cuts the amplification product of the Fusarium head blight-resistant barley, Russia 6, but not the amplification product of the Fusarium head blight-susceptible barley, H.E.S. 4, and the size of the cleaved fragments were checked to judge whether it was the genetic marker according to the present invention or not.

More specifically, if the HhaI digestion cuts, at one site, the amplification fragment (254bp) obtained by the amplification reaction, so that the fragments of 194bp and 60bp are detected, then it is judged that it is the genetic marker (FM426) of the resistant type, and if the HhaI digestion does not cleave the amplification fragment, so that only the fragment of 254bp is detected, it is judged that it is the genetic marker (FM426) of the susceptible type.

FIG. 5(b) illustrates the result of the electrophoresis of the HhaI-digested amplification products. From the leftmost lane, the results of Russia 6, H.E.S. 4, and the RI lines are illustrated in this order. According to the results of FIG. 5(b), the fragments of 194bp and 60bp were clearly observed in the lane of Russia 6, which is a resistant barley cultivar, meanwhile only the fragment of 254bp was observed in the lane of H.E.S. 4, which is a susceptible barley cultivar. Thus, if the fragments of 194bp and 60bp was detected from an RI line, then it is judged that the RI line highly probably has the 5H-1 factor, and that the RI line highly probably a Fusarium head blight-resistant barley. Accordingly, it was judged that b3 and b4 among the RI lines in FIG. 5(b) were highly probably with the 5H-1 factor, and that these RI lines were highly probably Fusarium head blight-resistant barleys.

It is judged that an RI lines having the fragment of 254bp highly probably has no 5H-1 factor, and that such an RI line is a Fusarium head blight-susceptible barley. Accordingly, it was judged that b1, b2, and b5 among the RI lines in FIG. 5(b) highly probably had no 5H-1 factor and these RI lines were Fusarium head blight- susceptible barleys.

### [Example 3: Relationship between Results of Judgment whether Barley is Fusarium Head Blight-Resistant Barley or not, and Fusarium Head Blight-Resistance Score]

Evaluated was a relationship between (a) the results of judgment whether a barley was a Fusarium head blight-resistant barley or not and (b) the Fusarium head blight resistance score.

### <Tested Barley>

Used were recombinant inbed lines (RI lines) (n = 121) derived from the cross of Russia 6 (two-row, resistant) and H.E.S.4 (six-row, susceptible), which are barley cultivar largely different in resistance against Fusarium head blight.

### <Fusarium Head Blight Resistance Score>

The Fusarium head blight resistance score (Scab score) was evaluated by the modified method of "Cut-Spike Test" (see Non-Patent Document 4). The method is briefly explained below.

The barley (RI lines) to be tested was cultivated according to a generally-used method. For each RI line, a spike was cut off, with a flag leaf, from a stem at the first internode in flowering period. The spikes were held stand in a stainless tray into which tap water was continuously run. To the spikes the inoculum was sufficiently spayed, which was prepared such that fifteen conidiums of the Fusarium bacteria were observed per field of vision of a microscope of 200 × magnification. The spikes were cultivated at 25°C and under 100% humidity for first two days after the inoculation, and at 18°C and under approximately 90% humidity for next 6 days. Lighting condition was 14 hours day length and 10,000 lux luminance. On eighth day from the inoculation, the spikes were observed visually to be scored by 0 (resistant) to 10 (susceptible) according to the standard shown in Table 3.

### <Detection of Genetic Marker>

According to the method described in Example 2, the genetic markers were judged whether they were of the resistant type or the susceptible type. The judgment was performed with respect to the Fusarium head blight-resistance factors, the 2H-2 factor, 5H-1 factor, and vrs1 gene.

### <Result>

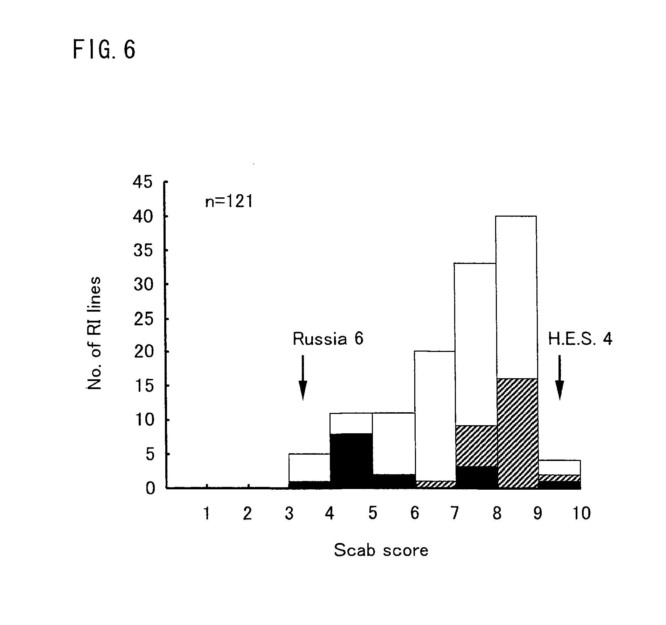

FIG. 6 illustrates the results. In FIG. 6, the horizontal axis indicates the Fusarium head blight-resistance scores (Scab scores), and the vertical axis indicates the number of RI lines (No. of RI lines). The arrows indicate the Fusarium head blight-resistant Russia 6 and the Fusarium head blight-susceptible H.E.S. 4. The "filled" symbols in FIG. 6 indicate the results of the Fusarium head blight resistance scores of RI lines that all the three genetic markers were of the Fusarium head blight resistant type. The "shaded" symbols in FIG. 6 indicate the results of the Fusarium head blight resistance scores of RI lines that all the three genetic markers were of the Fusarium head blight susceptible type. The "open" symbols indicates the results of the Fusarium head blight resistance scores of RI lines that one or some of the genetic markers were of the Fusarium head blight resistant type, and the other was of the Fusarium head blight susceptible type.

According to the result of FIG. 6, the RI lines that all the three genetic markers were of the Fusarium head blight resistant type had low Fusarium head blight resistance scores. That is, such RI lines were highly probably Fusarium head blight-resistant cultivar. On the other hand, the RI lines that all the three genetic markers were of the Fusarium head blight susceptible type had high Fusarium head blight resistance scores. That is, such RI lines were highly probably Fusarium head blight-susceptible cultivar.

Therefore, it was found that the method in which the genetic markers according to the present invention are used for judging whether a barley is a Fusarium head blight-resistant barley or not is quite effective.

### [Second Embodiment]

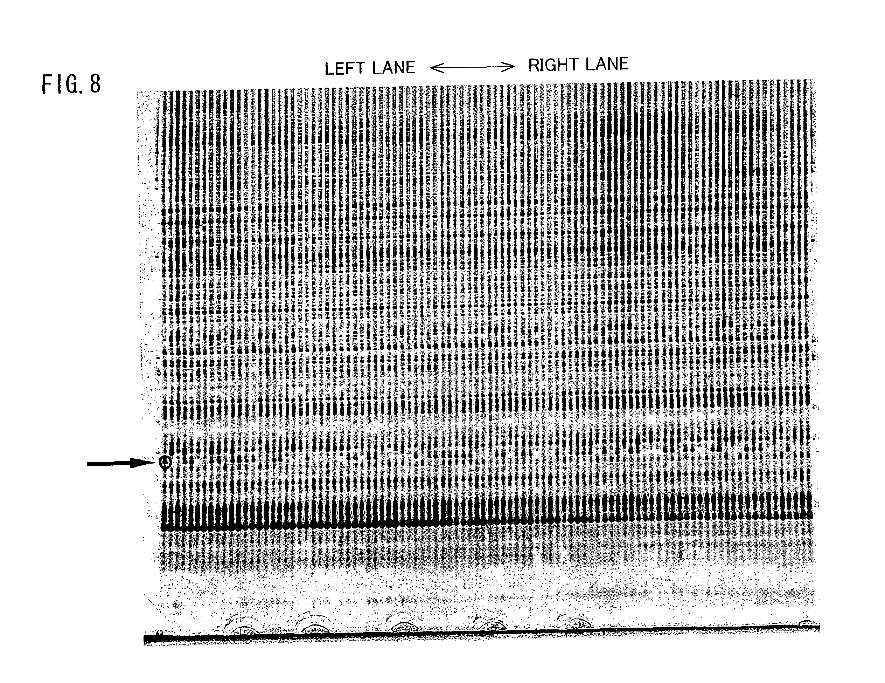

Another embodiment of the present invention is described below, referring to FIGS. 8 to 22.

The present invention relates to genetic markers linked to Fusarium head blight-resistance factor (such as locus relating to Fusarium head blight resistance), and utilization thereof. In the following, each will be described.

### (1) Genetic marker according to the present invention

Any genetic marker that exists in the genomic DNA of a Gramineae plant and linked to Fusarium head blight can be a genetic marker according to the present invention. As the Gramineae plant, Hordeum and the like are preferable, and barley is especially preferable. The Gramineae plant is not particularly limited, provided that the plant is Hordeum, Triticum, Gramineae and the like. Here, the "Hordeum and(or) the like" encompasses, for example, barley, wheat, rye, triticale, and(or) the like.

"Fusarium head blight" is a disease caused by infection of Fusarium spp. to Hordeum and the like, as described above. Fusarium head blight is a serious disease because not only poor ripening and low crop yield are caused but also mycotoxin (e.g., deoxynivalenol) is produced in Fusarium head blight, which mycotoxin would cause food poisoning in human and animals fed with the Hordeum and the like in which this disease occurs.

There are some barley cultivar having resistance to Fusarium head blight. The mechanism of the Fusarium head blight resistance has not been understood in details, but the researches so far suggests that traits such as row type, spike length, heading date, rachis-internode length, relates to the Fusarium head blight resistance. Moreover, it is said that barley has no immunological resistance to the Fusarium head blight and a relatively small number of genes are quantitative traits relating to the resistance. Regarding the quantitative trait, QTL analysis can be used to deduce where the loci relating to the quantitative trait are located on a chromosome. For barley, the inventors of the present invention developed the genetic markers linked to the loci relating to the Fusarium head blight resistance. In the following, the genetic markers are explained. Note that the genetic markers according to the present invention are not limited to the genetic markers described below.

The inventors of the present invention performed QTL analysis for the Fusarium head blight resistance of barley in order to search for the Fusarium head blight-resistance factor (the loci relating to the Fusarium head blight resistance. Specifically, the QTL analysis was performed as follows. As materials, RI lines (RHI population) derived from a cross of Russia 6 (two-row, resistant) and H.E.S. 4 (six-row, susceptible), RI lines (RI2 population) derived from a cross of Harbin 2-row (resistance) and Turkey 6 (susceptible), and DH lines (DHHS population) derived from a cross of Haruna Nijo (resistant) and H602 (susceptible) were used. The Fusarium head blight resistance was evaluated by a modified "Cut-Spike test" (see Non-Patent Document 4), and scored by 0 (resistance) to 10 (susceptible). The algorisums used in the QTL analysis were simple interval mapping (SIM) and composite interval mapping (CIM). MAPMARKER/QTL and QTL Cartographer were used as analysis software respectively.

As a result of the QTL analysis, one QTL was detected on each 2H and 4H chromosome in the RHI population, 2H, 4H, and 6H chromosome in RI2 population, and 2H, 4H, and 5H chromosome in the DHHS population. Among the QTLs thus detected, the QTL detected on 2H chromosome in the RHI population was identical with the one described in the first embodiment.

Based on information from high-density linkage map created by the inventors of the present invention for each segregated population, novel genetic markers linked to Fusarium head blight-resistance factors (loci relating to the Fusarium head blight resistance) were found.

Novel genetic markers, namely "MMtgaEatc128" and "FMgcgEatc530", linked to the Fusarium head blight-resistance factor (locus relating to the Fusarium head blight resistance) on 4H chromosome were detected in the RHI population.

"MMtgaEatc 128" is a genetic marker that is detected by so-called AFLP (Amplified Fragment Length Polymorphisms). AFLP is performed by as follows. MseI adaptors having the base sequences of GACGATGAGTCCTGAG (S.E.Q. ID. NO. 16) and TACTCAGGACTCAT (S.E.Q. ID. NO. 17) and EcoRI adaptors having the base sequence of CTCGTAGACTGCGTACC (S.E.Q. ID. NO. 14) and AATTGGTACGCAGTCTAC (S.E.Q. ID. NO. 15) were ligated to a DNA fragment obtained by digesting the genomic DNA of a Gramineae plant such as barley with restriction enzymes MseI and EcoRI. Then, the ligated DNA fragment was pre-amplified with an MseI universal primer having the base sequence of GATGAGTCCTGAGTAA (S.E.Q. ID. NO. 19), and an EcoRI universal primer having the base sequence of GACTGCGTACCAATTC (S.E.Q. ID. NO. 18). The pre-amplified fragment obtained by the pre-amplification was amplified with the eleventh primer set including a primer having the base sequence of GATGAGTCCTGAGTAAATC (S.E.Q. ID. NO. 32), and a primer having the base sequence of GACTGCGTACCAATTCTGA (S.E.Q. ID. NO. 33). The genetic marker is located on a position distanced from the Fusarium head blight-resistance factor (locus relating to the Fusarium head blight resistance) by approximately 0.1 centiMorgan (hereinafter, cM) on the short-arm side (5' end side).

The AFLP mentioned above, and procedure of detection of the AFLP described below are not particularly limited, and may be carried out by a method described in the literature (Pieter Vos, Rene Hogers, Marjo Bleeker, Martin Reijans, Theo van de Lee, Miranda Hornes, Adrie Frijters, Jerina Pot, Johan Peleman, Martin Kuiper and Marc Zabeau. (1995) AFLP: a new technique for DNA fingerprinting. Nucleic Asids Research. 23:21:4407-4414.), or a modified method thereof. Moreover, amplification reactions of PCR and the like may by performed under generally-adopted conditions or optimal conditions appropriately chosen.

In the amplified fragments finally obtained by amplification with the eleventh primer set, there are resistant type (Russia 6 type) and susceptible type (H.E.S. 4 type) to Fusarium head blight. The amplified fragment of the resistant type (Russia 6 type) has a fragment length of approximately 128bp, whereas the amplified fragment of the susceptible type (H.E.S. type) has a fragment length of 0bp. In other words, an amplified fragment of approximately 128bp is obtained from the resistant type (Russia 6 type) against the Fusarium head blight, but no amplified fragment of approximately 128bp is obtained from the susceptible type (H.E.S. 4 type) to the Fusarium head blight. Thus, by checking, with a well-known means such as electrophoresis or the like, a fragment length of the amplification product obtained by the AFLP detection operation, it is possible to test a plant to judge whether a gene allelic to the Fusarium head blight-resistance factor (locus relating to the Fusarium head blight resistance) located on 4H chromosome is of resistant genotype or of susceptible genotype.

"FMgcgEatc530" is a genetic marker that is detected by so-called AFLP (Amplified Fragment Length Polymorphisms). AFLP is performed by as follows. MseI adaptors having the base sequences of GACGATGAGTCCTGAG (S.E.Q. ID. NO. 16) and TACTCAGGACTCAT (S.E.Q. ID. NO. 17) and EcoRI adaptors having the base sequence of CTCGTAGACTGCGTACC (S.E.Q. ID. NO. 14) and AATTGGTACGCAGTCTAC (S.E.Q. ID. NO. 15) were ligated to a DNA fragment obtained by digesting the genomic DNA of a Gramineae plant such as barley with restriction enzymes MseI and EcoRI. Then, the ligated DNA fragment was pre-amplified with an MseI universal primer having the base sequence of GATGAGTCCTGAGTAA (S.E.Q. ID. NO. 19), and an EcoRI universal primer having the base sequence of GACTGCGTACCAATTC (S.E.Q. ID. NO. 18). The pre-amplified fragment obtained by the pre-amplification was amplified with the twelfth primer set including a primer having the base sequence of GATGAGTCCTGAGTAAATC (S.E.Q. ID. NO. 34), and a primer having the base sequence of GACTGCGTACCAATTCGCG (S.E.Q. ID. NO. 35). The genetic marker is located on a position distanced from the Fusarium head blight-resistance factor (locus relating to the Fusarium head blight resistance) by approximately 8.0 cM on the long-arm side (3' end side) of 4H chromosome.

In the amplified fragments finally obtained by amplification with the twelfth primer set, there are resistant type (Russia 6 type) and susceptible type (H.E.S. 4 type) to Fusarium head blight. The amplified fragment of the resistant type (Russia 6 type) has a fragment length of 0bp, whereas the amplified fragment of the susceptible type (H.E.S. type) has a fragment length of approximately 530bp. In other words, no amplified fragment of approximately 530bp is obtained from the resistant type (Russia 6 type) against the Fusarium head blight, but an amplified fragment of approximately 530bp is obtained from the susceptible type (H.E.S. 4 type) to the Fusarium head blight. Thus, by checking, with a well-known means such as electrophoresis or the like, a fragment length of the amplification product obtained by the AFLP detection operation, it is possible to test a plant to judge whether a gene allelic to the Fusarium head blight-resistance factor (locus relating to the Fusarium head blight resistance) located on 4H chromosome is of resistant genotype or of susceptible genotype.

A novel genetic marker, namely "FXLRRfor_XLRRrev119", linked to the Fusarium head blight-resistance factor (locus relating to the Fusarium head blight resistance) on 2H chromosome was detected in the RI2 population.

"FXLRRfor_XLRRrev119" is a so-called RGA (Resistant Gene Analogs) marker, which is amplified with the sixth primer set that is a combination of the primer having the base sequence of CCGTTGGACAGGAAGGAG (S.E.Q. ID. NO. 22) and the primer having the base sequence of CCCATAGACCGGACTGTT (S.E.Q. ID. NO. 23). FXLRRfor_XLRRrev119 is located at a position distanced from the Fusarium head blight-resistance factor (locus relating to the Fusarium head blight resistance) by approximately 0.2cM on the short-arm side of 2H chromosome. The primer sequence is designed from base sequence information (gene bank accession No: U37133) of Oryza sativa receptor kinase-like protein (Xa21) gene.

The procedure of the detection of RGA marker is not particularly limited, and may be performed a method described in the literature (Chen XM, Line RF, Leung H (1998) Genome scanning for resistance-gene analogs in rice; barley, and wheat by high-resolution electrophoresis. Theor Appl Genet 98: 345-355) or a modified method thereof. Moreover, amplification reactions of PCR and the like may be performed under generally-adopted conditions or optimal conditions appropriately chosen.

In the amplified fragments obtained by amplification with the sixth primer set, there are resistant type (Harbin 2-row type) and susceptible type (Turkey 6 type) to Fusarium head blight. The amplified fragment of the resistant type (Harbin 2-row type) has a fragment length of 0bp, whereas the amplified fragment of the susceptible type (Turkey 6 type) has a fragment length of approximately 119bp. In other words, no amplified fragment of approximately 119bp is obtained from the resistant type (Harbin 2-row type) against the Fusarium head blight, but an amplified fragment of approximately 119bp is obtained from the susceptible type (Turkey 6 type) to the Fusarium head blight. Thus, by checking, with a well-known means such as electrophoresis or the like, a fragment length of the amplification product obtained by the above-mentioned operation, it is possible to test a plant to judge whether a gene allelic to the Fusarium head blight-resistance factor (locus relating to the Fusarium head blight resistance) located on 2H chromosome is of resistant genotype or of susceptible genotype.

Novel genetic markers, namely "FMacgEcgt288" and "HVM67", linked to the Fusarium head blight-resistance factor (locus relating to the Fusarium head blight resistance) on 4H chromosome was detected in the RI2 population.

"FMgcgEatc530" is a genetic marker that is detected by so-called AFLP (Amplified Fragment Length Polymorphism). AFLP is performed by as follows. MseI adaptors having the base sequences of GACGATGAGTCCTGAG (S.E.Q. ID. NO. 16) and TACTCAGGACTCAT (S.E.Q. ID. NO. 17) and EcoRI adaptors having the base sequence of CTCGTAGACTGCGTACC (S.E.Q. ID. NO. 14) and AATTGGTACGCAGTCTAC (S.E.Q. ID. NO. 15) were ligated to a DNA fragment obtained by digesting the genomic DNA of a Gramineae plant such as barley with restriction enzymes MseI and EcoRI. Then, the ligated DNA fragment was pre-amplified with an MseI universal primer having the base sequence of GATGAGTCCTGAGTAA (S.E.Q. ID. NO. 19), and an EcoRI universal primer having the base sequence of GACTGCGTACCAATTC (S.E.Q. ID. NO. 18). The pre-amplified fragment obtained by the pre-amplification was amplified with the thirteen primer set including a primer having the base sequence of GATGAGTCCTGAGTAAACG (S.E.Q. ID. NO. 36), and a primer having the base sequence of GACTGCGTACCAATTCCGT (S.E.Q. ID. NO. 37). The genetic marker is located on a position distanced from the Fusarium head blight-resistance factor (locus relating to the Fusarium head blight resistance) by approximately 0.3 cM on the short-arm side of 4H chromosome.

In the amplified fragments finally obtained by amplification with the thirteen primer set, there are resistant type (Harbin 2-row type) and susceptible type (Turkey 6 type) to Fusarium head blight. The amplified fragment of the resistant type (Harbin 2-row type) has a fragment length of 0bp, whereas the amplified fragment of the susceptible type (Turkey 6 type) has a fragment length of approximately 288bp. In other words, no amplified fragment of approximately 288bp is obtained from the resistant type (Harbin 2-row type) against the Fusarium head blight, but an amplified fragment of approximately 288bp is obtained from the susceptible type (Turkey 6 type) to the Fusarium head blight. Thus, by checking, with a well-known means such as electrophoresis or the like, a fragment length of the amplification product obtained by the AFLP detection operation, it is possible to test a plant to judge whether a gene allelic to the Fusarium head blight-resistance factor (locus relating to the Fusarium head blight resistance) located on 4H chromosome is of resistant genotype or of susceptible genotype.

"HVM67" is a genetic marker, which is amplified using the barley genomic DNA as a template with the fourteenth primer set that is a combination of the primer having the base sequence of GTCGGGCTCCATTGCTCT (S.E.Q. ID. NO. 38) and the primer having the base sequence of CCGGTACCCAGTGACGAC (S.E.Q. ID. NO. 39). HVM67 is located at a position distanced from the Fusarium head blight-resistance factor (locus relating to Fusarium head blight resistance) by approximately 46.8cM on the long-arm side of 4H chromosome. This genetic marker is a so-called SSR (Simple Sequence Repeat) marker.

Amplification for HVM67 is not particularly limited, and may be performed under optimum conditions appropriately chosen.

In the amplified fragments obtained by amplification with the fourteenth primer set, there are resistant type (Harbin 2-row type) and susceptible type (Turkey 6 type) to Fusarium head blight. The amplified fragment of the resistant type (Harbin 2-row type) has a fragment length of 160bp, whereas the amplified fragment of the susceptible type (Turkey 6 type) has a fragment length of approximately 140bp. Thus, by checking, with a well-known means such as electrophoresis or the like, a fragment length of the amplification product obtained by the above-mentioned operation, it is possible to test a plant to judge whether a gene allelic to the Fusarium head blight-resistance factor (locus relating to the Fusarium head blight resistance) located on 4H chromosome is of resistant genotype or of susceptible genotype.

Novel genetic markers, namely "FMataEagc408" and "HVM11", linked to the Fusarium head blight-resistance factor (locus relating to the Fusarium head blight resistance) on 6H chromosome was detected in the RI2 population.

"FMataEagc408" is a genetic marker that is detected by so-called AFLP (Amplified Fragment Length Polymorphism). AFLP is performed by as follows. MseI adaptors having the base sequences of GACGATGAGTCCTGAG (S.E.Q. ID. NO. 16) and TACTCAGGACTCAT (S.E.Q. ID. NO. 17) and EcoRI adaptors having the base sequence of CTCGTAGACTGCGTACC (S.E.Q. ID. NO. 14) and AATTGGTACGCAGTCTAC (S.E.Q. ID. NO. 15) were ligated to a DNA fragment obtained by digesting the genomic DNA of a Gramineae plant such as barley with restriction enzymes MseI and EcoRI. Then, the ligated DNA fragment was pre-amplified with an MseI universal primer having the base sequence of GATGAGTCCTGAGTAA (S.E.Q. ID. NO. 19), and an EcoRI universal primer having the base sequence of GACTGCGTACCAATTC (S.E.Q. ID. NO. 18). The pre-amplified fragment obtained by the pre-amplification was amplified with the seventeenth primer set including a primer having the base sequence of GATGAGTCCTGAGTAAATA (S.E.Q. ID. NO. 44), and a primer having the base sequence of GACTGCGTACCAATTCAGC (S.E.Q. ID. NO. 45). The genetic marker is located on a position distanced from the Fusarium head blight-resistance factor (locus relating to the Fusarium head blight resistance) by approximately 4.1 cM on the short-arm side of 6H chromosome.

In the amplified fragments finally obtained by amplification with the seventeenth primer set, there are resistant type (Harbin 2-row type) and susceptible type (Turkey 6 type) to Fusarium head blight. The amplified fragment of the resistant type (Harbin 2-row type) has a fragment length of 0bp, whereas the amplified fragment of the susceptible type (Turkey 6 type) has a fragment length of approximately 408bp. In other words, no amplified fragment of approximately 408bp is obtained from the resistant type (Harbin 2-row type) against the Fusarium head blight, but an amplified fragment of approximately 408bp is obtained from the susceptible type (Turkey 6 type) to the Fusarium head blight. Thus, by checking, with a well-known means such as electrophoresis or the like, a fragment length of the amplification product obtained by the detection operation, it is possible to test a plant to judge whether a gene allelic to the Fusarium head blight-resistance factor (locus relating to the Fusarium head blight resistance) located on 6H chromosome is of resistant genotype or of susceptible genotype.

"HVM 11" is a genetic marker, which is amplified using the barley genomic DNA as a template with the eighteenth primer set that is a combination of the primer having the base sequence of CCGGTCGGTGCAGAAGAG (S.E.Q. ID. NO. 46) and the primer having the base sequence of AAATGAAAGCTAAATGGGCGATAT (S.E.Q. ID. NO. 47). HVM11 is located at a position distanced from the Fusarium head blight-resistance factor (locus relating to Fusarium head blight resistance) by approximately 9.4cM on the long-arm side of 6H chromosome. This genetic marker is a so-called SSR (Simple Sequence Repeat) marker.

Amplification for HVM 11 is not particularly limited, and may be performed under optimum conditions appropriately chosen.

In the amplified fragments obtained by amplification with the eighteenth primer set, there are resistant type (Harbin 2-row type) and susceptible type (Turkey 6 type) to Fusarium head blight. The amplified fragment of the resistant type (Harbin 2-row type) has a fragment length of 144bp, whereas the amplified fragment of the susceptible type (Turkey 6 type) has a fragment length of approximately in a range of 100bp to 160bp. Thus, by checking, with a well-known means such as electrophoresis or the like, a fragment length of the amplification product obtained by the above-mentioned operation, it is possible to test a plant to judge whether a gene allelic to the Fusarium head blight-resistance factor (locus relating to the Fusarium head blight resistance) located on 6H chromosome is of resistant genotype or of susceptible genotype.

Novel genetic markers, namely "Bmag125" and "k04002", linked to the Fusarium head blight-resistance factor (locus relating to the Fusarium head blight resistance) on 2H chromosome was detected in the DHHS population.

"Bmag125" is a genetic marker, which is amplified using the barley genomic DNA as a template with the seventh primer set that is a combination of the primer having the base sequence of AATTAGCGAGAACAAAATCAC (S.E.Q. ID. NO. 24) and the primer having the base sequence of AGATAACGATGCACCACC (S.E.Q. ID. NO. 25). Bmag125 is located at a position distanced from the Fusarium head blight-resistance factor (locus relating to Fusarium head blight resistance) by approximately 0cM on the short-arm side of 2H chromosome. This genetic marker is a so-called SSR (Simple Sequence Repeat) marker.

Amplification for Bmag125 is not particularly limited, and may be performed under optimum conditions appropriately chosen.

In the amplified fragments obtained by amplification with the seventh primer set, there are resistant type (Haruna Nijo type) and susceptible type (H602 type) to Fusarium head blight. The amplified fragment of the resistant type (Haruna Nijo type) has a fragment length of approximately 142bp, whereas the amplified fragment of the susceptible type (H602 type) has a fragment length of approximately 134bp. Thus, by checking, with a well-known means such as electrophoresis or the like, a fragment length of the amplification product obtained by the above-mentioned operation, it is possible to test a plant to judge whether a gene allelic to the Fusarium head blight-resistance factor (locus relating to the Fusarium head blight resistance) located on 2H chromosome is of resistant genotype or of susceptible genotype.

"k04002" is a genetic marker, which is amplified with the eighth primer set that is a combination of the primer having the base sequence of GACACAGGACCTGAAGCACA (S.E.Q. ID. NO. 26) and the primer having the base sequence of CGGCAGGCTCTACTATGAGG (S.E.Q. ID. NO. 27). k04002 is located at a position distanced from the Fusarium head blight-resistance factor (locus relating to the Fusarium head blight resistance) by approximately 10cM on the long-arm side of 2H chromosome. The primer sequence is designed based on one of barley EST sequences uniquely developed by the inventors of the present invention (EST clone name: bah32c06, SEQ. ID. NOs. 55 and 56). Note that S.E.Q. ID. NO. 40 shows the base sequence from the 5' end, while S.E.Q. ID. NO. 41 shows the base sequence from the 3' end. Amplification for k04002 is not particularly limited, and may be performed under optimum conditions appropriately chosen.

In the amplified fragments obtained by amplification with the eighth primer set, there are resistant type (Haruna Nijo type) and susceptible type (H602 type) to Fusarium head blight. The amplified fragment of the resistant type (Haruna Nijo type) has a fragment length of approximately 350bp, whereas the amplified fragment of the susceptible type (H602 type) has a fragment length of approximately 440bp. Thus, by checking, with a well-known means such as electrophoresis or the like, a fragment length of the amplification product obtained by the above-mentioned detection operation, it is possible to test a plant to judge whether a gene allelic to the Fusarium head blight-resistance factor (locus relating to the Fusarium head blight resistance) located on 2H chromosome is of resistant genotype or of susceptible genotype.

Novel genetic markers, namely "k05042" and "k03289", linked to the Fusarium head blight-resistance factor (locus relating to the Fusarium head blight resistance) on 4H chromosome was detected in the DHHS population.

"k05042" is a genetic marker, which is amplified using the barley genomic DNA as a template with the fifteenth primer set that is a combination of the primer having the base sequence of ATACATGCATGCCATTGTGG (S.E.Q. ID. NO. 40) and the primer having the base sequence of ATCCATCCACTGTTTGAGGG (S.E.Q. ID. NO. 41). k05042 is located at a position distanced from the Fusarium head blight-resistance factor (locus relating to Fusarium head blight resistance) by approximately 1.2cM on the short-arm side of 4H chromosome. The primer sequence is designed based on one of barley EST sequences uniquely developed by the inventors of the present invention (EST clone name: basdle04, SEQ. ID. NO. 48).

Amplification for k05042 is not particularly limited, and may be performed under optimum conditions appropriately chosen.

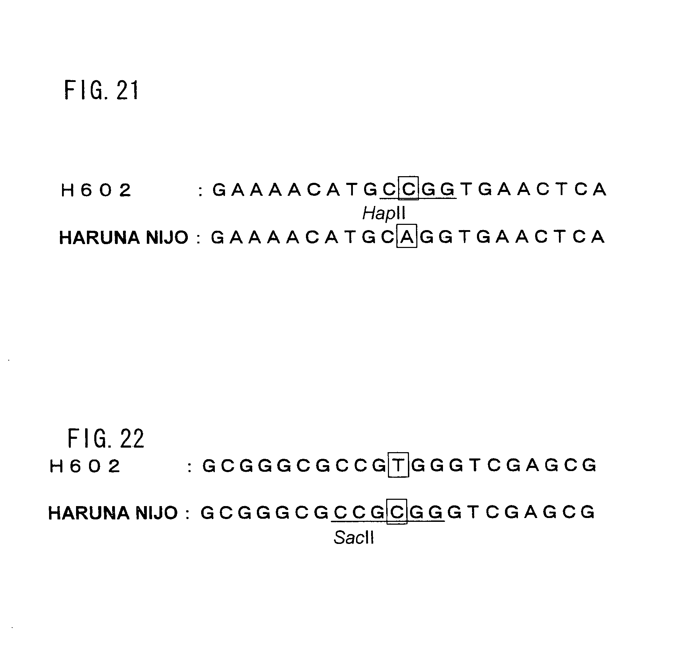

In the amplification fragment, which is amplified with the fifteenth primer set, there is a resistant type and susceptible type to Fusarium head blight, and there is an SNP (single nucleotide polymorphism) therebetween. FIG. 21 illustrates that portions of the base sequences of the amplification products of both the types, which are including the SNPs. The lower one is the base sequence of the susceptible type (Haruna Nijo type) (S.E.Q. ID. NO. 51), and the upper one is resistant type (H602 type) (S.E.Q. ID. NO. 52). The bases surrounded with a square are the SNPs. The underline indicates the recognition sequence (CCGG) of the restriction enzyme HapII. As clearly understood from FIG. 21, the amplification product of the resistant type, which has the recognition sequence (CCGG) of the restriction enzyme HapII, is cleaved by HapII. However, the amplification product of the susceptible type is not cleaved by HapII, because mutation to replace one C in the identification sequence with A (CAGG) occurs. That is, this genetic marker k05042 is a CAPS (cleaved amplified polymorphic sequence) marker. Based on the amplification with the fifteenth primer set and the cleavage of the amplification product with the restriction enzyme HapII, it is possible to test a plant to judge whether a gene allelic to the Fusarium head blight-resistance factor (locus relating to the Fusarium head blight resistance) located on 4H chromosome is of resistant genotype or of susceptible genotype.

The Fusarium head blight-resistance factor (locus relating to Fusarium head blight resistance) to which this genetic marker is linked is the Fusarium head blight-resistance factor (locus relating to Fusarium head blight resistance) that H602, which is a Fusarium head blight-resistant cultivar, has. For the detection of this genetic marker, the Haruna Nijo type is the susceptible type and the H602 type is the resistant type.

"k03289" is a genetic marker, which is amplified with the sixteenth primer set that is a combination of the primer having the base sequence of TGCTCTGCATTTCATTCAGC (S.E.Q. ID. NO. 42) and the primer having the base sequence of CAGCGTTACAGGCATTCTCA (S.E.Q. ID. NO. 43). k03289 is located at a position distanced from the Fusarium head blight-resistance factor (locus relating to the Fusarium head blight resistance) by approximately 4.0 cM on the long-arm side of 4H chromosome. The primer sequence is designed based on one of barley EST sequences uniquely developed by the inventors of the present invention (EST clone name: bags3h19, SEQ. ID. NO. 49).

Amplification for k03289 is not particularly limited, and may be performed under optimum conditions appropriately chosen.

In the amplification fragment, which is amplified with the sixteenth primer set, there is a resistant type and susceptible type to Fusarium head blight, and there is an SNP (single nucleotide polymorphism) therebetween. FIG. 22 illustrates that portions of the base sequences of the amplification products of both the types, which are including the SNP. The lower one is the base sequence of the susceptible type (Haruna Nijo type) (S.E.Q. ID. NO. 53), and the upper one is resistant type (H602 type) (S.E.Q. ID. NO. 54). The bases surrounded with a square are the SNPs. The underline indicates the recognition sequence (CCGCGG) of the restriction enzyme SacII. As clearly understood from FIG. 22, the amplification product of the susceptible type, which has the recognition sequence (CCGCGG) of the restriction enzyme SacII, is cleaved by SacII. However, the amplification product of the resistant type is not cleaved by SacII, because mutation to replace one C in the identification sequence with T occurs (CCGTGG). That is, this genetic marker k03289 is a CAPS (cleaved amplified polymorphic sequence) marker. Based on the amplification with the sixteenth primer set and the cleavage of the amplification product with the restriction enzyme SacII, it is possible to test a plant to judge whether a gene allelic to the Fusarium head blight-resistance factor (locus relating to the Fusarium head blight resistance) located on 4H chromosome is of resistant genotype or of susceptible genotype.

The Fusarium head blight-resistance factor (locus relating to Fusarium head blight resistance) to which this genetic marker is linked is the Fusarium head blight-resistance factor (locus relating to Fusarium head blight resistance) that H602, which is a Fusarium head blight-resistant cultivar, has. For the detection of this genetic marker, the Haruna Nijo type is the susceptible type and the H602 type is the resistant type.

Novel genetic markers, namely "HvLOX" and "k00835", linked to the Fusarium head blight-resistance factor (locus relating to the Fusarium head blight resistance) on 5H chromosome was detected in the DHHS population.

"HvLOX" is a genetic marker, which is amplified using the barley genomic DNA as a template with the ninth primer set that is a combination of the primer having the base sequence of CAGCATATCCATCTGATCTG (S.E.Q. ID. NO. 28) and the primer having the base sequence of CACCCTTATTTATTGCCTTAA (S.E.Q. ID. NO. 29). HvLOX is located at a position distanced from the Fusarium head blight-resistance factor (locus relating to Fusarium head blight resistance) by approximately 0.5cM on the short-arm side of 5H chromosome. This genetic marker is a so-called SSR (Simple Sequence Repeat) marker.

Amplification for HvLOX is not particularly limited, and may be performed under optimum conditions appropriately chosen.

In the amplified fragments obtained by amplification with the ninth primer set, there are resistant type (Haruna Nijo type) and susceptible type (H602 type) to Fusarium head blight. The amplified fragment of the resistant type (Haruna Nijo type) has a fragment length of approximately 155bp, whereas the amplified fragment of the susceptible type (H602 type) has a fragment length of approximately 157bp. Thus, by checking, with a well-known means such as electrophoresis or the like, a fragment length of the amplification product obtained by the above-mentioned operation, it is possible to test a plant to judge whether a gene allelic to the Fusarium head blight-resistance factor (locus relating to the Fusarium head blight resistance) located on 5H chromosome is of resistant genotype or of susceptible genotype.

"k00835" is a genetic marker, which is amplified with the tenth primer set that is a combination of the primer having the base sequence of TCCATGTTCCCAGCTACACA (S.E.Q. ID. NO. 30) and the primer having the base sequence of AGGAACACATTGGTTCTGGC (S.E.Q. ID. NO. 31). k00835 is located at a position distanced from the Fusarium head blight-resistance factor (locus relating to the Fusarium head blight resistance) by approximately 1.9cM on the long-arm side of 5H chromosome. The primer sequence is designed based on one of barley EST sequences uniquely developed by the inventors of the present invention (EST clone name: baak46e06, SEQ. ID. NO. 50).

Amplification for k00835 is not particularly limited, and may be performed under optimum conditions appropriately chosen.

In the amplified fragments obtained by amplification with the tenth primer set, there are resistant type (Haruna Nijo type) and susceptible type (H602 type) to Fusarium head blight. The amplified fragment of the resistant type (Haruna Nijo type) has a fragment length of approximately 900bp, whereas the amplified fragment of the susceptible type (H602 type) has a fragment length of approximately 880bp. Thus, by checking, with a well-known means such as electrophoresis or the like, a fragment length of the amplification product obtained by the above-mentioned detection operation, it is possible to test a plant to judge whether a gene allelic to the Fusarium head blight-resistance factor (locus relating to the Fusarium head blight resistance) located on 5H chromosome is of resistant genotype or of susceptible genotype.

Here, morgan (M) is explained. 1 morgan (M), which is a unit of a distance on a chromosome, corresponds to such a probability that one crossover event occurs during one meiosis on average. For example, 2.2 cM indicates that recombination between the Fusarium head blight-resistance factor and the genetic marker occurs 22/ 1000 times per chromatid on average. That is, it is indicated that recombination percentage is approximately 2.2% in this case.

Incidentally, the genomic DNA used as the template in the amplification can be extracted from a plant tissue of barley in conventional and well-known methods. Specifically, One suitable example of such methods is a generally-used method for extracting a genomic DNA from a plant tissue (see Murray, M.G. and W.F.Thompson (1980) Nucleic Acids Res. 8: 4321-4325, etc.) Moreover, the genomic DNA can be extracted from any tissues such as roots, stems, leaves, reproductive organs, and the like, which constitute the plant tissue of the barley. Moreover, in some cases, the genomic DNA can be extracted from callus of barley. The reproductive organs encompasses flower organ (including male/female reproductive organs) and seeds. The genomic DNA is extracted, e.g., from a leaf of barley during seedling stage. This is because trituration of the tissue is relatively easy, a mixture ratio of impurity such as polysaccarides is relatively low, and only short time is necessary to grow a plant from a seed to seedling.

The amplification using the genomic DNA of barley as a template and the combination of the primers can be performed by a conventional and well-known DNA amplification method. In general, a PCR method (polymerase chain reaction method), or a modified PCR method is used. There is no particular limitation as to conditions under with the PCR method or the modified PCR method is performed. It is possible to perform the PCR method or the modified PCR method under conditions similar to generally adopted conditions.

The use of a genetic marker according to the present invention makes it possible to isolate a DNA fragment including the Fusarium head blight-resistance factor (locus relating to Fusarium head blight resistance). The DNA fragment can be utilized for finding a gene relating to the Fusarium head blight resistance of barley, and for understanding the mechanism of the Fusarium head blight resistance. Moreover, by introducing the DNA fragment into a plant (e.g., a Hordeum or the like such as barley), it is possible to produce (breed) a Fusarium head blight-resistant plant.

Moreover, the genetic markers are linked to the Fusarium head blight-resistance factors (loci relating to the Fusarium head blight resistance). Thus, it is possible to test a plant (a Gramineae plant (e.g., Hordeum or the like such as barley)) to judge whether the plant (a Gramineae plant (e.g., Hordeum or the like such as barley)) has a Fusarium head blight-resistance factor or not, by detecting for polymorphism of the genetic markers in the genomic DNA of the plant. Similarly, because the genetic markers are linked to the Fusarium head blight-resistance factors (loci relating to the Fusarium head blight resistance), it is possible to test a plant (a Gramineae plant (e.g., Hordeum or the like such as barley)) to judge whether the plant (a Gramineae plant (e.g., Hordeum or the like such as barley)) is a Fusarium head blight-resistant plant or not, by detecting for polymorphism of the genetic markers in the genomic DNA of the plant. A kit developed by comprising a primer for amplification of any of the genetic markers or a DNA microarray to which any of the genetic markers is fixed can be provided as a kit for judging whether or not a Fusarium head blight-resistance factor are present in a plant (e.g., Hordeum or the like such as barley), or a kit for judging whether a plant is a Fusarium head blight-resistant plant or not.

As described above, the genetic marker according to the present invention is widely applicable to various uses evidently. Examples of the uses of the genetic markers according to the present invention will be explained later in detail.

### (2) Use of Genetic Marker According to the Present Invention

### <Method for Isolating DNA Fragment Including Fusarium Head Blight-Resistance Factor (Locus Relating to Fusarium Head Blight Resistance)>

As described above, the genetic markers, "FXLRRfor_XLRRrev119", "Bmag125", and "k04002" according to the present invention are linked to the Fusarium head blight-resistance-factors (loci relating to Fusarium head blight resistance) detected on barley 2H chromosome. The genetic markers, "MMtgaEatc128", "FMgcgEatc530", "FMacgEcgt288", "HVM67", "k05042", and "k03289" according to the present invention are linked to the Fusarium head blight-resistance-factors (loci relating to Fusarium head blight resistance) detected on barley 4H chromosome. the genetic markers, "HvLOX", and "k00835" according to the present invention are linked to the Fusarium head blight-resistance-factors (loci relating to Fusarium head blight resistance) detected on barley 5H chromosome. The genetic markers, "FMataEagc408", and "HVM 11" according to the present invention are linked to the Fusarium head blight-resistance-factors (loci relating to Fusarium head blight resistance) detected on barley 6H chromosome.

Therefore, by using any of the genetic markers "FXLRRfor_XLRRrev119", "Bmag125", and "k04002", it is possible to isolate a DNA fragment including a Fusarium head blight-resistance (locus relating to Fusarium head blight resistance) detected on barley 2H chromosome. By using any of the genetic markers "MMtgaEatc 128", "FMgcgEatc530", "FMacgEcgt288", "HVM67", "k05042", and "k03289", it is possible to isolate a DNA fragment including a Fusarium head blight-resistance (locus relating to Fusarium head blight resistance) detected on barley 4H chromosome. By using any of the genetic markers "HvLOX" and "k00835", it is possible to isolate a DNA fragment including a Fusarium head blight-resistance (locus relating to Fusarium head blight resistance) detected on barley 5H chromosome. By using any of the genetic markers "FMataEagc408", and "HVM11", it is possible to isolate a DNA fragment including a Fusarium head blight-resistance (locus relating to Fusarium head blight resistance) detected on barley 6H chromosome.

The term "isolation" encompasses, needless to say, cloning of a targeted DNA fragment, that is, cloning of a DNA fragment including the Fusarium head blight-resistance factor (locus relating to Fusarium head blight resistance), but in a broad sense, the term "isolation" also encompasses preparation of homozygous gene line by introducing, into a target cultivar, a genomic region of a plant to which one of the parents has given a DNA fragment including the Fusarium head blight-resistance factor (locus relating to Fusarium head blight resistance), the plant being selected by backcross of a hybrid F1 or the like method.

There is no particular limitation in how to isolate the DNA fragment including the Fusarium head blight-resistance factor (locus relating to Fusarium head blight resistance) by using the genetic marker according to the present invention. For example, the isolation may be carried out as follows.

There has been developed two BAC libraries of genomic DNA, encompassing "Haruna Nijo" that has been developed by the inventors of the present invention, and some more BAC libraries are under development at this moment. By using such a BAC library, identification of a BAC clone including the genetic marker of the present invention can be performed with the Fusarium head blight-resistance factors and the genetic markers according to the present invention by using a conventional and well-known map based cloning method. Based on the identification, it is possible to prepare a contiguous sequences of BAC, thereby identifying its base sequence. This finally leads to finding of the Fusarium head blight-resistance factors.

Moreover, as described above, it is possible to perform the isolation (in a broad sense) by backcrossing a hybrid F1 with one of its parents, and introducing, into a target cultivar, a DNA fragment including a Fusarium head blight-resistance factor (locus relating to the Fusarium head blight resistance) of the other parent.

It is preferable that the isolation of the DNA fragment including the Fusarium head blight-resistance factor (locus relating to the Fusarium head blight resistance) be performed by using a genetic marker located closer to the targeted Fusarium head blight-resistance factor (locus relating to the Fusarium head blight resistance). This lowers the possibility that recombination occurs between the targeted Fusarium head blight-resistance factor (locus relating to the Fusarium head blight resistance) and the genetic marker. Therefore, this makes it possible to more surely isolate the fragment including the Fusarium head blight-resistance factor (locus relating to the Fusarium head blight resistance).

### <Method for Producing Fusarium Head Blight-Resistant Plant (Gramineae plant), and Fusarium Head Blight-Resistant Plant (Gramineae plant) obtained by Same Method>

It is possible to produce a Fusarium head blight-resistant plant (Gramineae plant) by introducing, into genomic DNA of a plant (Gramineae plant), a DNA fragment including the Fusarium head blight-resistance factor, the DNA fragment being obtained by the isolation method according to the present invention for isolating the DNA fragment including the Fusarium head blight-resistance factor. It is preferable that the plant be a Gramineae plant. It is preferable that the Gramineae plant be Hordeum or the like, and it is especially preferable that the Gramineae plant be barley. Moreover, for example, it is possible to introduce, into a plant (Gramineae plant) other than barley, a DNA fragment including the Fusarium head blight-resistance factor, the DNA fragment being isolated from barley. Thus, the present invention is not limited to the isolation and implementation between the same cultivar.

The implementation of the DNA fragment is not particularly limited, and may be performed by a well-known method appropriately selected. More specifically, the production may be performed by introducing the DNA fragment into genomic DNA of a plant (Gramineae plant) by using, for example, *Agrobacterium* or particle gun method. In this way, a Fusarium head blight resistant cultivar can be obtained. For example, in a magazine "The Plant Journal" (1997) 11(6),1369-137, Sonia Tingay et al. disclose a method for transforming barley using *Agrobacterium tumefaciens.* It is possible to produce a transformant barley by using this method.

Moreover, it is possible to implement, into a plant, a DNA fragment including a targeted Fusarium head blight-resistance factor (locus relating to Fusarium head blight resistance) by crossing (a) a plant having the targeted Fusarium head blight-resistance factor (locus relating to the Fusarium head blight resistance) with (b) a plant to which the DNA fragment is to be introduced. With this method, it is possible to implement the DNA fragment having the Fusarium head blight-resistance factor (locus relating to Fusarium head blight resistance) from a plant of one cultivar or genus to a plant of another specie or genus.

The Fusarium head blight-resistant plant (Gramineae plant) according to the present invention is obtained by the method according to the present invention for producing the same. The Fusarium head blight-resistant plant (Gramineae plant) will be a cultivar having a higher Fusarium head blight resistance than an original cultivar if a DNA fragment including a locus having a phenotype of the Fusarium head blight resistance (Fusarium head blight-resistant type). The Fusarium head blight-resistant plant (Gramineae plant) will be a cultivar having a lower Fusarium head blight resistance than an original cultivar if a DNA fragment including a locus having a phenotype of the Fusarium head blight susceptibility (Fusarium head blight-susceptible type). Thus, it is preferable to implement a DNA fragment including a locus of the Fusarium head blight-resistant type.

Furthermore, it is possible to produce a Fusarium head blight-resistant plant (a Gramineae plant (e.g., Hordeum or the like such as barley) by introducing a plant (e.g., Hordeum or the like such as barley) any of the DNA fragments on 2H chromosome, 4H chromosome, 5H chromosome, and 6H chromosome. Meanwhile, it is possible to produce a Fusarium head blight-resistant plant (a Gramineae plant) having a further higher Fusarium head blight-resistance by introducing, into one plant, a plurality of DNA fragments into the same plant (a Gramineae plant (e.g., Hordeum or the like such as barley)).

The inventors of the present invention confirmed that a higher Fusarium head blight resistance was attained by introducing, into barley in which the DNA fragment on the 2H fragment or 5H fragment is implement, a DNA fragment (hereinafter, vrs1 fragment) including the Fusarium head blight-resistance factor (locus relating to the Fusarium head blight resistance) that matches with the locus of the row type gene vrs1, compared with the barley in which the DNA fragment on the 2H fragment or 5H fragment is implement solely. Furthermore, the inventors of the present invention confirmed that the implementation of the vrs1 fragment into barley in which the DNA fragments on the 2H fragment and 5H fragment is introduced improves the Fusarium head blight resistance of the barley.

With the Fusarium head blight-resistant Gramineae plant, such as the Fusarium head blight-resistant barley, obtained by the method according to the present invention, it becomes possible to effectively avoid the Fusarium head blight-causing quality deterioration and crop yield reduction, and damages to human and domestic animals caused when they are fed with the plant infected with the Fusarium head blight. Thus, the Fusarium head blight-resistant plant is quite effective in the agricultural and livestock industries and the like. Further, the Fusarium head blight-resistant plant can alleviate food shortage problem.

The terms "Fusarium head blight-resistant plant", "Fusarium head blight-resistant Gramineae", and "Fusarium head blight-resistant barley" encompass a plant (e.g., Gramineae plant such as barley) that originally has no Fusarium head blight resistance at all but is bred to have the Fusarium head blight resistance, and a plant (e.g., Gramineae plant such as barley) that originally has the Fusarium head blight resistance, but is bred to have an improved Fusarium head blight resistance.

### <Method for Judging Whether Plant is Fusarium Head Blight Resistant Plant (Gramineae)>

The method according to the present invention for judging whether a plant is a Fusarium head blight resistant plant (Gramineae plant) (i.e., selecting a Fusarium head blight resistant plant (Gramineae plant)) should include detecting a genetic marker according to the present invention, and is not limited in terms of the another or other steps, conditions, materials, and the like. For example, it is possible to use a conventional and well-known method for breeding a crop.

More specifically, one example of such a method is a method including (a) extracting genomic DNA of a plant produced by crossing or the like, and then (b) performing the judgment (selection) for the plant using a genotype of the genetic marker according to the present invention. An example of means for detecting the genetic marker is to observe a DNA fragment in terms of its fragment length or its pattern of digestion with a restriction enzyme, the DNA fragment being amplified using genomic DNA extracted from the plant to be tested and any one of the first to eighteenth primer sets. Here, the test of a plant to judge, from a DNA fragment by using the respective genetic markers as indicators, whether a gene allelic to the locus relating to the Fusarium head blight resistance is of a resistant genotype or susceptible genotype is carried out as explained in the section discussing the genetic marker according to the present invention.

The accuracy (probability) of the judgment of the present judging method is as follows. Recombination between a Fusarium head blight-resistance factor (locus relating to the Fusarium head blight resistance) and a genetic marker located at a position distanced from the Fusarium head blight-resistance factor (locus relating to the Fusarium head blight resistance) by 1.2cM occurs 12/1000 times on average per chromatid. That is, the recombination occurs with a probability of approximately 1.2%. Thus, if a Fusarium head blight resistant type of this genetic marker was detected, it would be judged with 98.8% probability that the Fusarium head blight-resistance factor (locus relating to the Fusarium head blight resistance) is present. Thus, the judgment whether the Fusarium head blight-resistance factor (locus relating to the Fusarium head blight resistance) is present or not can be performed with high accuracy. That is, it is possible to perform the judgment (selection) of the Fusarium head blight-resistance Gramineae plant) with high accuracy.

From the reasons explained above, although any of the genetic markers to detect the polymorphism can be used to judge whether a Fusarium head blight resistance is present or not, it is preferable to arrange the method to detect a genetic marker located closer to the Fusarium head blight-resistance factor (locus relating to the Fusarium head blight resistance). For example, in case where the genetic marker linked to the Fusarium head blight-resistance factor (locus relating to the Fusarium head blight resistance) on 4H chromosome is to be detected in the present judging method, "k05042" is more preferable than "k03289", because k05042 is distanced by approximately 1.2cM from the Fusarium head blight-resistance factor (locus relating to the Fusarium head blight resistance) while k03289 is distanced by approximately 4.0cM from the Fusarium head blight-resistance factor (locus relating to the Fusarium head blight resistance).

The present judging method may be arranged to detect polymorphisms of a plurality of genetic markers. Especially to improve the judgment in accuracy (probability), genetic markers sandwiching a Fusarium head blight-resistance factor (locus relating to the Fusarium head blight resistance) are selected and detected for their polymorphisms. One example of such a pair of genetic markers is a combination of "k05042" and "k03289". "k05042" is distanced from the Fusarium head blight-resistance factor (locus relating to the Fusarium head blight resistance) by approximately 1.2cM on the short-arm side, while "k03289" is distanced from the Fusarium head blight-resistance factor (locus relating to the Fusarium head blight resistance) by approximately 4.0cM. Discussing this combination by way of example, the accuracy (probability) of the present judging method is described more concretely for the case where the polymorphism of either of the genetic markers is solely detected, and the case where the polymorphisms of both the genetic markers are detected.

"k05042" is distanced from the Fusarium head blight-resistance factor (locus relating to the Fusarium head blight resistance) by approximately 1.2cM on the short-arm side. So, the accuracy (probability) of the present judging method for the detection of the polymorphism of this genetic marker alone is (1-12 ÷ 1000) × 100 = approximately 98.8%. On the other hand, k03289 is distanced from the Fusarium head blight-resistance factor (locus relating to the Fusarium head blight resistance) by approximately 4.0cM. The accuracy (probability) of the present judging method for the detection of the polymorphism of this genetic marker alone is 96.0%, which is calculated in a similar manner. In the case where the polymorphisms of both the genetic markers are detected, the accuracy is calculated as: (1-(12 ÷ 1000) × (40 ÷ 1000)) × 100 = 99.952%. Thus, in this case, it is possible to judge with high accuracy whether the Fusarium head blight-resistance factor (locus relating to the Fusarium head blight resistance) on 4H is present or not.

Therefore, it is preferable to perform the judgment by detecting genetic markers sandwiching a Fusarium head blight-resistance factor (locus relating to the Fusarium head blight resistance). Other that the combination of k05042 and k03289, the following combinations are examples: MMtgaEatc 128 and FMgcgEatc530; FMacgEcgt288 and HVM67; FMataEagc408 and HVM11; Bmag125 and k04002; and HvLOX and k00835.

The present judging method can be used as an effective means for screening in breeding the Fusarium head blight-resistant plant. For example, the transformant plant (Gramineae plant (e.g., Hordeum or the like such as barley) to which the DNA fragment including the Fusarium head blight-resistance factor is introduced can be easily screened out from among a large number of transformant candidates in producing the Fusarium head blight-resistant plant ((Gramineae plant (e.g., Hordeum or the like such as barley) according to the present invention. Furthermore, the present judging method is also applicable as means for screening for a plant (e.g., Hordeum or the like such as barley) attained by mutation using a chemical or the other means, or by breeding such as crossing.

### <Kit for Judging a Fusarium Head Blight-Resistant Plant (Gramineae) >

A kit (hereinafter, just referred to as "judging kit", where appropriate) for judging whether a plant is a Fusarium head blight-resistant plant (Gramineae plant) or not can be developed by providing the kit with a reagent(s), an enzyme(s), and the like necessary for performing "the method for judging whether a plant is a Fusarium head blight-resistant plant (Gramineae plant) or not". As explained in the section discussing "(Method for judging) Fusarium head blight-resistant plant (Gramineae plant)", the use of a genetic marker according to the present invention makes it possible to test a Gramineae plant to judge whether a gene allelic to a Fusarium head blight-resistance factor (loci relating to the Fusarium head blight resistance) is of the resistant genotype or of the susceptible genotype. That is, it is possible to test a plant (Gramineae plant) to judge whether the plant is resistant or susceptible to the Fusarium head blight. Thus, with the judging kit, it is possible to judge more easily whether a plant is a Fusarium head blight-resistant plant (Gramineae plant) or not.

The judging kit preferably includes at least one of the primer sets (first to eighteenth primer sets) that can at least detect a genetic marker according to the present invention. It is more preferable that the judging kit includes all the primer sets (first to eighteenth primer sets). Apart from these, the judging kit may include a primer set necessary for detecting a marker linked to a well-known Fusarium head blight resistance.

Besides these contents, the present judging kit may contain an enzyme, reagent, and/or the like for PCR, and may contain a reagent, a buffer, centrifugal tube for the preparation of a genomic DNA that acts as a template, and may contain a genetic marker (such as MMtgaEatc128, FMgcgEatc530, FXLRRfor_XLRRrev119, FMacgEcgt288, HVM67, FMataEagc408, HVM11, Bmag125, k04002, k05042, k03289, HvLOX, and k00835) necessary for detecting a targeted DNA size band, or an appropriate DNA size marker.

### < Gene Detecting Apparatus (DNA Microarray etc.)>

A gene detecting apparatus such as DNA microarray includes a genetic marker(s) according to the present invention (such as MMtgaEatc128, FMgcgEatc530, FXLRRfor_XLRRrev119, FMacgEcgt288, HVM67, FMataEagc408, HVM11, Bmag125, k04002, k05042, k03289, HvLOX, and/or k00835) fixed on an appropriate substrate (glass, silicon wafer, nylon membrane, or the like). The present gene detecting apparatus such as DNA microarray is reacted with a probe prepared from a plant to be tested, and a signal emitted from the reaction, whereby it is possible to detect a plurality of genetic markers at the same time easily. Therefore, the present gene detecting apparatus such as DNA microarray can be used as means for performing detection of a polymorphism of genetic marker according to the present invention. Accordingly, the present gene detecting apparatus is applicable as means for detecting in the method for judging whether a Fusarium head blight-resistance factor or not, and in the method for judging whether a plant (Gramineae plant (e.g., Hordeum or the like such as barley)) is a Fusarium head blight-resistant plant or not. Furthermore, it is possible to include the present gene detecting apparatus such as DNA microarray in the kit for judging whether a Fusarium head blight-resistance factor is present or not, or in the kit for judging whether a plant (Gramineae plant (e.g., Hordeum or the like such as barley)) is a Fusarium head blight-resistant factor or not. The kits may includes a reagent, tool, apparatus, or the like for detecting the signal.

It is sufficient for the present gene detecting apparatus such as DNA microarray that at least one of the genetic markers (such as MMtgaEatc128, FMgcgEatc530, FXLRRfor_XLRRrev119, FMacgEcgt288, HVM67, FMataEagc408, HVM11, Bmag125, k04002, k05042, k03289, HvLOX, and k00835) according to the present invention is fixed on the substrate. Moreover, the present gene detecting apparatus may be such that one or both of the genetic markers of the resistance type and of the susceptible type is fixed on the substrate. For higher accuracy (probability) in the judgment whether a Fusarium head blight-resistance factor (locus relating to the Fusarium head blight resistance) is present or not, it is preferable that a combination of a plurality of genetic markers whose loci sandwich a Fusarium head blight-resistance factor (locus relating to the Fusarium head blight resistance) to detect be fixed on the substrate. Examples of such a combinations of genetic markers are: k05042 and k03289; MMtgaEatc 128 and FMgcgEatc530; FMacgEcgt288 and HVM67; FMataEagc408 and HVM11; Bmag125 and k04002; and HvLOX and k00835. Needless to say, it is most preferably to fix all the genetic markers (resistant type and susceptible type) on the substrate.

By using the gene detecting apparatus (such as DNA microarray) on which the plurality of genetic markers are fixed, it is possible to easily detect a plurality of genetic markers without repeating the test, and also it is possible to perform the judgment with high accuracy (probability) whether the Fusarium head blight-resistance factor (locus relating to the Fusarium head blight resistance) is present or not.

The present gene detecting apparatus may be arranged such that another or other genetic markers located in the vicinity of the genetic marker according to the present invention may be fixed to the present gene detecting apparatus in addition to the genetic marker(s) according to the present invention.

The present gene detecting apparatus (such as DNA microarray) is preferably arranged such that the genetic markers according to the present invention are fixed in the order in which they are aligned on the chromosome of barley, or that the genetic markers according to the present invention are fixed with sequence position information that corresponds to the order in which they are aligned on the chromosome of barley. With this arrangement, it is possible to perform the detection with higher accuracy when barley is to be tested. In analysis using a gene detecting apparatus such as a conventional DNA microarray or the like, it is necessary to double check in the event that no signal is obtained at a certain spot, in order to find out whether the lack of the signal indicates that the genetic marker that is the target for the detection is absent, or that the signal is not obtained due to an experimental error in the analysis. On the other hand, with the present gene detecting apparatus (such as DNA microarray), in which the fixed genetic markers are so aliened that the order of the genetic markers on the chromosome can be confirmed, it is easy to judge whether the lack of the signal is due to an experimental error or not.

To specifically explain this, suppose a signal is obtained at spots before and after a spot at which no signal is obtained, for example. In the present gene detecting apparatus (such as DNA microarray), each spot is so aligned that the order on the chromosome can be confirmed. In general, to recombine only one of genes next to each other in line on the chromosome, recombination should occur twice in the vicinity of the recombination of only the one of the genes next to each other. Such a phenomenon occurs with quite low probability. So, it is judged that the lack of the signal occurs due to an experimental error. As described above, the present gene detecting apparatus (such as DNA microarray) can improve the accuracy of the analysis because it is possible to judge whether the lack of a signal at a spot is due to an experimental error or not.

Needless to say, the base sequences of the primers and the like used in the description of the present invention above are not particularly limited, and may be such that one or a few of the bases are substituted, deleted, or added, provided that the primer or the like allows amplification for a genetic marker of the present invention.

The present invention is described in more details below via Examples, which are not to limit the present invention.

### [Example 4: QTL Analysis Regarding Fusarium Head Blight Resistance of Barley]

A QTL analysis was conducted for searching for a locus relating to the Fusarium head blight resistance of barley.

### <Material>

Used were RI lines derived from a cross of barley cultivar different in the resistance against Fusarium head blight, namely: an RI line (RHI population) derived from a cross of Russia 6 (two-row, resistant) and H.E.S.4 (six-row, susceptible); an RI line (RI2 population) derived from a cross of Harbin 2-row (resistant) and Turkey 6 (susceptible); and an RI line (DHHS population) derived from a cross of Haruna Nijo (resistant) and H602 (susceptible).

### <Evaluation method for Fusarium Head Blight Resistance>

A modified "Cut-Spike Test" was used for the evaluation. To explain briefly, the Fusarium head blight was evaluated as follows.

The material was cultivated according to a generally-used method. For each RI line, a spike was cut off, with a flag leaf, from a stem at the first internode in flowering period. The spikes were held stand in a stainless tray into which tap water was continuously run. To the spikes the inoculum was sufficiently spayed, which was prepares such that fifteen conidiums of the Fusarium bacteria were observed per field of vision of a microscope of 200 × magnification. The spikes were cultivated at 25°C and under 100% humidity for first two days after the inoculation, and at 18°C and under approximately 90% humidity for next 6 days. Lighting condition was 14 hours day length and 10,000 lux luminance. On eighth day from the inoculation, the spikes were observed visually to be scored by 0 (resistant) to 10 (susceptible) according to the standard shown in Table 1.

**Table 4**

| | | | | | | |
|---|---|---|---|---|---|---|
| Score | 0 | 2 | 4 | 6 | 8 | 10 |
| SR(%) | 0 | 0 to 5 | 6 to 20 | 21 to 40 | 41 to 60 | 61 to 100 |

| | | | | | | |
|---|---|---|---|---|---|---|
| Abbreviation: SR stands for susceptible ratio (%). | | | | | | |

The algorisms used in the QTL analysis were simple interval mapping (SIM) and composite interval mapping (CIM). MAPMARKER/QTL and QTL Cartographer were used as analysis software respectively. A threshold value of an LOD score was set to 2. If the LOD score exceeds 2, it was deduced that there was an QTL at a position of a largest LOD in a region between the two markers.

### <Results>

Table 5, Table 6, and Table 7 respectively show the result of the RHI population, RI2 population, and DHHS population.

**Table 5**

| "C" | Marker Interval (M1-A-QTL-B-M2) | D (cM)A+B | P ^{a)} (cM)A | P (cM)B | LOD^{b)} score | Var.^{c)} (%) | W^{d)} |
|---|---|---|---|---|---|---|---|
| 2H | MEgacMacc314-FEgtaMacg677 | 0.6 | 0.0 | 0.6 | 5.0 | 23.7 | -1.5 |
| 4H | MMtgaEatc128-FMgcgEatc530 | 8.1 | 0.1 | 8.0 | 2.7 | 12.4 | -1.1 |

| | | | | | | | |
|---|---|---|---|---|---|---|---|
| Abbreviation: "C" stands for chromosome. "D" stands for distance. "P" stands for position. "W" stands for weight. a): Distance of peak LOD score position from the left side marker b): Peak LOD score of significant marker interval c): Explained variance of peak LOD score d): Estimated additive effect | | | | | | | |

As clearly understood from Table 5, one QTL relating to the Fusarium head blight was detected on 2H chromosome, and one QTL relating to the Fusarium head blight was detected on 4H chromosome in the RHI population. The QTL detected on 2H chromosome was the one that had been detected by the previous study conducted by the inventors of the present invention. Therefore, the present study found, on 4H chromosome, one novel QTL relating to the Fusarium head blight resistance.

This QTL is located in a position sandwiched by the genetic markers MMtgaEatc128 and FMgcgEatc530. This QTL is distanced from MMtgaEatc 128 by 0.0cM from, while this QTL is distanced from FMgcgEatc530 by 8.0 cM on the short-arm side. The LOD score is 2.7. This QTL accounts for 12.4% of variance of the phenotype. Moreover, the presence of this QTL lowers the score of "Cut-Spike Test" by 1.1 (that is, lowers the resistance).

**Table 6**

| "C" | Marker Interval (M1-A-QTL-B-M2) | D (cM)A+B | P ^{a)} (cM)A | P (cM)B | LOD^{b)} score | Var.^{c)} (%) | W^{d)} |
|---|---|---|---|---|---|---|---|
| 2H | FXLRRfor_XLRRrev119 -FEgtaMacg677 | 3.5 | 0.2 | 3.3 | 2.6 | 10.1 | -1.3 |
| 4H | FMacgEcgt288-HVM67 | 47.1 | 0.3 | 46.8 | 2.2 | 25.5 | -0.9 |
| 6H | FMataEagc408-HVM11 | 13.5 | 4.1 | 9.4 | 2.7 | 28.6 | -1.0 |

| | | | | | | | |
|---|---|---|---|---|---|---|---|
| Abbreviation: "C" stands for chromosome. "D" stands for distance. "P" stands for position. "W" stands for weight. a): Distance of peak LOD score position from the left side marker b): Peak LOD score of significant marker interval c): Explained variance of peak LOD score d): Estimated additive effect | | | | | | | |

For RI2 population, the QTL analysis was repeated twice. In the first QTL analysis, one QTL relating to the Fusarium head blight resistance was detected on 2H chromosome. In the second QTL analysis, one QTL was detected on each 4H chromosome and 6H chromosome.

The QTL detected on 2H chromosome is located at a position sandwiched between the genetic markers FXLRRfor_XLRRrev119 and FegtaMacg677. This QTL is distanced from the FXLRRfor_XLRRrev119 by 0.2cM on the long-arm side, and from FegtaMacg677 by 3.3cM on the short-arm side. The LOD score is 2.6. This QTL accounts for 10.1% of the variance of the phenotype. Moreover, the presence of this QTL lowers the score of" Cut-Spike Test" by 1.3 (that is, increases the resistance).

The QTL detected on 4H chromosome is located at a position sandwiched between the genetic markers FMacgEcgt288 and HVM67. This QTL is distanced from the FMacgEcgt288 by 0.3cM on the long-arm side, and from HVM67 by 46.8cM on the short-arm side. The LOD score is 2.2. This QTL accounts for 25.5% of the variance of the phenotype. Moreover, the presence of this QTL lowers the score of "Cut-Spike Test" by 0.9 (that is, increases the resistance).

The QTL detected on 6H chromosome is located at a position sandwiched between the genetic markers FMataEagc408 and HVM 11. This QTL is distanced from the FMataEagc408 by 4.1cM on the long-arm side, and from HVM11 by 9.4cM on the short-arm side. The LOD score is 2.7. This QTL accounts for 28.6% of the variance of the phenotype. Moreover, the presence of this QTL lowers the score of "Cut-Spike Test" by 1.0 (that is, increase the resistance).

**Table 7**

| "C" | Marker Interval (M1-A-QTL-B-M2) | D (cM)A+B | P ^{a)} (cM)A | P (cM)B | LCD^{b)} score | Var.^{c)} (%) | W^{d)} |
|---|---|---|---|---|---|---|---|
| 2H | Bmag125-k04002 | 10.0 | 0.0 | 10.0 | 2.0 | 12.2 | -2.2 |
| 4H | k05042-k03289 | 5.2 | 1.2 | 4.0 | 3.2 | 27.7 | 2.8 |
| 5H | HvLOX-k00835 | 2.4 | 0.5 | 1.9 | 3.1 | 19.7 | -2.4 |

| | | | | | | | |
|---|---|---|---|---|---|---|---|
| Abbreviation: "C" stands for chromosome. "D" stands for distance. "P" stands for position. "W" stands for weight. a): Distance of peak LOD score position from the left side marker b): Peak LOD score of significant marker interval c): Explained variance of peak LOD score d): Estimated additive effect | | | | | | | |

As clearly understood from Table 7, one QTL relating to the Fusarium head blight resistance is detected on each 2H chromosome, 4H chromosome, and 5H chromosome in the DHHS population.

The QTL detected on 2H chromosome is located at a position sandwiched between the genetic markers Bmag125 and k04002. This QTL is distanced from the Bmag125 by 0.0cM, and from k04002 by 10.0cM on the short-arm side. The LOD score is 2.0. This QTL accounts for 12.2% of the variance of the phenotype. Moreover, the presence of this QTL lowers the score of " Cut-Spike Test" by 2.2 (that is, increases the resistance).

The QTL detected on 4H chromosome is located at a position sandwiched between the genetic markers k05042 and k03289. This QTL is distanced from the k05042 by 1.2cM on the long-arm side, and from k03289 by 10.0cM on the short-arm side. The LOD score is 3.2. This QTL accounts for 27.7% of the variance of the phenotype. Moreover, the presence of this QTL increases the score of "Cut-Spike Test" by 2.8 (that is, lowers the resistance).

The QTL detected on 5H chromosome is located at a position sandwiched between the genetic markers HvLox and k00835. This QTL is distanced from the HvLox by 0.5cM on the long-arm side, and from k00835 by 1.9cM on the short-arm side. The LOD score is 3.1. This QTL accounts for 19.7% of the variance of the phenotype. Moreover, the presence of this QTL lowers the score of " Cut-Spike Test" by 2.4 (that is, increases the resistance).

Note the meanings of the terms used in Tables 5 to 7 are as follows: what is meant by the term "C" (Chromosome) is "a chromosome on which the genetic marker is located"; what is meant by the term "Marker interval (M1-A-QTL-B-M2)" is "two genetic markers (M1 and M2) being located in the vicinity of QTL and sandwiching the QTL therebetween"; what is meant by "D (cM)A+B" (Distance (cM)A+B) is "a distance between the two genetic markers sandwiching the QTL therebetween"; what is meant by "P^{a)} (cM)A" (Position^{a)} (cM)A) is "a distance between the genetic marker M1 and the QTL"; what is meant by "P^{b)} (cM)B" (Position^{b)} (cM)B) is "a distance between the genetic marker M2 and the QTL"; what is meant by "LOD^{b)} Score" is "a peak value of LOD score"; what is meant by "Var. (%)^{c)}" is "a value showing how much percentage of the variance of the phenotype can be explained by the presence of this QTL"; and what is meant by "Weight ^{d)}" is "a value showing how much the presence of this QTL increases the score of "Cut-Spike Test".

### [Example 5: Detection of Genetic marker "MMtgaEatc 128"]

### (Method)

50ng of a genomic DNA of a barley to be tested was subjected to double digestion with EcoRI (Takara bio Inc.) and MseI (NEW ENGLAND Biolabs Inc.) of 1.5U each in a reaction system of 25µl in total for 12 hours at 37°C. The DNA treated with the restriction enzymes were then ligated with 5µM of EcoRI adaptors (whose base sequences are shown in S.E.Q. ID. NOs. 14 and 15), and 50µM of MseI adaptors (whose base sequences are shown in S.E.Q. ID. NOs. 16 and 17), and 25U of T4 ligase (Takara bio Inc.) at 37°C for 3 hours.

The DNA fragment after ligation was pre-amplified with a universal primer (whose base sequence is shown in S.E.Q. ID. NO. 18) of EcoRI, and a universal primer (whose base sequence is shown in S.E.Q. ID. NO. 19) of MseI. Using 0.07mg/ml of a reaction solution of the pre-amplification, amplification reaction was performed with a selective primer of EcoRI whose base sequence is shown in S.E.Q. ID. NO. 33, and a selective primer of MseI whose base sequence is shown in S.E.Q. ID. NO. 32. The amplification reaction was carried out with Takara Ex Taq (Takara bio Inc.)

The pre-amplification reaction was carried out with 2-minute heating at 94°C, and then 20 reaction cycles of 30 seconds at 94°C, 1 minute at 56°C, and 1 minute at 72°C thereafter.

The amplification reaction was carried out with heating of 30 seconds at 94°C, 30 seconds at 68°C, and 1 minute at 72°C, and then heating of 30 seconds at 94°C, 30 seconds at 68°C, 30 seconds at 72°C, 30 seconds at 94°C, 30 seconds at 67.3°C, 1 minute at 72°C, 30 seconds at 94°C, 30 seconds at 66.6°C, 1 minute at 72°C, 30 seconds at 94°C, 30 seconds at 65.9°C, 1 minute at 72°C, 30 seconds at 94°C, 30 seconds at 65.2°C, 1 minute at 72°C, 30 seconds at 94°C, 30 seconds at 64.5°C, 1 minute at 72°C, 30 seconds at 94°C, 30 seconds at 63.8°C, 1 minute at 72°C, 30 seconds at 94°C, 30 seconds at 63.1°C, 1 minute at 72°C, 30 seconds at 94°C, 30 seconds at 62.4°C, 1 minute at 72°C, 30 seconds at 94°C, 30 seconds at 61.7°C, 1 minute at 72°C, 30 seconds at 94°C, 30 seconds at 61.0°C, 1 minute at 72°C, 30 seconds at 94°C, 30 seconds at 60.3°C, 1 minute at 72°C, 30 seconds at 94°C, 30 seconds at 59.6°C, 1 minute at 72°C, 30 seconds at 94°C, 30 seconds at 58.9°C, 1 minute at 72°C, 30 seconds at 94°C, 30 seconds at 58.2°C, 1 minute at 72°C, 30 seconds at 94°C, 30 seconds at 57.5°C, 1 minute at 72°C, 30 seconds at 94°C, 30 seconds at 56.8°C, 1 minute at 72°C, and then 23 cycles of 30 seconds at 94°C, 30 seconds at 56°C, and 1 minutes at 72°C.

### (Results)

FIG. 8 shows results of electrophoresis of an amplified fragment obtained by the amplification reaction as above. In FIG. 8, the leftmost and rightmost lanes indicate size markers. The second lane from the leftmost lane shows a result of AFLP. detection for Russia 6, which is a Fusarium head blight-resistant cultivar. The third lane from the leftmost lane shows a result of AFLP detection for H.E.S. 4, which is a Fusarium head blight-susceptible cultivar. The other lanes show results of AFLP detection for the RHI population of the recombinant inbred lines (RI lines) derived from the cross of Russia 6 and H.E.S. 4.

According to the results of FIG. 8, there are resistant type (Russia 6 type) and susceptible type (H.E.S. 4 type) to Fusarium head blight. The amplified fragment of the resistant type (Russia 6 type) has a fragment length of approximately 128bp (indicated by the arrow), whereas the amplified fragment of the susceptible type (H.E.S. type) has a fragment length of 0bp. In other words, an amplified fragment of approximately 128bp is obtained from the resistant type (Russia 6 type) against the Fusarium head blight, but no amplified fragment of approximately 128bp is obtained from the susceptible type (H.E.S. 4 type) to the Fusarium head blight. Thus, by the AFLP detection operation of the plant to test, and performing the judgment using the presence of the amplification fragment of approximately 128bp, it is possible to test a plant to judge whether a gene allelic to the Fusarium head blight-resistance factor (locus relating to the Fusarium head blight resistance) located on 4H chromosome is of resistant genotype or of susceptible genotype.

### [Example 6: Detection of Genetic marker "FMgcgEatc530"]

### (Method)

The same method as in Example 5 was performed, except that a selective primer EcoRI having the base sequence of S.E.Q. ID. NO. 35, and a selective primer MseI having the base sequence of S.E.Q. ID. NO. 34 were used in the amplification reaction.

### (Result)

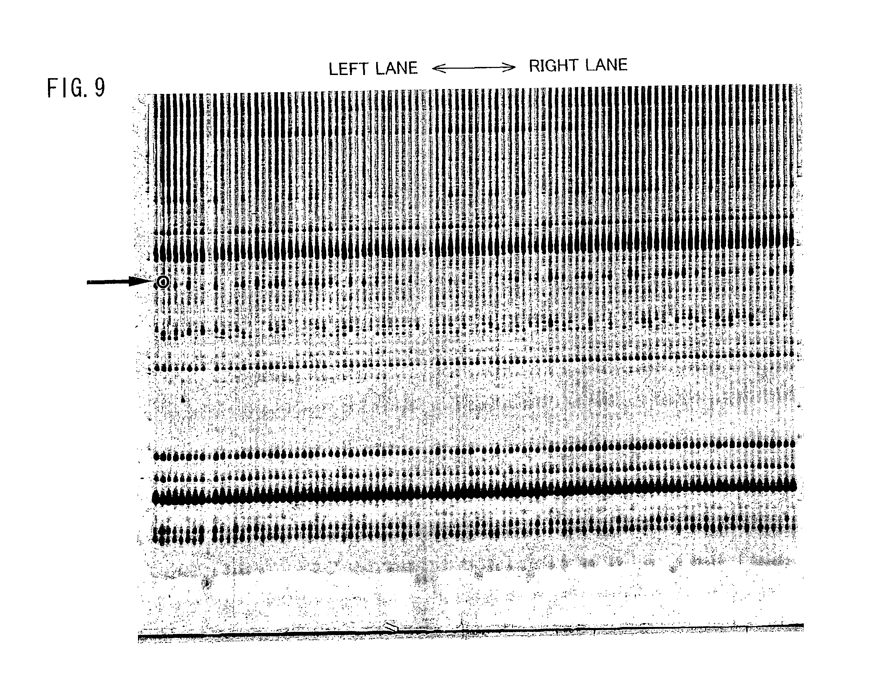

FIG. 9 shows results of electrophoresis of an amplified fragment obtained by the amplification reaction as above. In FIG. 9, the leftmost and rightmost lanes indicate size markers. The second lane from the leftmost lane shows a result of AFLP detection for Russia 6, which is a Fusarium head blight-resistant cultivar. The third lane from the leftmost lane shows a result of AFLP detection for H.E.S. 4, which is a Fusarium head blight-susceptible cultivar. The other lanes show results of AFLP detection for the RHI population of the recombinant inbred lines (RI lines) derived from the cross of Russia 6 and H.E.S. 4.

According to the results of FIG. 9, there are resistant type (Russia 6 type) and susceptible type (H.E.S. 4 type) to Fusarium head blight. The amplified fragment of the resistant type (Russia 6 type) has a fragment length of 0bp (indicated by the arrow), whereas the amplified fragment of the susceptible type (H.E.S. 4 type) has a fragment length of approximately 530bp. In other words, no amplified fragment of approximately 530bp is obtained from the resistant type (Russia 6 type) against the Fusarium head blight, but an amplified fragment of approximately 530bp is obtained from the susceptible type (H.E.S. 4 type) to the Fusarium head blight. Thus, by the AFLP detection operation of the plant to test, and performing the judgment using the presence of the amplification fragment of approximately 530bp, it is possible to test a plant to judge whether a gene allelic to the Fusarium head blight-resistance factor (locus relating to the Fusarium head blight resistance) located on 4H chromosome is of resistant genotype or of susceptible genotype.

### [Example 7: Detection of Genetic marker "FXLRRfor_XLRRrev119"]

### (Method)

Using, as the template, genomic DNA of a barley to be tested, amplification reaction was carried out with primers having the base sequences of S.E.Q. ID. NOs. 22 and 23, and with 5 minute heating at 94°C, and then 45 reaction cycles of 1 minute at 94°C, 1 minute at 45°C, two minutes at 72°C, followed by 7 minute heating at 72°C thereafter.

### (Result)

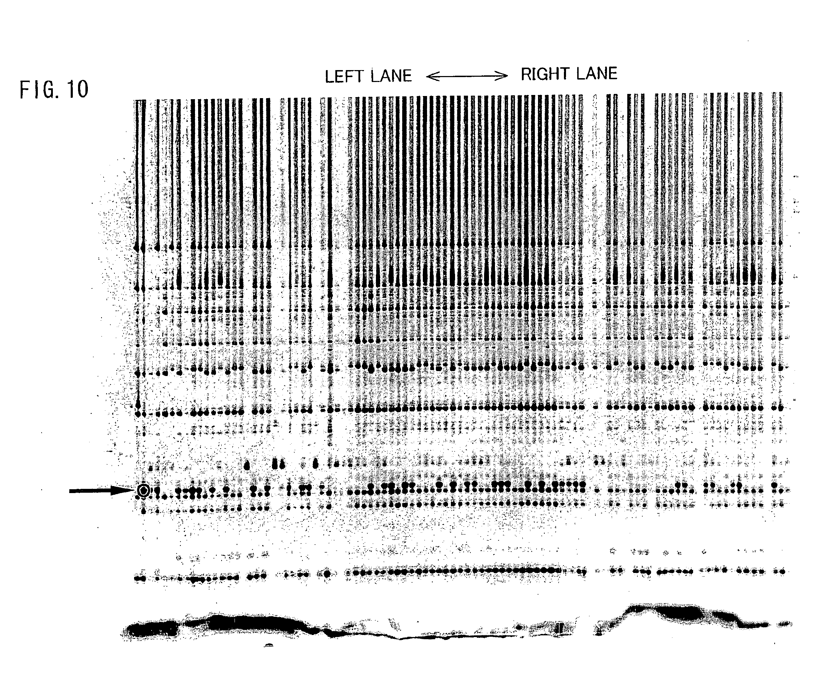

FIG. 10 shows results of electrophoresis of an amplified fragment obtained by the amplification reaction as above. In FIG. 10, the leftmost and rightmost lanes indicate size markers. The second lane from the leftmost lane shows a result of amplified fragments from Harbin 2-row, which is a Fusarium head blight-resistant cultivar. The third lane from the leftmost lane shows a result of amplified fragments from Turkey 6, which is a Fusarium head blight-susceptible cultivar. The other lanes show results of amplified fragments for the RI2 population of the recombinant inbred lines (RI lines) derived from the cross of Harbin 2-row and Turkey 6.

According to the results of FIG. 10, there are resistant type (Harbin 2-row type) and susceptible type (Turkey 6 type) to Fusarium head blight. The amplified fragment of the resistant type (Harbin 2-row type) has a fragment length of 0bp, whereas the amplified fragment of the susceptible type (Turkey 6 type) has a fragment length of approximately 119bp (indicated by the arrow). In other words, no amplified fragment of approximately 119bp is obtained from the resistant type (Harbin 2-row type) against the Fusarium head blight, but an amplified fragment of approximately 119bp is obtained from the susceptible type (Turkey 6 type) to the Fusarium head blight. Thus, by the AFLP detection operation of the plant to test, and performing the judgment using the presence of the amplification fragment of approximately 119bp, it is possible to test a plant to judge whether a gene allelic to the Fusarium head blight-resistance factor (locus relating to the Fusarium head blight resistance) located on 2H chromosome is of resistant genotype or of susceptible genotype.

### [Example 8: Detection of Genetic Marker "FmacgEcgt288"]

### (Method)

The same method as in Example 5 was performed, except that a selective primer EcoRI having the base sequence of S.E.Q. ID. NO. 37, and a selective primer MseI having the base sequence of S.E.Q. ID. NO. 36 were used in the amplification reaction.

### (Result)

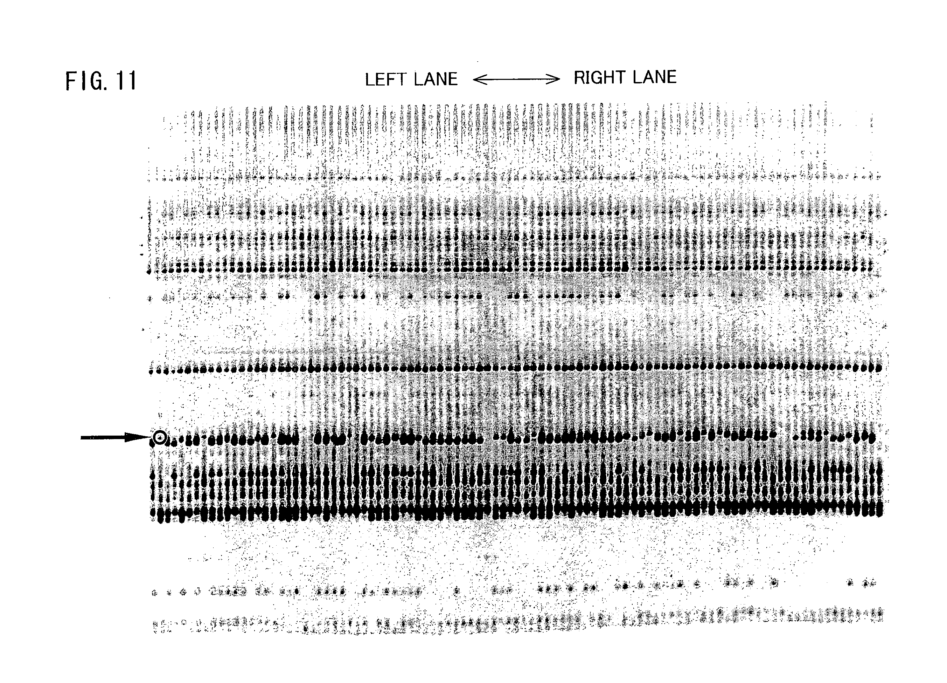

FIG. 11 shows results of electrophoresis of an amplified fragment obtained by the amplification reaction as above. In FIG. 11, the leftmost and rightmost lanes indicate size markers. The second lane from the leftmost lane shows a result of an amplified fragment for Harbin 2-row, which is a Fusarium head blight-resistant cultivar. The third lane from the leftmost lane shows a result of an amplified fragment for Turkey 6, which is a Fusarium head blight-susceptible cultivar. The other lanes show results of amplified fragments for the RI2 population of the recombinant inbred lines (RI lines) derived from the cross of Harbin 2-row and Turkey 6.

According to the results of FIG. 11, there are resistant type (Harbin 2-row type) and susceptible type (Turkey 6 type) to Fusarium head blight. The amplified fragment of the resistant type (Harbin 2-row type) has a fragment length of 0bp, whereas the amplified fragment of the susceptible type (Turkey 6 type) has a fragment length of approximately 288bp (indicated by the arrow). In other words, no amplified fragment of approximately 288bp is obtained from the resistant type (Harbin 2-row type) against the Fusarium head blight, but an amplified fragment of approximately 288bp is obtained from the susceptible type (Turkey 6 type) to the Fusarium head blight. Thus, by the AFLP detection operation of the plant to test, and performing the judgment using the presence of the amplification fragment of approximately 288bp, it is possible to test a plant to judge whether a gene allelic to the Fusarium head blight-resistance factor (locus relating to the Fusarium head blight resistance) located on 4H chromosome is of resistant genotype or of susceptible genotype.

### [Example 9: Detection of Genetic marker "HVM67"]

### (Method)

Using, as the template, genomic DNA of a barley to be tested, amplification reaction was carried out with primers having the base sequences of S.E.Q. ID. NOs. 38 and 39, and with 3-minute heating at 94°C, and then 10 cycles of 1 minute at 94°C, 1 minute at 64°C (this temperature was reduced by 1°C every cycle), 1 minute at 72°C, followed by 30 cycles of 1minute at 94°C, 1 minute at 55°C, and 1 minute at 72°C, and then 5-minute heating at 72°C thereafter.

### (Result)

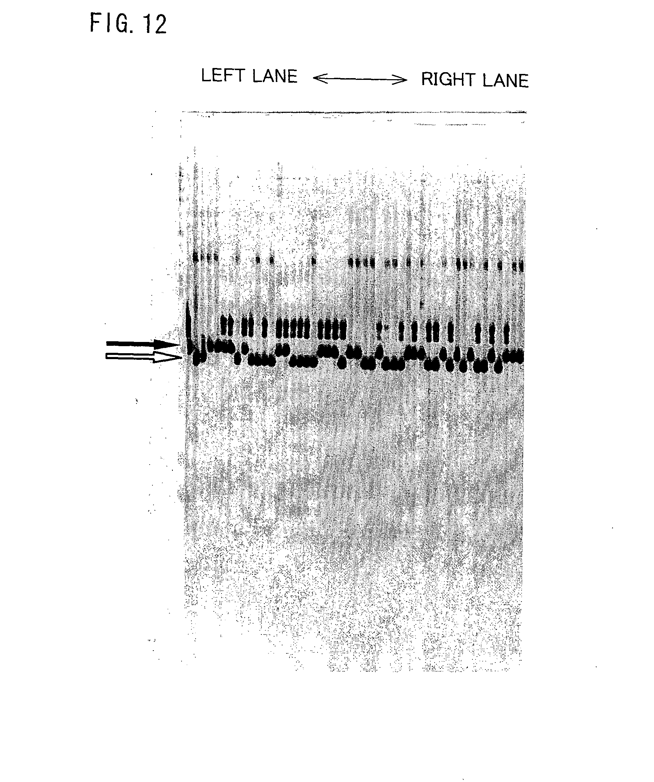

FIG. 12 shows results of electrophoresis of an amplified fragment obtained by the amplification reaction as above. In FIG. 12, the leftmost and rightmost lanes indicate size markers. The second lane from the leftmost lane shows a result of amplified fragments from Harbin 2-row, which is a Fusarium head blight-resistant cultivar. The third lane from the leftmost lane shows a result of amplified fragments from Turkey 6, which is a Fusarium head blight-susceptible cultivar. The other lanes show results of amplified fragments for the RI2 population of the recombinant inbred lines (RI lines) derived from the cross of Harbin 2-row and Turkey 6.

According to the results of FIG. 12, there are resistant type (Harbin 2-row type) and susceptible type (Turkey 6 type) to Fusarium head blight. The amplified fragment of the resistant type (Harbin 2-row type) has a fragment length of 160bp (indicated by the arrow), whereas the amplified fragment of the susceptible type (Turkey 6 type) has a fragment length of approximately 140bp (indicated by "open" arrow). Thus, by the above-described detection operation of the plant to test, and performing the judgment using the presence of the amplification fragment of approximately 160bp or approximately 140bp, it is possible to test a plant to judge whether a gene allelic to the Fusarium head blight-resistance factor (locus relating to the Fusarium head blight resistance) located on 4H chromosome is of resistant genotype or of susceptible genotype.

### [Example 10: Detection of Genetic Marker "FMataEagc408"]

### (Method)

The same method as in Example 5 was performed, except that a selective primer EcoRI having the base sequence of S.E.Q. ID. NO. 45, and a selective primer MseI having the base sequence of S.E.Q. ID. NO. 44 were used in the amplification reaction.

### (Result)

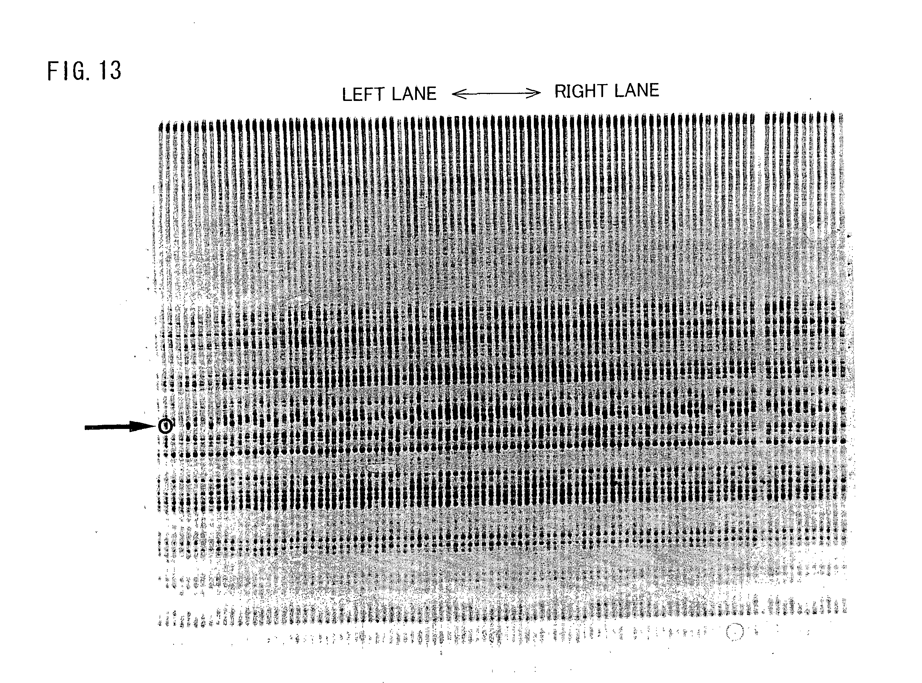

FIG. 13 shows results of electrophoresis of an amplified fragment obtained by the amplification reaction as above. In FIG. 13, the leftmost and rightmost lanes indicate size markers. The second lane from the leftmost lane shows a result of an amplified fragment for Harbin 2-row, which is a Fusarium head blight-resistant cultivar. The third lane from the leftmost lane shows a result of an amplified fragment for Turkey 6, which is a Fusarium head blight-susceptible cultivar. The other lanes show results of amplified fragments for the RI2 population of the recombinant inbred lines (RI lines) derived from the cross of Harbin 2-row and Turkey 6.

According to the results of FIG. 13, there are resistant type (Harbin 2-row type) and susceptible type (Turkey 6 type) to Fusarium head blight. The amplified fragment of the resistant type (Harbin 2-row type) has a fragment length of 0bp, whereas the amplified fragment of the susceptible type (Turkey 6 type) has a fragment length of approximately 408bp (indicated by the arrow). In other words, no amplified fragment of approximately 408bp is obtained from the resistant type (Harbin 2-row type) against the Fusarium head blight, but an amplified fragment of approximately 408bp is obtained from the susceptible type (Turkey 6 type) to the Fusarium head blight. Thus, by the AFLP detection operation of the plant to test, and performing the judgment using the presence of the amplification fragment of approximately 408bp, it is possible to test a plant to judge whether a gene allelic to the Fusarium head blight-resistance factor (locus relating to the Fusarium head blight resistance) located on 6H chromosome is of resistant genotype or of susceptible genotype.

### [Example 11: Detection of Genetic marker "HVM11"]

Using, as the template, genomic DNA of a barley to be tested, amplification reaction was carried out with primers having the base sequences of S.E.Q. ID. NOs. 46 and 47, and with 3-minute heating at 94°C, and then 10 cycles of 1 minute at 94°C, 1 minute at 64°C (this temperature was reduced by 1°C every cycle), 1 minute at 72°C, followed by 30 cycles of 1 minute at 94°C, 1 minute at 55°C, and 1 minute at 72°C, and then 5-minute heating at 72°C thereafter.

### (Result)

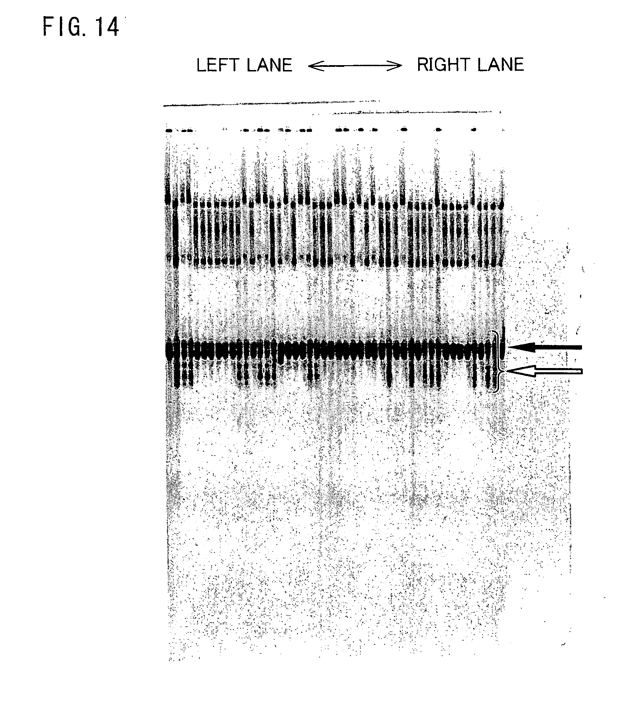

FIG. 14 shows results of electrophoresis of an amplified fragment obtained by the amplification reaction as above. In FIG. 14, the leftmost and rightmost lanes indicate size markers. The second lane from the leftmost lane shows a result of amplified fragments from Harbin 2-row, which is a Fusarium head blight-resistant cultivar. The third lane from the leftmost lane shows a result of amplified fragments from Turkey 6, which is a Fusarium head blight-susceptible cultivar. The other lanes show results of amplified fragments for the RI2 population of the recombinant inbred lines (RI lines) derived from the cross of Harbin 2-row and Turkey 6.

According to the results of FIG. 14, there are resistant type (Harbin 2-row type) and susceptible type (Turkey 6 type) to Fusarium head blight. The amplified fragment of the resistant type (Harbin 2-row type) has a fragment length of 144bp (indicated by the arrow), whereas the amplified fragment of the susceptible type (Turkey 6 type) has a fragment length in a range of approximately 100bp to 160bp (indicated by "open" arrow). Thus, by the above-described detection operation of the plant to test, and performing the judgment using the presence of the amplification fragment of approximately 144bp or in a range of approximately 144bp to 160bp, it is possible to test a plant to judge whether a gene allelic to the Fusarium head blight-resistance factor (locus relating to the Fusarium head blight resistance) located on 6H chromosome is of resistant genotype or of susceptible genotype.

### [Example 12: Detection of Genetic marker "Bmag125"]

### (Method)

Using, as the template, genomic DNA of a barley to be tested, amplification reaction was carried out with primers having the base sequences of S.E.Q. ID. NOs. 24 and 25, and with heating of 3 minutes at 94°C, 1 minute at 55°C and 1 minute at 72°C, and then 30 cycles of 1 minute at 94°C, 1 minute at 55°C, and 1 minute at 72°C, followed by 5-minute heating at 72°C thereafter.

### (Result)

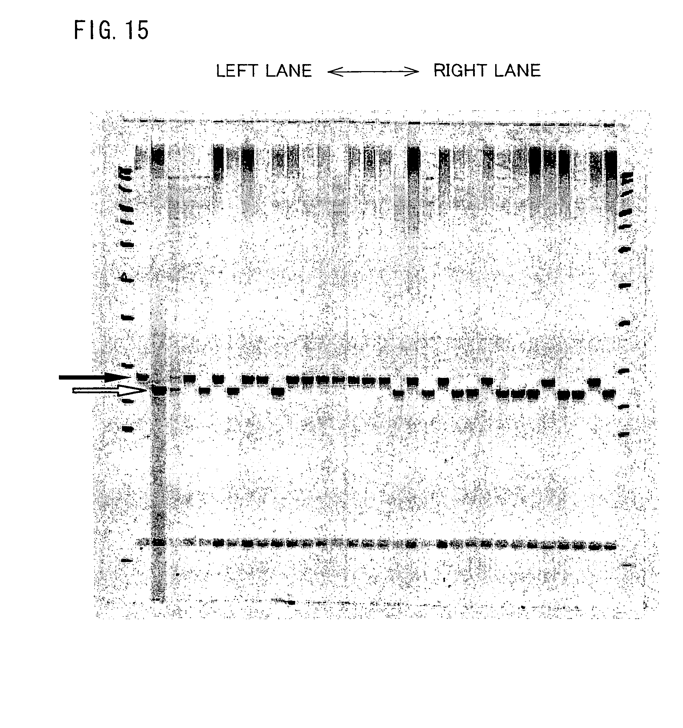

FIG. 15 shows results of electrophoresis of an amplified fragment obtained by the amplification reaction as above. In FIG. 15, the leftmost and rightmost lanes indicate size markers. The second lane from the leftmost lane shows a result of amplified fragments from Haruna Nijo, which is a Fusarium head blight-resistant cultivar. The third lane from the leftmost lane shows a result of amplified fragments from H602, which is a Fusarium head blight-susceptible cultivar. The fourth lane from the leftmost lane shows a result of amplified fragments from F1 from them. The other lanes show results of amplified fragments for the double haploid lines (DH lines) DHHS derived from the cross of Haruna Nijo and H602.

According to the results of FIG. 15, there are resistant type (Haruna Nijo type) and susceptible type (H602 type) to Fusarium head blight. The amplified fragment of the resistant type (Haruna Nijo type) has a fragment length of 142bp (indicated by the arrow), whereas the amplified fragment of the susceptible type (H602 type) has a fragment length of approximately 134bp (indicated by "open" arrow). Thus, by the above-described detection operation of the plant to test, and performing the judgment using the presence of the amplification fragment of approximately 144bp or approximately 134bp, it is possible to test a plant to judge whether a gene allelic to the Fusarium head blight-resistance factor (locus relating to the Fusarium head blight resistance) located on 2H chromosome is of resistant genotype or of susceptible genotype.

### [Example 13: Detection of Genetic marker "k04002"]

### (Method)

Using, as the template, genomic DNA of a barley to be tested, amplification reaction was carried out with primers having the base sequences of S.E.Q. ID. NOs. 26 and 27, and with 2-minute heating at 94°C, and then 5 cycles of 30 seconds at 94°C, 30 seconds at 65°C (this temperature was reduced by 1°C every cycle), 2 minutes at 72°C, followed by 35 cycles of 30 seconds at 94°C, 30 seconds at 60°C, and 2 minutes at 72°C, and then 7-minute heating at 72°C thereafter.

### (Result)

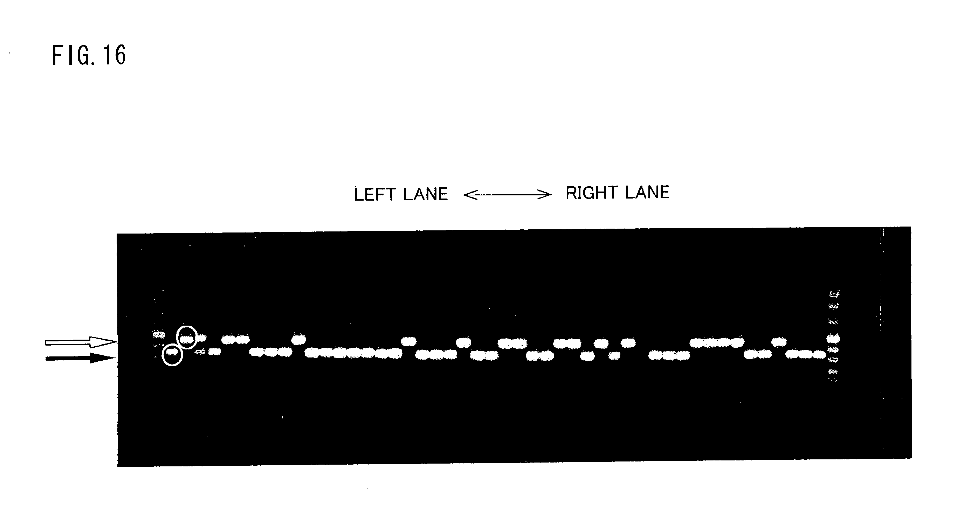

FIG. 16 shows results of electrophoresis of an amplified fragment obtained by the amplification reaction as above. In FIG. 16, the leftmost and rightmost lanes indicate size markers. The second lane from the leftmost lane shows a result of amplified fragments from Haruna Nijo, which is a Fusarium head blight-resistant cultivar. The third lane from the leftmost lane shows a result of amplified fragments from H602, which is a Fusarium head blight-susceptible cultivar. The fourth lane from the leftmost lane shows a result of amplified fragments from F1 from them. The other lanes show results of amplified fragments for the double haploid lines (DH lines) DHHS derived from the cross of Haruna Nijo and H602.

According to the results of FIG. 16, there are resistant type (Haruna Nijo type) and susceptible type (H602 type) to Fusarium head blight. The amplified fragment of the resistant type (Haruna Nijo type) has a fragment length of 350bp (indicated by the arrow), whereas the amplified fragment of the susceptible type (H602 type) has a fragment length of approximately 440bp (indicated by "open" arrow). Thus, by the above-described detection operation of the plant to test, and performing the judgment using the presence of the amplification fragment of approximately 350bp or approximately 440bp, it is possible to test a plant to judge whether a gene allelic to the Fusarium head blight-resistance factor (locus relating to the Fusarium head blight resistance) located on 2H chromosome is of resistant genotype or of susceptible genotype.

### [Example 14: Detection of Genetic marker "k05042"]

### (Method)

Using, as the template, genomic DNA of a barley to be tested, amplification reaction was carried out with primers having the base sequences of S.E.Q. ID. NOs. 40 and 41, and with 2-minute heating at 94°C, and then 5 cycles of 30 seconds at 94°C, 30 seconds at 65°C (this temperature was reduced by 1°C every cycle), 2 minutes at 72°C, followed by 35 cycles of 30 seconds at 94°C, 30 seconds at 60°C, and 2 minutes at 72°C, and then 7-minute heating at 72°C thereafter.

The amplification product was digested with 1.6U of a restriction enzyme HapII (Takara bio Inc.) for 15 hours at 37°C.

### (Result)

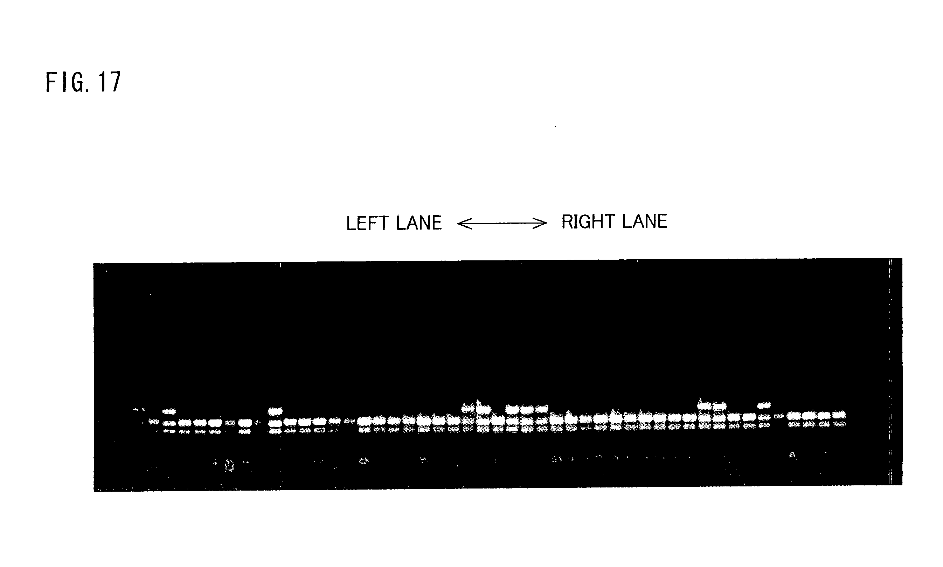

FIG. 17 shows results of electrophoresis of an amplified fragment obtained by the amplification reaction and restriction enzyme digestion as above. In FIG. 17, the leftmost lane shows a result of amplified fragments from Haruna Nijo, which is a Fusarium head blight-resistant cultivar. The second lane from the leftmost lane shows a result of amplified fragments from H602, which is a Fusarium head blight-susceptible cultivar. The third lane from the leftmost lane shows a result of amplified fragments from F1 from them. The other lanes show results of amplified fragments for the double haploid lines (DH lines) DHHS derived from the cross of Haruna Nijo and H602.

This genetic marker is, as described above, a CAPS marker, which causes different digestion patterns when the digestion with the restriction enzyme (HapII) is performed. According to the results of FIG. 17, in the digested fragment obtained by the above-mentioned operation there are Haruna Nijo type and resistant H602 type against the Fusarium head blight. The digested fragment of Haruna Nijo type has fragment lengths of 150bp, 350bp, and 500bp, approximately. The digested fragment of H602 type has fragment lengths of 300bp, 340bp, and 360bp, approximately. The amplified fragments H602 type of approximately 340bp and 360bp would apparently overlap each other sometime in electrophoresis with low resolution. Thus, by the above-described detection operation of the plant to test, and performing the judgment using the pattern of the digested fragment, it is possible to test a plant to judge whether a gene allelic to the Fusarium head blight-resistance factor (locus relating to the Fusarium head blight resistance) located on 4H chromosome is of resistant genotype or of susceptible genotype.

Note that, as described above, the Fusarium head blight-resistance factor (locus relating to the Fusarium head blight resistance) to which the present genetic marker is linked is the Fusarium head blight-resistance factor (locus relating to the Fusarium head blight resistance) having H602, which is a Fusarium head blight susceptible cultivar. Thus, in the detection of the present genetic marker, the Haruna Nijo type is regarded as the susceptible type, and the H602 type is regarded as the resistant type.

### [Example 15: Detection of Genetic marker "k03289"]

### (Method)

Using, as the template, genomic DNA of a barley to be tested, amplification reaction was carried out with primers having the base sequences of S.E.Q. ID. NOs. 42 and 43, and with 2-minute heating at 94°C, and then 5 cycles of 30 seconds at 94°C, 30 seconds at 65°C (this temperature was reduced by 1°C every cycle), 2 minutes at 72°C, followed by 35 cycles of 30 seconds at 94°C, 30 seconds at 60°C, and 2 minutes at 72°C, and then 7-minute heating at 72°C thereafter.

The amplification product was digested with 1.6U of a restriction enzyme SacII (Takara bio Inc.) for 15 hours at 37°C.

### (Result)

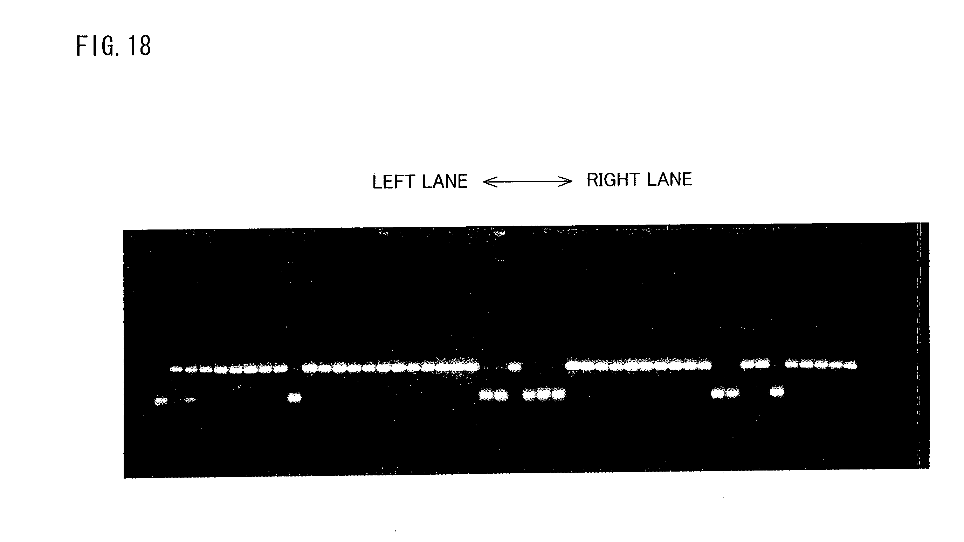

FIG. 18 shows results of electrophoresis of an amplified fragment obtained by the amplification reaction and restriction enzyme digestion as above. In FIG. 18, the leftmost lane shows a result of amplified fragments from Haruna Nijo, which is a Fusarium head blight-resistant cultivar. The second lane from the leftmost lane shows a result of amplified fragments from H602, which is a Fusarium head blight-susceptible cultivar. The third lane from the leftmost lane shows a result of amplified fragments from F1 from them. The other lanes show results of amplified fragments for the double haploid lines (DH lines) DHHS derived from the cross of Haruna Nijo and H602.

This genetic marker is, as described above, a CAPS marker, which causes different digestion patterns when the digestion with the restriction enzyme (SacII) is performed. According to the results of FIG. 18, in the digested fragment obtained by the above-mentioned operation there are Haruna Nijo type and resistant H602 type against the Fusarium head blight. The digested fragment of Haruna Nijo type has fragment lengths of 250bp, and 250bp, approximately. The digested fragment of H602 type has fragment length of approximately 500bp. Thus, by the above-described detection operation of the plant to test, and performing the judgment using the pattern of the digested fragment, it is possible to test a plant to judge whether a gene allelic to the Fusarium head blight-resistance factor (locus relating to the Fusarium head blight resistance) located on 4H chromosome is of resistant genotype or of susceptible genotype.

Note that, as described above, the Fusarium head blight-resistance factor (locus relating to the Fusarium head blight resistance) to which the present genetic marker is linked is the Fusarium head blight-resistance factor (locus relating to the Fusarium head blight resistance) that H602 has, which is a Fusarium head blight susceptible cultivar. Thus, in the detection of the present genetic marker, the Haruna Nijo type is regarded as the susceptible type, and the H602 type is regarded as the resistant type.

### [Example 16: Detection of Genetic marker "HvLOX"]

### (Method)

Using, as the template, genomic DNA of a barley to be tested, amplification reaction was carried out with primers having the base sequences of S.E.Q. ID. NOs. 28 and 29, and with heating of 3 minutes at 94°C, 1 minute at 58°C, and 1 minute at 72°C, and then 30 cycles of 30 seconds at 94°C, 30 seconds at 58°C, and 30 seconds at 72°C, followed by 5-minute heating at 72°C thereafter.

### (Result)

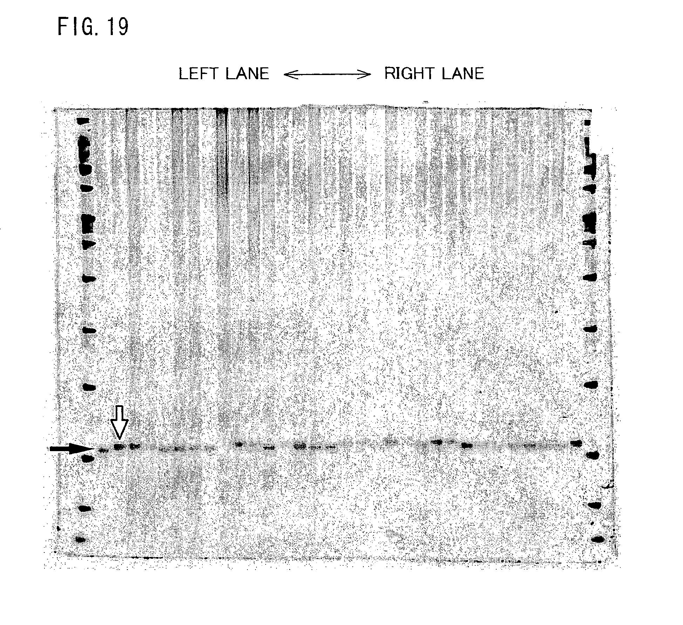

FIG. 19 shows results of electrophoresis of an amplified fragment obtained by the amplification reaction as above. In FIG. 19, the leftmost and rightmost lanes indicate size markers. The second lane from the leftmost lane shows a result of amplified fragments from Haruna Nijo, which is a Fusarium head blight-resistant cultivar. The third lane from the leftmost lane shows a result of amplified fragments from H602, which is a Fusarium head blight-susceptible cultivar. The other lanes show results of amplified fragments for the double haploid lines (DH lines) DHHS derived from the cross of Haruna Nijo and H602.

According to the results of FIG. 19, there are resistant type (Haruna Nijo type) and susceptible type (H602 type) to Fusarium head blight. The amplified fragment of the resistant type (Haruna Nijo type) has a fragment length of 155bp (indicated by the arrow), whereas the amplified fragment of the susceptible type (H602 type) has a fragment length of approximately 157bp (indicated by "open" arrow). Thus, by the above-described detection operation of the plant to test, and performing the judgment using the presence of the amplification fragment of approximately 155bp or approximately 157bp, it is possible to test a plant to judge whether a gene allelic to the Fusarium head blight-resistance factor (locus relating to the Fusarium head blight resistance) located on 5H chromosome is of resistant genotype or of susceptible genotype.

### [Example 17: Detection of Genetic Marker "k00835"]

### (Method)

Using, as the template, genomic DNA of a barley to be tested, amplification reaction was carried out with primers having the base sequences of S.E.Q. ID. NOs. 30 and 31, and with 2-minute heating at 94°C, and then 5 cycles of 30 seconds at 94°C, 30 seconds at 65°C (this temperature was reduced by 1°C every cycle), 2 minutes at 72°C, followed by 35 cycles of 30 seconds at 94°C, 30 seconds at 60°C, and 2 minutes at 72°C, and then 7-minute heating at 72°C thereafter.

### (Result)

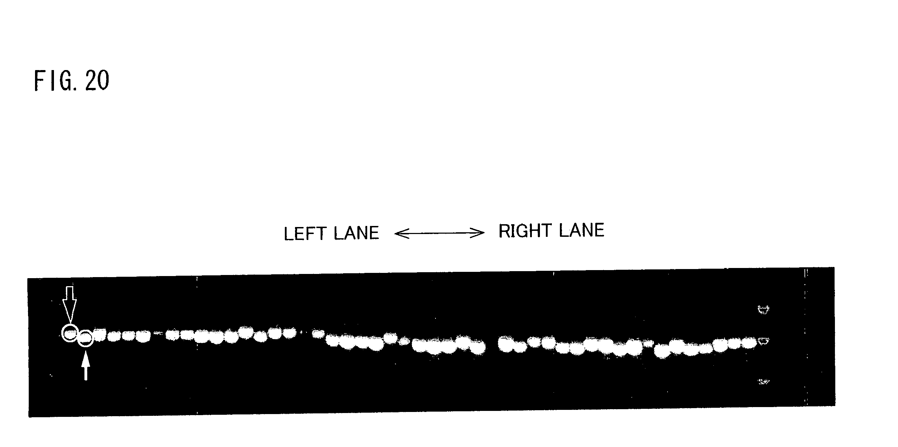

FIG. 20 shows results of electrophoresis of an amplified fragment obtained by the amplification reaction as above. In FIG. 20, the leftmost and rightmost lanes indicate size markers. The second lane from the leftmost lane shows a result of amplified fragments from Haruna Nijo, which is a Fusarium head blight-resistant cultivar. The third lane from the leftmost lane shows a result of amplified fragments from H602, which is a Fusarium head blight-susceptible cultivar. The fourth lane from the leftmost lane shows a result of amplified fragments from F1 from them. The other lanes show results of amplified fragments for the double haploid lines (DH lines) DHHS derived from the cross of Haruna Nijo and H602.

According to the results of FIG. 20, there are resistant type (Haruna Nijo type) and susceptible type (H602 type) to Fusarium head blight. The amplified fragment of the resistant type (Haruna Nijo type) has a fragment length of approximately 900bp (indicated by the arrow), whereas the amplified fragment of the susceptible type (H602 type) has a fragment length of approximately 880bp (indicated by "open" arrow). Thus, by the above-described detection operation of the plant to test, and performing the judgment using the presence of the amplification fragment of approximately 900bp or approximately 880bp, it is possible to test a plant to judge whether a gene allelic to the Fusarium head blight-resistance factor (locus relating to the Fusarium head blight resistance) located on 5H chromosome is of resistant genotype or of susceptible genotype.

The invention being thus described, it will be obvious that the same may be varied in many ways. Such variations are not to be regarded as a departure from the spirit and scope of the invention, and all such modifications as would be obvious to one skilled in the art are intended to be included within the scope of the following claims.

### INDUSTRIAL APPLICABILITY

According to a genetic marker of the present invention, which is linked to a Fusarium head blight-resistance factor, and the utilization of the same, it is possible to isolate a DNA fragment including the Fusarium head blight-resistance factor. By using the DNA fragment, it is possible to understand a gene of barley, which relates to the Fusarium head blight resistance, and mechanism of the Fusarium head blight resistance, and the like.

Moreover, according to the present invention, it is possible to produce a plant (Gramineae plant (e.g., Hordeum or the like such as barley)) having Fusarium head blight resistance, or a plant (Gramineae plant (e.g., Hordeum or the like such as barley)) whose Fusarium head blight resistance is improved. This is effective to alleviate Fusarium head blight-causing crop yield reduction and quality deterioration of barley, which is an important edible crop.

Moreover, because the genetic marker according to the present invention is linked to the Fusarium head blight-resistance factor, the use of the genetic marker according to the present invention as an indicator makes it possible to breed a Fusarium head blight-resistant Gramineae plant (Hordeum or the like such as barley). This is effective to realize efficient breeding of a Fusarium head blight-resistant Gramineae plant. More specifically, because it is possible to select the targeted plant at the stage of seedling, it is not necessary to select the target after the Fusarium head blight resistance of the plant is actually evaluated. Thus, the present invention shortens the breeding period. Moreover, because it is possible to detect a plurality of loci at the same time, the acquisition of the targeted genotype can be performed more surely than by selecting via observation. Further, it is possible to reduce the labor and field area.

Further, by performing selective breeding with the genetic marker according to the present invention as an indicator, it is possible to protect the phenotype from being influenced by environmental factor on growth. This is effective to select a useful gene (locus) surely.

Therefore, according to the present invention, it becomes possible to: understand the mechanism of Fusarium head blight resistance of barley; produce a Fusarium head blight-resistant plant (Gramineae plant (e.g., Hordeum or the like such as barley)); judge whether a Fusarium head blight-resistance factor is present or not; judge whether a plant is a Fusarium head blight-resistant plant (Gramineae plant (e.g., Hordeum or the like such as barley)) or not; and do the like tasks. Thus, when the present invention is applied to plants (Gramineae plant) such as barley, in which Fusarium spp. has caused the crop yield reduction, quality reduction, health problems, and the like, it is possible to reduce the damage from the Fusarium head blight in the plants. Therefore, the present invention is applicable to agriculture at large such as crop production. Furthermore, the present invention is effectively applicable to food industry in which barley or the like is used as raw materials.

## Claims

1. A genetic marker, which exists in a genomic DNA of a Gramineae plant and is linked to a Fusarium head blight-resistance factor, wherein:
a distance from the Fusarium head blight-resistance factor to the Genetic marker is within a range of approximately 0 to 10cM.

2. The genetic marker as set forth in Claim 1, wherein the Gramineae plant is a Hordeum or Triticum.

3. The genetic marker as set forth in Claim 2, wherein the Hordeum or Triticum is barley.

4. The genetic marker as set forth in Claim 3, wherein the genomic DNA is 2H chromosome.

5. The genetic marker as set forth in any one of Claims 1 to 4, wherein the genetic marker is for being amplified with a first primer set that is a combination of a primer having the base sequence of S.E.Q. ID. NO. 1, and a primer having the base sequence of S.E.Q. ID. NO. 2.

6. The genetic marker as set forth in Claim 5, being distanced from the Fusarium head blight-resistance factor by approximately 0cM.

7. The genetic marker as set forth in any one of Claims 1 to 4, wherein the genetic marker is for being amplified with a second primer set that is a combination of a primer having the base sequence of S.E.Q. ID. NO. 3, and a primer having the base sequence of S.E.Q. ID. NO. 4.

8. The genetic marker as set forth in Claim 7, being distanced from the Fusarium head blight-resistance factor by approximately 0.6cM.

9. The genetic marker as set forth in Claim 8, wherein the Genetic marker has the base sequence of S.E.Q. ID. NO. 8 or 9.

10. The genetic marker as set forth in any one of Claims 1 to 4, wherein the genetic marker is for being amplified with a sixth primer set that is a combination of a primer having the base sequence of S.E.Q. ID. NO. 22, and a primer having the base sequence of S.E.Q. ID. NO. 23.

11. The genetic marker as set forth in any one of Claims 1 to 4, wherein the genetic marker is for being amplified with a seventh primer set that is a combination of a primer having the base sequence of S.E.Q. ID. NO. 24, and a primer having the base sequence of S.E.Q. ID. NO. 25.

12. The genetic marker as set forth in any one of Claims 1 to 4, wherein the genetic marker is for being amplified with an eighth primer set that is a combination of a primer having the base sequence of S.E.Q. ID. NO. 26, and a primer having the base sequence of S.E.Q. ID. NO. 27.

13. The genetic marker as set forth in Claim 2, wherein the genomic DNA is 5H chromosome.

14. The genetic marker as set forth in Claim 1, 2, or 13, wherein the genetic marker is for being amplified with a third primer set that is a combination of a primer having the base sequence of S.E.Q. ID. NO. 5, and a primer having the base sequence of S.E.Q. ID. NO. 6.

15. The genetic marker as set forth in Claim 14, being distanced from the Fusarium head blight-resistance factor by approximately 9cM.

16. The genetic marker as set forth in Claim 15, wherein the genetic marker has the base sequence of S.E.Q. ID. NO. 10 or 11.

17. The genetic marker as set forth in Claim 1, 2, or 13, wherein the genetic marker is for being amplified with a fourth primer set that is a combination of a primer having the base sequence of S.E.Q. ID. NO. 5, and a primer having the base sequence of S.E.Q. ID. NO. 7.

18. The genetic marker as set forth in Claim 17, being distanced from the Fusarium head blight-resistance factor by approximately 9cM.

19. The genetic marker as set forth in Claim 18, wherein the genetic marker has the base sequence of S.E.Q. ID. NO. 12 or 13.

20. The genetic marker as set forth in Claim 1, 2, 3, or 13, wherein:
amplification of the genetic marker is carried out by:
(a) ligating MseI adaptors having the base sequences of S.E.Q. ID. NOs. 16 and 17 and EcoRI adaptors having the base sequences of S.E.Q. ID. NOs. 14 and 15 to a DNA fragment obtained by digesting a genomic DNA of a Gramineae plant with restriction enzymes MseI and EcoRI,
(b) performing pre-amplification of the ligated DNA fragment by using Msel universal primer having the base sequence of S.E.Q. ID. NO. 19, and EcoRI universal primer having the base sequence of S.E.Q. ID. NO. 18, and
(c) amplifying the pre-amplified fragment by using a fifth primer set that is a combination of a primer having the base sequence of S.E.Q. ID. NO. 21, and a primer having the base sequence of S.E.Q. ID. NO. 20.

21. The genetic marker as set forth in Claim 13 or 20, being distanced from the Fusarium head blight-resistance factor by approximately 2.2cM.

22. The genetic marker as set forth in Claim 1, 2, 3, or 13, wherein the genetic marker is for being amplified with a ninth primer set that is a combination of a primer having the base sequence of S.E.Q. ID. NO. 28, and a primer having the base sequence of S.E.Q. ID. NO. 29.

23. The genetic marker as set forth in Claim 1, 2, 3, or 13, wherein the genetic marker is for being amplified with a tenth primer set that is a combination of a primer having the base sequence of S.E.Q. ID. NO. 30, and a primer having the base sequence of S.E.Q. ID. NO. 31.

24. The genetic marker as set forth in Claim 3, wherein the genomic DNA is 4H chromosome.

25. The genetic marker as set forth in Claim 1, 2, 3, or 22, wherein:
amplification of the genetic marker is carried out by
(a) ligating MseI adaptors having the base sequences of S.E.Q. ID. NOs. 16 and 17 and EcoRI adaptors having the base sequences of S.E.Q. ID. NOs. 14 and 15 to a DNA fragment obtained by digesting a genomic DNA of a Gramineae plant with restriction enzymes MseI and EcoRI,
(b) performing pre-amplificatiori of the ligated DNA fragment by using MseI universal primer having the base sequence of S.E.Q. ID. NO. 19, and EcoRI universal primer having the base sequence of S.E.Q. ID. NO. 18, and
(c) amplifying the pre-amplified fragment by using a tenth primer set that is a combination of a primer having the base sequence of S.E.Q. ID. NO. 32, and a primer having the base sequence of S.E.Q. ID. NO. 33.

26. The genetic marker as set forth in Claim 1, 2, 3, or 22, wherein:
amplification of the genetic marker is carried out by
(a) ligating MseI adaptors having the base sequences of S.E.Q. ID. NOs. 16 and 17 and EcoRI adaptors having the base sequences of S.E.Q. ID. NOs. 14 and 15 to a DNA fragment obtained by digesting a genomic DNA of a Gramineae plant with restriction enzymes MseI and EcoRI,
(b) performing pre-amplification of the ligated DNA fragment by using MseI universal primer having the base sequence of S.E.Q. ID. NO. 19, and EcoRI universal primer having the base sequence of S.E.Q. ID. NO. 18, and
(c) amplifying the pre-amplified fragment by using a twelfth primer set that is a combination of a primer having the base sequence of S.E.Q. ID. NO. 34, and a primer having the base sequence of S.E.Q. ID. NO. 35.

27. The genetic marker as set forth in Claim 1, 2, 3, or 22, wherein:
amplification of the genetic marker is carried out by
(a) ligating MseI adaptors having the base sequences of S.E.Q. ID. NOs. 16 and 17 and EcoRI adaptors having the base sequences of S.E.Q. ID. NOs. 14 and 15 to a DNA fragment obtained by digesting a genomic DNA of a Gramineae plant with restriction enzymes MseI and EcoRI,
(b) performing pre-amplification of the ligated DNA fragment by using MseI universal primer having the base sequence of S.E.Q. ID. NO. 19, and EcoRI universal primer having the base sequence of S.E.Q. ID. NO. 18, and
(c) amplifying the pre-amplified fragment by using a thirteenth primer set that is a combination of a primer having the base sequence of S.E.Q. ID. NO. 36, and a primer having the base sequence of S.E.Q. ID. NO. 37.

28. The genetic marker as set forth in Claim 1, 2, 3, or 22, wherein the genetic marker is for being amplified with a fourteenth primer set that is a combination of a primer having the base sequence of S.E.Q. ID. NO. 38, and a primer having the base sequence of S.E.Q. ID. NO. 39.

29. The genetic marker as set forth in Claim 1, 2, 3, or 22, wherein the genetic marker is for being amplified with a fifteenth primer set that is a combination of a primer having the base sequence of S.E.Q. ID. NO. 40, and a primer having the base sequence of S.E.Q. ID. NO. 41.

30. The genetic marker as set forth in Claim 1, 2, 3, or 22, wherein the genetic marker is for being amplified with a sixteenth primer set that is a combination of a primer having the base sequence of S.E.Q. ID. NO. 42, and a primer having the base sequence of S.E.Q. ID. NO. 43.

31. The genetic marker as set forth in Claim 3, wherein the genomic DNA is 6H chromosome.

32. The genetic marker as set forth in Claim 1, or 2, or 3, or 31, wherein:
amplification of the genetic marker is carried out by
(a) ligating MseI adaptors having the base sequences of S.E.Q. ID. NOs. 16 and 17 and EcoRI adaptors having the base sequences of S.E.Q. ID. NOs. 14 and 15 to a DNA fragment obtained by digesting a genomic DNA of a Gramineae plant with restriction enzymes MseI and EcoRI,
(b) performing pre-amplification of the ligated DNA fragment by using MseI universal primer having the base sequence of S.E.Q. ID. NO. 19, and EcoRI universal primer having the base sequence of S.E.Q. ID. NO. 18, and
(c) amplifying the pre-amplified fragment by using a seventeenth primer set that is a combination of a primer having the base sequence of S.E.Q. ID. NO. 44, and a primer having the base sequence of S.E.Q. ID. NO. 45.

33. The genetic marker as set forth in Claim 1, 2, 3, or 31, wherein the genetic marker is for being amplified with an eighteenth primer set that is a combination of a primer having the base sequence of S.E.Q. ID. NO. 46, and a primer having the base sequence of S.E.Q. ID. NO. 47.

34. A DNA fragment isolating method, comprising:
isolating, by using the genetic marker as set forth in any one of Claims 1 to 33, a DNA fragment including a Fusarium head blight-resistance factor.

35. A method for producing a Fusarium head blight-resistance plant, comprising:
introducing, into a genomic DNA of a plant, the DNA fragment that is obtained by the method as set forth in Claim 34 and includes the Fusarium head blight-resistance factor.

36. The genetic marker as set forth in Claim 35, wherein the plant is Gramineae.

37. The genetic marker as set forth in Claim 36, wherein the Gramineae plant is Hordeum or Triticum.

38. The genetic marker as set forth in Claim 37, wherein the Hordeum or Triticum is barley.

39. A Fusarium head blight-resistant plant obtained by the method as set forth in any one of Claims 35 to 38.

40. A method for judging whether a plant is a Fusarium head blight-resistant plant or not, comprising:
detecting the genetic marker as set forth in any one of Claims 1 to 33.

41. A kit for judging, by the method as set forth in Claim 40, whether a plant is a Fusarium head blight-resistant plant or not.

42. A gene detecting apparatus, wherein at least one of the genetic markers as set forth in Claims 1 to 33 is fixed.

43. A primer population for use in detecting the genetic markers as set forth in Claims 1 to 33, comprising:
at least two of primers having the base sequences of S.E.Q. ID. NOs. 1 to 7, and 20 to 47.
